# EUROPEAN PATENT APPLICATION

(11) **EP 4 610 372 A2**
(43) Date of publication of application: **03.09.2025**
(21) Application number: 25187372.5
(22) Date of filing: 23.01.2019
(51) Int. Cl.: C12R 1/07

(54) **PLANT MICROBIAL PREPARATIONS, COMPOSITIONS AND FORMULATIONS COMPRISING SAME AND USES THEREOF**

(30) Priority: 29.01.2018 US 201862623029 P
(62) Divisional of application: 19744568.7
(71) Applicant: Lavie Bio Ltd., 7612002 Rehovot (IL)
(72) Inventor: IONESCU, Michael, 7682327 Mazkeret Batia (IL); ADATO, Avital, 5232717 Ramat Gan (IL); ETZIONI, Adi, 9980300 Doar-Na Shimshon (IL); KARCHI, Hagai, 7683400 Moshav Sitriya (IL)
(74) Representative: Bals & Vogel Patentanwälte PartGmbB

(57) **Abstract**

Provided are microbial strains and functional homologs of same and compositions comprising same. Also provided are methods of manufacturing microbial compositions and uses thereof in improving agricultural traits.

## Description

### FIELD AND BACKGROUND OF THE INVENTION

### RELATED APPLICATION

This application claims the benefit of priority from U.S. Provisional Patent Application No. 62/623,029 filed on January 29, 2018, the contents of which are incorporated herein by reference in their entirety.

The present invention, in some embodiments thereof, relates to plant microbial preparations, compositions and formulations comprising same and uses thereof.

Global demands for food and fiber will increase up to 70 % by 2050. This increase in agricultural productivity needs to be obtained from existing arable land, under harsher climate conditions and with declining soil and water quality.

Research is thus very much focused at improving any of nutrient acquisition, disease resistance, resilience to abiotic stresses and fitness in novel environments.

Conventional farming that uses chemicals in the form of fertilizers and pesticides has substantially increased agriculture productivity and contributed immensely to food access and poverty alleviation goals. However, excessive and indiscriminate use of these chemicals has resulted in food contamination, negative environmental outcomes and disease resistance which together have a significant impact on human health and food security.

Traditional plant breeding strategies to enhance plant traits has been used from the dawn of humanity. In fact, the advantage of breeding to meet the nutritional demands of the population is said to have been a major driver for the Industrial Revolution. However, this approach is slow and may have exhausted its potential. For example, breeding plants for increased tolerance to abiotic stress requires abiotic stress-tolerant founder lines for crossing with other germplasm to develop new abiotic stress-resistant lines. Limited germplasm resources for such founder lines and incompatibility in crosses between distantly related plant species represent significant problems encountered in conventional breeding. Breeding for stress tolerance has often been inadequate given the incidence of stresses and the impact that stresses have on crop yields in most environments of the world.

Genetically modified (GM) crops are increasingly used to improve plant productivity. Herbicide-tolerant and insect-resistant transgenic crops have been adopted by many countries as a food security measure. Nevertheless, the fate of GM crops lies on the balance between growing these crops for hunger management, nutrient fulfilment, pest resistance and efficacy of crops, and their secondary effects beyond their target objectives, including multi-trophic effects on non-target species.

The microbiome technology has the potential to minimize this environmental footprint and at the same time sustainably increase the quality and quantity of farm produce with less resource-based inputs.

### SUMMARY OF THE INVENTION

According to an aspect of some embodiments of the present invention there is provided a preparation comprising a microbial strain selected from the group consisting of:
(1) an EVO33432 strain, deposited as Accession Number 42921 at NCIMB or a functionally homologous strain;
(2) an EVO33410 strain, deposited as Accession Number 42961 at NCIMB or a functionally homologous strain;
(3) an EVO33407 strain, deposited as Accession Number 42922 at NCIMB or a functionally homologous strain;
(4) an EVO33401 strain, deposited as Accession Number 42923 at NCIMB or a functionally homologous strain;
(5) an EVO33393 strain, deposited as Accession Number 42924 at NCIMB or a functionally homologous strain;
(6) an EVO33661 strain, deposited as Accession Number 42925 at NCIMB or a functionally homologous strain;
(7) an EVO33398 strain, deposited as Accession Number 42926 at NCIMB or a functionally homologous strain;
(8) an EVO33395 strain, deposited as Accession Number 42927 at NCIMB or a functionally homologous strain;
(9) an EVO33394 strain, deposited as Accession Number 42928 at NCIMB or a functionally homologous strain;
(10) an EVO32844 strain, deposited as Accession Number 42929 at NCIMB or a functionally homologous strain;
(11) an EVO32845 strain, deposited as Accession Number 42930 at NCIMB or a functionally homologous strain;
(12) an EVO33405 strain, deposited as Accession Number 42931 at NCIMB or a functionally homologous strain;
(13) an EVO32831 strain, deposited as Accession Number 42932 at NCIMB or a functionally homologous strain;
(14) an EVO33746 strain, deposited as Accession Number 42933 at NCIMB or a functionally homologous strain;
(15) an EVO33872 strain, deposited as Accession Number 42959 at NCIMB or a functionally homologous strain;
(16) an EVO33887 strain, deposited as Accession Number 42934 at NCIMB or a functionally homologous strain;
(17) an EVO11090 strain, deposited as Accession Number 42935 at NCIMB or a functionally homologous strain;
(18) an EVO33657 strain, deposited as Accession Number 42936 at NCIMB or a functionally homologous strain;
(19) an EVO33447 strain, deposited as Accession Number 42937 at NCIMB or a functionally homologous strain;
(20) an EVO33415 strain, deposited as Accession Number 42938 at NCIMB or a functionally homologous strain;
(21) an EVO40185 strain, deposited as Accession Number 42939 at NCIMB or a functionally homologous strain;
(22) an EVO32828 strain, deposited as Accession Number 42940 at NCIMB or a functionally homologous strain;
(23) an EVO32834 strain, deposited as Accession Number 42941 at NCIMB or a functionally homologous strain;
(24) an EVO32868 strain, deposited as Accession Number 42942 at NCIMB or a functionally homologous strain;
(25) an EVO33402 strain, deposited as Accession Number 42943 at NCIMB or a functionally homologous strain;
(26) an EVO40194 strain, deposited as Accession Number 42944 at NCIMB or a functionally homologous strain;
(27) an EVO32839 strain, deposited as Accession Number 42945 at NCIMB or a functionally homologous strain; and
(28) an EVO33441 strain, deposited as Accession Number 42960 at NCIMB or a functionally homologous strain;
wherein the microbial strain or the functionally homologous strain improves an agricultural trait of a cultivated plant heterologous to the microbial strain or the functionally homologous strain as compared to a control plant not treated with the microbial strain or the functionally homologous strain, and wherein the microbial strain or the functionally homologous strain is present in the preparation at a concentration which exceeds that found in nature.

According to some embodiments of the invention, the functionally homologous strain has substantially the same coding and/or non-coding sequence orientation as that of the microbial strain homologous thereto.

According to some embodiments of the invention, the agricultural trait is selected from the group consisting of increased early vigor, increased biomass establishment, increased photosynthetic capacity, increased leaf transpiration rate, increased biomass accumulation up to VT, increased kernel number per plant, increased yield, increased stem conductance, increased assimilate partitioning, kernel volume, increased kernel weight, increased grain filling duration, increased main ear size and increased cob conductance.

According to some embodiments of the invention, the microbial strain or functionally homologous strain are characterized by the phenotypes disclosed in Tables 2-58 below.

According to some embodiments of the invention, the functionally homologous strain has at least 95 % sequence identity to the corresponding sub-genomic sequences of Table 60.

According to some embodiments of the invention, the functionally homologous strain has at least 99.5 % sequence identity to a genome of the microbial strain or at least 99.5 % sequence identity to a 16S of the microbial strain.

According to some embodiments of the invention, the amount is sufficient to interact, colonize and/or localize in the cultivated plant.

According to some embodiments of the invention, the amount is at least 100 CFU or spores.

According to an aspect of some embodiments of the present invention there is provided a composition comprising the preparation and further comprising an agriculturally effective amount of a compound or composition selected from the group consisting of a fertilizer, an acaricide, a bactericide, a fungicide, an insecticide, a microbicide, a nematicide, a pesticide, a plant growth regulator, a rodenticide, a nutrient.

According to an aspect of some embodiments of the present invention there is provided a formulation comprising the preparation or composition.

According to some embodiments of the invention, the formulation is selected from the group consisting of an emulsion, a colloid, a dust, a granule, a pellet, a powder, a spray and a solution.

According to some embodiments of the invention, the formulation further comprises at least one of a stabilizer, a tackifier, a preservative, a carrier, a surfactant, an anticomplex agent and a combination thereof.

According to some embodiments of the invention, the formulation is substantially stable for at least 180 days at 37 °C or 4 °C.

According to some embodiments of the invention, the formulation is a liquid, solid, semi-solid, gel or powder.

According to an aspect of some embodiments of the present invention there is provided a microbial culture comprising the preparation.

According to some embodiments of the invention, the microbial culture is at least 99.1 % pure.

According to some embodiments of the invention, the preparation, composition, formulation, microbial culture comprises no more than 10 bacterial strains.

According to some embodiments of the invention, the preparation, composition, formulation, microbial culture is soil-free.

According to an aspect of some embodiments of the present invention there is provided a method of treating a cultivated plant or portion thereof, the method comprising contacting the plant or portion thereof with the preparation, composition or formulation.

According to an aspect of some embodiments of the present invention there is provided a method of improving an agricultural trait of a cultivated plant, the method comprising:
(a) contacting the plant or portion thereof with an effective amount of the preparation, composition or formulation; and
(b) growing the plant or portion thereof; and
(c) selecting for the agricultural trait.

According to some embodiments of the invention, the contacting comprises contacting the plant's surrounding.

According to some embodiments of the invention, the contacting is selected from the group consisting of spraying, immersing, coating, encapsulating, dusting.

According to some embodiments of the invention, the contacting comprises coating.

According to some embodiments of the invention, the microbial strain is present at a concentration of at least 100 CFU or spores per plant or portion thereof after the contacting.

According to some embodiments of the invention, the portion comprises a seed.

According to some embodiments of the invention, the portion comprises a seedling.

According to some embodiments of the invention, the portion comprises a cutting.

According to some embodiments of the invention, the portion comprises a rhizosphere.

According to some embodiments of the invention, the portion comprises a vegetative portion.

According to some embodiments of the invention, the portion comprises foliage.

According to some embodiments of the invention, the method further comprises growing the plant or portion thereof.

According to some embodiments of the invention, the growing is under biotic or abiotic stress.

According to some embodiments of the invention, the growing is under drought conditions.

According to some embodiments of the invention, the growing is under non-stress conditions.

According to some embodiments of the invention, the agricultural trait is selected from the group consisting of increased early vigor, increased biomass establishment, increased photosynthetic capacity, increased leaf transpiration rate, increased biomass accumulation up to VT, increased kernel number per plant, increased yield, increased stem conductance, increased assimilate partitioning, kernel volume, increased kernel weight, increased grain filling duration, increased main ear size and increased cob conductance.

According to some embodiments of the invention, the agricultural trait is selected from the group consisting of increased biomass, increased vigor, increased yield, increased resistance to abiotic stress, increased resistance to biotic stress and increased nitrogen utilization efficiency.

According to some embodiments of the invention, the agricultural trait is selected from the group consisting of increased root biomass, increased root length, increased height, increased shoot length, increased leaf number, increased water use efficiency, increased tolerance to low nitrogen stress, increased grain yield, increased photosynthetic rate, increased tolerance to drought, increased salt tolerance, increased resistance to nematode stress, increased resistance to a fungal pathogen, increased resistance to a bacterial pathogen and an increased resistance to a viral pathogen.

According to an aspect of some embodiments of the present invention there is provided a cultivated plant or portion thereof having been treated with the preparation, composition or formulation.

According to an aspect of some embodiments of the present invention there is provided a composition comprising the preparation, composition, culture or formulation and a cultivated plant or a portion thereof, the plant or portion thereof being heterologous to the microbial strain or culture.

According to some embodiments of the invention, the portion comprises a seed, seedling or cutting.

According to some embodiments of the invention, the microbial strain coats the portion.

According to some embodiments of the invention, the microbial strain is present in the coat at a concentration of at least 100 CFU or spores per seed.

According to an aspect of some embodiments of the present invention there is provided a method of processing a cultivated plant or portion thereof to a processed product of interest, the method comprising:
(a) providing the cultivated plant or portion thereof;
(b) subjecting the cultivated plant or portion thereof to a processing procedure so as to obtain the processed product.

According to an aspect of some embodiments of the present invention there is provided a processed product comprising the cultivated plant or portion thereof.

According to some embodiments of the invention, the processed product comprises DNA unique for the cultivated plant or portion thereof and to the microbial strain and which can be detected by deep-sequencing.

According to some embodiments of the invention, the processed product is selected from the group consisting of a flour, a syrup, a meal, an oil, a film, a packaging, a construction material, a paper, a nutraceutical product, a pulp, an animal feed, a fish fodder, a bulk material for industrial chemicals, a cereal product and a processed human-food product.

According to an aspect of some embodiments of the present invention there is provided an article of manufacture comprising the seed.

According to some embodiments of the invention, the article of manufacture is selected from the group consisting of a bag, a box, a bin, an envelope, a carton or a container.

According to an aspect of some embodiments of the present invention there is provided a method for preparing an agricultural composition, the method comprising inoculating a microbial strain selected from the group consisting of:
(1) an EVO33432 strain, deposited as Accession Number 42921 at NCIMB or a functionally homologous strain;
(2) an EVO33410 strain, deposited as Accession Number 42961 at NCIMB or a functionally homologous strain;
(3) an EVO33407 strain, deposited as Accession Number 42922 at NCIMB or a functionally homologous strain;
(4) an EVO33401 strain, deposited as Accession Number 42923 at NCIMB or a functionally homologous strain;
(5) an EVO33393 strain, deposited as Accession Number 42924 at NCIMB or a functionally homologous strain;
(6) an EVO33661 strain, deposited as Accession Number 42925 at NCIMB or a functionally homologous strain;
(7) an EVO33398 strain, deposited as Accession Number 42926 at NCIMB or a functionally homologous strain;
(8) an EVO33395 strain, deposited as Accession Number 42927 at NCIMB or a functionally homologous strain;
(9) an EVO33394 strain, deposited as Accession Number 42928 at NCIMB or a functionally homologous strain;
(10) an EVO32844 strain, deposited as Accession Number 42929 at NCIMB or a functionally homologous strain;
(11) an EVO32845 strain, deposited as Accession Number 42930 at NCIMB or a functionally homologous strain;
(12) an EVO33405 strain, deposited as Accession Number 42931 at NCIMB or a functionally homologous strain;
(13) an EVO32831 strain, deposited as Accession Number 42932 at NCIMB or a functionally homologous strain;
(14) an EVO33746 strain, deposited as Accession Number 42933 at NCIMB or a functionally homologous strain;
(15) an EVO33872 strain, deposited as Accession Number 42959 at NCIMB or a functionally homologous strain;
(16) an EVO33887 strain, deposited as Accession Number 42934 at NCIMB or a functionally homologous strain;
(17) an EVO11090 strain, deposited as Accession Number 42935 at NCIMB or a functionally homologous strain;
(18) an EVO33657 strain, deposited as Accession Number 42936 at NCIMB or a functionally homologous strain;
(19) an EVO33447 strain, deposited as Accession Number 42937 at NCIMB or a functionally homologous strain;
(20) an EVO33415 strain, deposited as Accession Number 42938 at NCIMB or a functionally homologous strain;
(21) an EVO40185 strain, deposited as Accession Number 42939 at NCIMB or a functionally homologous strain;
(22) an EVO32828 strain, deposited as Accession Number 42940 at NCIMB or a functionally homologous strain;
(23) an EVO32834 strain, deposited as Accession Number 42941 at NCIMB or a functionally homologous strain;
(24) an EVO32868 strain, deposited as Accession Number 42942 at NCIMB or a functionally homologous strain;
(25) an EVO33402 strain, deposited as Accession Number 42943 at NCIMB or a functionally homologous strain;
(26) an EVO40194 strain, deposited as Accession Number 42944 at NCIMB or a functionally homologous strain;
(27) an EVO32839 strain, deposited as Accession Number 42945 at NCIMB or a functionally homologous strain; and
(28) an EVO33441 strain, deposited as Accession Number 42960 at NCIMB or a functionally homologous strain;
wherein the microbial strain or the functionally homologous strain improves an agricultural trait of a cultivated plant heterologous to the microbial strain or the functionally homologous strain as compared to a control plant not treated with the microbial strain or the functionally homologous strain, into or onto a substratum, and allowing the microbial strain or the functional homolog to grow at a temperature of 1-37°C until obtaining a number of cells or spores of at least 10²-10³ per milliliter or per gram.

According to some embodiments of the invention, the cultivated plant is a monocot or dicot plant.

According to some embodiments of the invention, the cultivated plant is of the Gramineae family.

According to some embodiments of the invention, the functional homolog is characterized by at least one of:
at least 70 % DNA-DNA relatedness to the deposited strain with 5 uC or less DTm;
at least 70 % genomic DNA sequence identity to the genomic DNA sequence of the deposited strain;
having an average nucleotide identity (ANI) of at least about 97 % with the deposited strain;
having a tetranucleotide signature frequency correlation coefficient of at least about 0.99 with the deposited strain;
having a Dice similarity coefficient of at least 96 %;
being of the same ribotype as that of the deposited strain;
having a Pearson correlation coefficient of at least about 0.99 with the deposited strain;
having a multilocus sequence typing (MLST) of at least about 0.99 with the deposited strain;
having a -functionally conserved gene that is at least about 97 % identical to that of the deposited strain as determined at a level of a single gene or multilocus sequence analysis (MLSA);
having a 16S nucleic acid sequence that is at least about 97 % identical to that of the deposited strain;
having substantially the same biochemical profiling as determined by the GEN III redox chemistry; and
maintaining the coding and/or non-coding sequence order as that of the deposited strain.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### DESCRIPTION OF SPECIFIC EMBODIMENTS OF THE INVENTION

The present invention, in some embodiments thereof, relates to plant microbial preparations, compositions and formulations comprising same and uses thereof.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

Beneficial plant microbiota include the microorganisms that live in the plant surroundings as well as microorganisms that live within host plants for at least part of their life and do not cause apparent symptoms of diseases, also termed as "endophytes". In general, beneficial plant microbials promote host plant growth, increase plant nutrient uptake, inhibit plant pathogen growth, reduce disease severity, and enhance tolerance to environmental stresses.

As sustainable and renewable agricultural production increases in prominence, it is envisaged that plant microorganisms will play important roles and will offer environmentally-friendly methods to increase productivity while reducing chemical inputs. Among current challenges is the identification of microbial strains that truly impart commercially valuable traits to cultivated crop plants treated therewith, as evidenced in field test(s).

Whilst reducing embodiments of the invention to practice, the present inventors have devised a novel approach for the identification of microbial strains for plant bio-stimulatory activity. Sourcing was guided by at least one of the following assumptions:
1) That the plant microbiome is enriched with plant beneficial microbials that co-evolved with plants and developed a mutualistic interaction with plants (Bulgarelli, D., Schlaeppi, K., Spaepen, S., Ver Loren van Themaat, E., Schulze-Lefert, P. 2013. Structure and functions of the bacterial microbiota of plants. Annu. Rev. Plant Biol. 64:807-838).
2) That plants growing in the wild are dependent on functions provided by their microbiome for survival and reproduction. In contrast, domesticated plants are nurtured by farmers and therefore do not need the full extent of microbiome functions and therefore are not a primary source for beneficial microbial strains (Philippot, L., Raaijmakers, J.M., Lemanceau, P., and van der Putten, W.H. 2013. Going back to the roots: the microbial ecology of the rhizosphere. Nat. Rev. Microbiol. 11:789-799).
3) That microbial strains that provide plants with functions that alleviate drought stress are found in climatic zones, habitats and niches in which plant experience water deficiency such as arid and semi-arid climatic zones and sandy soil habitats, and such strains can be effective and be found even on domesticated plants.
4) That the microbiome of plants evolutionarily related to the target plant (*Zea maize*) such as various cereal plants including C4 and C3 plants, are enriched with microbial strains that can also interact, colonize and provide beneficial functions to the target plant.
5) That native plants co-evolved with the local microbial diversity to exploit the functional diversity available for their survival and reproduction, and therefore are a better source for microbial strains with plant bio-stimulatory activity than non-native plants.

Sourced strains were isolated and screened according to various selection criteria and those that passed the selections are described in Tables 1-60 hereinbelow. Also contemplated are functional homologs of these strains as defined and described hereinbelow.

Thus, according to an aspect of the invention there is provided a preparation comprising a microbial strain selected from the group consisting of:
(1) an EVO33432 strain, deposited as Accession Number 42921 at NCIMB or a functionally homologous strain;
(2) an EVO33410 strain, deposited as Accession Number 42961 at NCIMB or a functionally homologous strain;
(3) an EVO33407 strain, deposited as Accession Number 42922 at NCIMB or a functionally homologous strain;
(4) an EVO33401 strain, deposited as Accession Number 42923 at NCIMB or a functionally homologous strain;
(5) an EVO33393 strain, deposited as Accession Number 42924 at NCIMB or a functionally homologous strain;
(6) an EVO33661 strain, deposited as Accession Number 42925 at NCIMB or a functionally homologous strain;
(7) an EVO33398 strain, deposited as Accession Number 42926 at NCIMB or a functionally homologous strain;
(8) an EVO33395 strain, deposited as Accession Number 42927 at NCIMB or a functionally homologous strain;
(9) an EVO33394 strain, deposited as Accession Number 42928 at NCIMB or a functionally homologous strain;
(10) an EVO32844 strain, deposited as Accession Number 42929 at NCIMB or a functionally homologous strain;
(11) an EVO32845 strain, deposited as Accession Number 42930 at NCIMB or a functionally homologous strain;
(12) an EVO33405 strain, deposited as Accession Number 42931 at NCIMB or a functionally homologous strain;
(13) an EVO32831 strain, deposited as Accession Number 42932 at NCIMB or a functionally homologous strain;
(14) an EVO33746 strain, deposited as Accession Number 42933 at NCIMB or a functionally homologous strain;
(15) an EVO33872 strain, deposited as Accession Number 42959 at NCIMB or a functionally homologous strain;
(16) an EVO33887 strain, deposited as Accession Number 42934 at NCIMB or a functionally homologous strain;
(17) an EVO11090 strain, deposited as Accession Number 42935 at NCIMB or a functionally homologous strain;
(18) an EVO33657 strain, deposited as Accession Number 42936 at NCIMB or a functionally homologous strain;
(19) an EVO33447 strain, deposited as Accession Number 42937 at NCIMB or a functionally homologous strain;
(20) an EVO33415 strain, deposited as Accession Number 42938 at NCIMB or a functionally homologous strain;
(21) an EVO40185 strain, deposited as Accession Number 42939 at NCIMB or a functionally homologous strain;
(22) an EVO32828 strain, deposited as Accession Number 42940 at NCIMB or a functionally homologous strain;
(23) an EVO32834 strain, deposited as Accession Number 42941 at NCIMB or a functionally homologous strain;
(24) an EVO32868 strain, deposited as Accession Number 42942 at NCIMB or a functionally homologous strain;
(25) an EVO33402 strain, deposited as Accession Number 42943 at NCIMB or a functionally homologous strain;
(26) an EVO40194 strain, deposited as Accession Number 42944 at NCIMB or a functionally homologous strain;
(27) an EVO32839 strain, deposited as Accession Number 42945 at NCIMB or a functionally homologous strain; and
(28) an EVO33441 strain, deposited as Accession Number 42960 at NCIMB or a functionally homologous strain;
wherein the microbial strain or the functionally homologous strain improves an agricultural trait of a cultivated plant heterologous to the microbial strain or the functionally homologous strain as compared to a control plant not treated with the microbial strain or the functionally homologous strain, and wherein the microbial strain or the functionally homologous strain is present in the preparation at a concentration which exceeds that found in nature.

Accession numbers 42921-42945 were deposited at NCIMB, Ferguson Building, Craibstone Estate, Bucksburn, Aberdeen, AB21 9YA. United Kingdom, on December 14, 2017. Accession numbers 42959-42961 were deposited at NCIMB, Ferguson Building, Craibstone Estate, Bucksburn, Aberdeen, AB21 9YA. United Kingdom, on January 10, 2018.

According to a specific embodiment, the microbial strain or functional homolog thereof interacts with a host plant on or inside plant tissues, such as but not limited to, the rhizosphere (soil around root), rhizoplane (root surface), root endosphere (inside the root), stem endosphere (inside the stem), leaf endosphere (inside the leaf), phyllosphere (on the shoot, stem and leaf surface), seed surface and seed endosphere (inside the seed).

The microbial strain can be as deposited or a variant thereof, also referred to herein as a "functional homolog".

The term "microbial strain" can refer to the deposited strain and/or the functional homolog.

As used herein "functional homolog" or "functionally homologous" or "variant" or grammatical equivalents as used herein refers to a modification (i.e., mutant, at least one mutation) of the deposited microbial strain resulting in a microbial strain that is endowed with substantially the same ensemble of biological activities (+/- 10 %, 20 %, 40 %, 50 %, 60 % when tested under the same conditions) as that of the deposited strain (see Tables 2-58) and can be classified to the same species or strain based on known methods of species/strain classifications. The modification can be man-made or evolutionary, e.g., during propagation with or without selection.

Following are non-limiting criteria for identifying a functional homolog. These criteria, which are mostly genetic, combined with the functional characteristics as defined in Tables 2-58 above, will be apparent to the skilled artisan as defining the scope of the functional homolog.

Thus, according to a specific embodiment, the deposited strain and the functional homolog belong to the same operational taxonomic units (OTU).

An "OTU" (or plural, "OTUs") refers to a terminal leaf in a phylogenetic tree and is defined by a nucleic acid sequence, e.g., the entire genome, or a specific genetic sequence, and all sequences that share sequence identity to this nucleic acid sequence at the level of species. In some embodiments the specific genetic sequence may be the 16S-rRNA sequence or a portion of the 16S-rRNA (also referred to herein as "16S") sequence, or other functionally conserved genes as listed below. In other embodiments, the entire genomes of two entities are sequenced and compared. In another embodiment, select regions such as multilocus sequence tags (MLST, MLSA), specific genes, or sets of genes may be genetically compared. In 16S-rRNA embodiments, OTUs that share at least 97 % average nucleotide identity across the entire 16S or some variable region of the 16S are considered the same OTU (see e.g. Claesson M J, Wang Q, O'Sullivan O, Greene-Diniz R, Cole J R, Ros R P, and O'Toole P W. 2010. Comparison of two next-generation sequencing technologies for resolving highly complex microbiota composition using tandem variable 16S rRNA gene regions. Nucleic Acids Res 38: e200. Konstantinidis K T, Ramette A, and Tiedje J M. 2006. The bacterial species definition in the genomic era. Philos Trans R Soc Lond B Biol Sci 361: 1929-1940). In embodiments involving the complete genome, MLSTs, specific genes, or sets of genes OTUs that share at least 95% average nucleotide identity are considered the same OTU (see e.g. Achtman M, and Wagner M. 2008. Microbial diversity and the genetic nature of microbial species. Nat. Rev. Microbiol. 6: 431-440. Konstantinidis K T, Ramette A, and Tiedje J M. 2006. The bacterial species definition in the genomic era. Philos Trans R Soc Lond B Biol Sci 361: 1929-1940). OTUs are frequently defined by comparing sequences between organisms. Such characterization employs, e.g., WGS data or a whole genome sequence.

According to a specific embodiment, the classification is based on DNA-DNA pairing data and/or sequence identity to functionally conserved genes or fragments thereof.

According to a specific embodiment, a species/strain can be defined by DNA-DNA hybridization involving a pairwise comparison of two entire genomes and reflects the overall sequence similarity between them. According to a specific embodiment, a species is defined as a set of strains with at least about 70 %, e.g., at least about 75 %, at least about 80 %, at least about 85 %, at least about 90 %, at least about 91 %, at least about 92 %, at least about 93 %, at least about 94 %, at least about 95 % or more DNA-DNA relatedness and with 5 uC or less DTm and having the activities as defined per strain in Tables 2-58 below.

According to a specific embodiment, the genomic nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95% 99.95%, at least about 99.99 %, at least about 99.999 %, at least about 99.9999 %, at least about 99.99999 %, at least about 99.999999 % or more DNA-DNA relatedness and with 5 uC or less DTm and having the activities as defined per strain in Tables 2-58 below.

Thus, for example, if there is DNA-DNA hybridization on the basis of the article of Goris et al. [Goris, J., Konstantinidis, K. T., Klappenbach, J. A., Coenye, T., Vandamme, P., and Tiedje, J M. (2007). DNA-DNA hybridization values and their relationship to whole-genome sequence similarities. Int J Syst Evol Microbiol 57:81-91], some microorganisms expressing a DNA-DNA relatedness value of 70 % or more (as described above) can be regarded as functional homologs according to some embodiments of the invention.

As used herein, "sequence identity" or "identity" or grammatical equivalents as used herein in the context of two nucleic acid or polypeptide sequences includes reference to the residues in the two sequences which are the same when aligned. When percentage of sequence identity is used in reference to proteins it is recognized that residue positions which are not identical often differ by conservative amino acid substitutions, where amino acid residues are substituted for other amino acid residues with similar chemical properties (e.g. charge or hydrophobicity) and therefore do not change the functional properties of the molecule. Where sequences differ in conservative substitutions, the percent sequence identity may be adjusted upwards to correct for the conservative nature of the substitution. Sequences which differ by such conservative substitutions are considered to have "sequence similarity" or "similarity". Means for making this adjustment are well-known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., according to the algorithm of Henikoff S and Henikoff JG. [Amino acid substitution matrices from protein blocks. Proc. Natl. Acad. Sci. U.S.A. 1992, 89(22): 10915-9].

Identity can be determined using any homology comparison software, including for example, the BlastN software of the National Center of Biotechnology Information (NCBI) such as by using default parameters.

According to some embodiments of the invention, the identity is a global identity, *i.e.,* an identity over the entire nucleic acid sequence (*i.e.,* query coverage) of the invention and not over portions thereof.

The *query coverage* - a percentage that describes how much of the *query* sequence is *covered* by the target sequence.

According to a specific embodiment, identity of marker sequence is defined as at least 90% *query coverage with at least 95 % identity,* such as further described herein.

In other cases the identity of 16S sequence is defined as at least 100% *query coverage with at least 97 % identity.*

According to a specific embodiment, the genomic nucleic acid sequence is at least about 70 %, at least 75 %, at least about 80 %, at least about 85 %, at least about 90 %, at least about 91 %, at least about 92 %, at least about 93 %, at least about 94 %, at least about 95 %, at least about 96 % least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95% 99.95%, at least about 99.99 %, at least about 99.999 %, at least about 99.9999 %, at least about 99.99999 %, at least about 99.999999 % or more to the genomic sequence of the deposited strain.

According to a specific embodiment, the genomic nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, 99.95%, at least about 99.99 %, at least about 99.999 %, at least about 99.9999 %, at least about 99.99999 %, at least about 99.999999 % or more identical to that of the deposited strain.

According to an additional or alternative embodiment, a functional homolog is determined as the average nucleotide identity (ANI), which detects the DNA conservation of the core genome (Konstantinidis K and Tiedje J M, 2005, Proc. Natl. Acad. Sci. USA 102: 2567-2592). In some embodiments, the ANI between the functional homolog and the deposited strain is of at least about 95 %, at least about, 96 %, at least about 97 %, at least about 98 %, at least about 99 %, at least about 99.1 %, at least about 99.5 %, at least about 99.6 %, at least about 99.7 %, at least about 99.8 %, at least about 99.9 %, at least about 99.99 %, at least about 99.999 %, at least about 99.9999 %, at least about 99.99999 %, at least about 99.999999 % or more.

According to an additional or alternative embodiment, a functional homolog is determined by the degree of relatedness between the functional homolog and deposited strain determined as the Tetranucleotide Signature Frequency Correlation Coefficient, which is based on oligonucleotide frequencies (Bohlin J. et al. 2008, BMC Genomics, 9:104). In some embodiments, the Tetranucleotide Signature Frequency Correlation coefficient between the variant and the deposited strain is of about 0.99, 0.999, 0.9999, 0.99999, 0.999999, 0.999999 or more.

According to an additional or alternative embodiment, the degree of relatedness between the functional homolog and the deposited strain is determined as the degree of similarity obtained when analyzing the genomes of the parent and of the variant strain by Pulsed-field gel electrophoresis (PFGE) using one or more restriction endonucleases. The degree of similarity obtained by PFGE can be measured by the Dice similarity coefficient. In some embodiments, the Dice similarity coefficient between the variant and the deposited strain is of at least about 96 %, at least about 97 %, at least about 98 %, at least about 99 %, at least about 99.1 %, at least about 99.5 %, at least about 99.6 %, at least about 99.7 %, at least about 99.8 %, at least about 99.9 %, at least about 99.99 %, at least about 99.999 %, at least about 99.9999 %, at least about 99.99999 %, at least about 99.999999 % or more.

According to an additional or alternative embodiment, the functional homolog is defined as having the same ribotype, as obtained using any of the methods known in the art and described, for instance, by Bouchet et al. (Clin. Microbiol. Rev., 2008, 21:262-273).

According to an additional or alternative embodiment, the degree of relatedness between the functional homolog and the deposited strain is determined by the Pearson correlation coefficient obtained by comparing the genetic profiles of both strains obtained by repetitive extragenic palindromic element-based PCR (REP-PCR) (see e.g. Chou and Wang, Int J Food Microbiol. 2006, 110:135-48). In some embodiments, the Pearson correlation coefficient obtained by comparing the REP-PCR profiles of the variant and the deposited strain is of at least about 0.99, at least about 0.999, at least about 0.9999, at least about 0.99999, at least about 0.999999, at least about 0.999999 or more.

According to an additional or alternative embodiment, the degree of relatedness between the functional homolog and the deposited strains is defined by the linkage distance obtained by comparing the genetic profiles of both strains obtained by Multilocus sequence typing (MLST) (see e.g. Maiden, M. C., 1998, Proc. Natl. Acad. Sci. USA 95:3140-3145). In some embodiments, the linkage distance obtained by MLST of the functional homolog and the deposited strain is of at least about 0.99, at least about 0.999, at least about 0.9999, at least about 0.99999, at least about 0.999999, at least about 0.999999 or more.

According to an additional or alternative embodiment, the functional homolog comprises a functionally conserved gene or a fragment thereof e.g., a house-keeping gene e.g., 16S-rRNA or Internal Transcribed Spacer" (ITS), *recA, glnII, atpD*, *gap, glnA, gltA, gyrB*, *pnp, rpoB, thrC* or *dnaK* that is at least about 97 %, at least about 98 %, at least about 99 %, at least about 99.1 %, at least about 99.5 %, at least about 99.6 %, at least about 99.7 %, at least about 99.8 %, at least about 99.9 %, at least about 99.99 %, at least about 99.999 %, at least about 99.9999 %, at least about 99.99999 %, at least about 99.999999 % or more identical to that of the deposited strain.

As mentioned, and according to a specific additional or an alternative embodiment, a functional homolog can also be determined on the basis of a multilocus sequence analysis (MLSA) determination of various functionally conserved genes or fragments thereof e.g., at least one, 2, 3, 4, 5, 6, 7, 8, 9, 10, 11, 12, 13, 14, 15 or more functionally conserved genes or fragments thereof, such as of e.g., *16S, ITS, recA, glnII, atpD*, *gap, glnA, gltA, gyrb*, *pnp, rpoB, thrC and dnaK.*

According to a specific embodiment, the bacterial strain comprises more than one 16S-rRNA (e.g., 2, see Table 44).

According to a specific embodiment, the 16S ribosomal RNA (16S-rRNA) nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain (see Table 44 and sequences therein).

According to a specific embodiment, the ITS nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *recA* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *atpD* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *dnaK* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *glnII* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *gap* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *glnA* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *gltA* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *gyrB* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *pnp* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *rpoB* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the *thrC* nucleic acid sequence is at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, at least about 99.95%, at least about 99.999 %, at least about 99.9999 %, at least about 99.99999 %, at least about 99.999999 % or more identical to that of the deposited strain.

According to a specific embodiment, the genomic nucleic acid sequence comprises at least one sub-genomic sequence (marker), at least 2 sub-genomic sequences, at least 3 sub-genomic sequences, at least 4 sub-genomic sequences or at least 5 sub-genomic sequences, which are at least about 95 %, at least about 95.5 %, at least about 96 %, at least about 96.5 %, at least about 97 %, at least about 97.1 %, at least about 97.2 %, at least about 97.3 %, at least about 97.4 %, at least about 97.5 %, at least about 97.6 %, at least about 97.7 %, at least about 97.8 %, at least about 97.9 %, at least about 98 %, at least about 98.1 %, at least about 98.2 %, at least about 98.3 %, at least about 98.4 %, at least about 98.5 %, at least about 98.6 %, at least about 98.7 %, at least about 98.8 %, at least about 98.9 %, at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.8 %, at least about 99.9 %, 99.95%, at least about 99.99 %, at least about 99.999 %, at least about 99.9999 %, at least about 99.99999 %, at least about 99.999999 % or more identical to that of those listed in Table 60.

According to an additional or alternative embodiment, the deposited strain and the functional homolog is characterized by substantially the same (+/- about 10 %, 20 %, 40 %, 50 %, 60 % when tested under the same conditions) biochemical profiling (e.g., biochemical fingerprinting) using for example, the GEN III redox chemistry (BIOLOG Inc. 21124 Cabot Blvd. Hayward CA, 94545, USA), which can analyze both Gram-negative and Gram-positive bacteria, for their ability to metabolize all major classes of biochemicals, in addition to determining other important physiological properties such as pH, salt, and lactic acid tolerance. Further details can be obtained in "Modern Phenotypic Microbial Identification", B.R. Bochner, Encyclopedia of Rapid Microbiological Methods, 2006, v.2, Ch. 3, pp. 55-73, which is incorporated herein by reference in its entirety.

According to an additional or alternative embodiment, the functional homolog is defined by a comparison of coding sequences (gene) order.

According to an additional or alternative embodiment, the functional homolog is defined by a comparison of order of non-coding sequences.

According to an additional or alternative embodiment, the functional homolog is defined by a comparison of order of coding and non-coding sequences.

According to some embodiments of the invention, the combined coding region of the functional homolog is such that it maintains the original order of the coding regions as within the genomic sequence of the bacterial isolate yet without the non-coding regions.

For example, in case the genomic sequence has the following coding regions, A, B, C, D, E, F, G, each flanked by non-coding sequences (e.g., regulatory elements, introns and the like), the combined coding region will include a single nucleic acid sequence having the A+B+C+D+E+F+G coding regions combined together while maintaining the original order of their genome, yet without the non-coding sequences.

According to some embodiments of the invention, the combined non-coding region of the functional homolog is such that it maintains the original order of the non-coding regions as within the genomic sequence of the bacterial isolate yet without the coding regions as originally present in the bacterial deposit.

According to some embodiments of the invention, the combined non-coding region and coding region (i.e., the genome) of the functional homolog is such that it maintains the original order of the coding and non-coding regions as within the genomic sequence of the microbial deposit.

As used herein, "maintains" relates to at least about 90 %, 91 %, 92 %, 93 %, 94 %, 95 %, 96 %, 97 %, 98 %, 99 % or 100 % maintained as compared to the deposited strain.

According to an additional or alternative embodiment, the functional homolog is defined by a comparison of gene content.

According to a specific embodiment, the functional homolog comprises a combined coding region where at least about 90 %, at least about 91 %, at least about 92 %, at least about 93 %, at least about 94 %, at least about 95 %, at least about 96 %, at least about 97 %, at least about 98 %, at least about 99 %, or more (*e.g.,* 100%) is identical to a combined coding region existing in the genome of the deposited strain.

As used herein "combined coding region" refers to a nucleic acid sequence including all of the coding regions of the bacterial isolate, yet without the non-coding regions of the bacterial isolate.

According to an additional or alternative embodiment, the functional homolog is defined by a comparison of nucleotide composition and codon usage.

According to an additional or alternative embodiment, the functional homolog is defined by a method based on random genome fragments and DNA microarray technology. These methods are of sufficiently high resolution for strain-to-species level identification.

One of ordinary skill in the art, based on knowledge of the classification criteria, would know how to identify strains that are considered functional homologs.

An additional and more detailed description of species-to-strain classification can be found in:
Cho and Tiedje 2001 Bacterial species determination from DNA-DNA hybridization by using genome fragments and DNA microarrays;
Coenye et al. 2005 Towards a parokaryotic genomic taxonomy. FEMS Microbiol. Rev. 29:147-167;
Konstantinidis and Tiedje (2005) Genomic insights that advance the species definition for prokaryotes. Proc. Natl. Acad. Sci. USA 102:189-197;
Konstantinidis et al. 2006 Toward a more robust assessment of intraspecies diversity using fewer genetic markers. Appl. Environ. Microbiol. 72:7286-7293.

It is to be understood that one or more methods as described herein can be used to identify a functional homolog.

Genomic data can be obtained by methods which are well known in the art, e.g., DNA sequencing, bioinformatics, electrophoresis, an enzyme-based mismatch detection assay and a hybridization assay such as PCR, RT-PCR, RNase protection, in-situ hybridization, primer extension, Southern blot, Northern Blot and dot blot analysis.

According to a specific embodiment, the functional homolog and the deposited strain belong to the same genus.

According to a specific embodiment, the functional homolog and the deposited strain belong to the same species.

According to a specific embodiment, the functional homolog and the deposited strain belong to the same sub-species.

As used herein, "preparation" refers to an isolate of bacteria in which the prevalence (i.e., concentration) of the microbial stain or functional homolog is enriched over that (exceeds that) found in nature. In nature, the microbial strain is typically part of the plant microbiome, consisting of more than thousands of microbial species. According to some embodiments of the invention, the preparation comprises less than 50, 20, 10, 9, 8, 7, 6, 5, or 4 microbial species, e.g., bacteria and fungi.

According to a specific embodiment, the microbial preparations comprises 1, 2, 3, 4, 5, 6, 7, 8, 9 or 10 microbial species.

According to a specific embodiment, the preparation comprises the microbial strain at a level of purity of at least about 20 %, at least about 30 %, at least about 40 %, at least about 50 %, at least about 60 %, at least about 70 %, at least about 80 %, at least about 85 %, at least about 90 %, at least about 91 %, at least about 92 %, at least about 93 %, at least about 94 %, at least about 95 % or more, say 100 % pure.

According to a specific embodiment, the preparation comprises the microbial strain at a level of purity of at least about 99 %, at least about 99.1 %, at least about 99.2 %, at least about 99.3 %, at least about 99.4 %, at least about 99.5 %, at least about 99.6 %, at least about 99.7 %, at least about 99.8 %, at least about 99.9 %, at least about 99.95 %, at least about 99.99 %, at least about 99.99 %, at least about 99.999 % or more, say 100 % pure.

According to a specific embodiment, the microbial strain comprises viable microbial cells (capable of replicating).

According to a specific embodiment, the microbial strain comprises sporulating microbes.

A "spore" or "spores" refers to microbes that are generally viable, more resistant to environmental influences such as heat and bactericidal or fungicidal agents than other forms of the same microbial species, and typically capable of germination and out-growth. Bacteria and fungi that are "capable of forming spores" are those bacteria and fungi comprising the genes and other necessary abilities to produce spores under suitable environmental conditions.

As used herein, "enriched" refers to 2-10,000,000 fold enrichment over that found in nature in an isolate of a microbiome of a plant comprising the deposited strain or a functional homolog of same.

As used in here, the phrase "CFUs" or "Colony Forming Units" refers to the number of microbial cells in a defined sample (e.g. milliliter of liquid, square centimeter of surface, one seed of grain, etc.) that form colonies and thereafter numbered, on a semi-solid bacteriological growth medium.

According to specific embodiments, the enrichment in the composition e.g., preparation, formulation, coated seed is 10² CFUs-10⁹ CFUs/ seed or 10² CFUs-10⁹ CFUs/ gr powder or 10² CFUs-10⁹ CFUs/ ml.

According to specific embodiments, the enrichment in the composition e.g., preparation, formulation, coated seed is 10² CFUs-10⁸ CFUs/ seed or 10² CFUs-10⁸ CFUs/ gr powder or 10² CFUs-10⁸ CFUs/ ml.

According to specific embodiments, the enrichment in the composition e.g., preparation, formulation, coated seed is 10² CFUs-10⁷ CFUs/ seed or 10² CFUs-10⁷ CFUs/ gr powder or 10² CFUs-10⁷ CFUs/ ml.

According to specific embodiments, the enrichment in the composition e.g., preparation, formulation, coated seed is 10² CFUs-10⁶ CFUs/ seed or 10² CFUs-10⁶ CFUs/ gr powder or 10² CFUs-10° CFUs/ ml.

According to specific embodiments, the enrichment in the composition e.g., preparation, formulation, coated seed is 10² CFUs-10⁵ CFUs/ seed or 10² CFUs-10⁵ CFUs/ gr powder or 10² CFUs-10⁵ CFUs/ ml.

According to specific embodiments, the enrichment in the composition e.g., preparation, formulation, coated seed is 10² CFUs-10⁴ CFUs/ seed or 10² CFUs-10⁴ CFUs/ gr powder or 10² CFUs-10⁴ CFUs/ ml.

According to specific embodiments, the enrichment in the composition e.g., preparation, formulation, coated seed is 10² CFUs-10³ CFUs/ seed or 10² CFUs-10³ CFUs/ gr powder or 10² CFUs-10³ CFUs/ ml.

According to specific embodiments, the enrichment in the composition e.g., preparation, formulation, coated seed is 10³ CFUs-10⁹ CFUs/ seed or 10³ CFUs-10⁹ CFUs/ gr powder or 10³ CFUs-10⁹ CFUs/ ml.

According to specific embodiments, the enrichment in the composition e.g., preparation, formulation, coated seed is 10⁴ CFUs-10⁹ CFUs/ seed or 10⁴ CFUs-10⁹ CFUs/ gr powder or 10⁴ CFUs-10⁹ CFUs/ ml.

According to specific embodiments, the enrichment in the composition e.g., preparation, formulation, coated seed is 10⁵ CFUs-10⁹ CFUs/ seed or 10⁵ CFUs-10⁹ CFUs/ gr powder or 10⁵ CFUs-10⁹ CFUs/ ml.

According to specific embodiments, the enrichment in the composition e.g., preparation, formulation, coated seed is 10⁶ CFUs-10⁹ CFUs/ seed or 10⁶ CFUs-10⁹ CFUs/ gr powder or 10⁶ CFUs-10⁹ CFUs/ ml.

According to specific embodiments, the enrichment in the composition e.g., preparation, formulation, coated seed is 10⁷ CFUs-10⁹ CFUs/ seed or 10⁷ CFUs-10⁹ CFUs/ gr powder or 10⁷ CFUs-10⁹ CFUs/ ml.

According to specific embodiments, the enrichment in the composition e.g., preparation, formulation, coated seed is 10⁸ CFUs-10⁹ CFUs/ seed or 10⁸ CFUs-10⁹ CFUs/ gr powder or 10⁸ CFUs-10⁹ CFUs/ ml.

According to specific embodiments, the enrichment in the composition e.g., preparation, formulation, coated seed is 10⁸ CFUs-10⁹ CFUs/ seed or 10⁸ CFUs-10⁹ CFUs/ gr powder or 10⁸ CFUs-10⁹ CFUs/ ml.

According to a specific embodiment the preparation comprises at least about 100 CFU or spores, at least about 10² CFUs/seed CFUs/gr or CFUs/ml, at least about 10³ CFUs/seed CFUs/gr or CFUs/ml, at least about 10⁴ CFUs/seed CFUs/gr or CFUs/ml, at least about 10⁵ CFUs/seed CFUs/gr or CFUs/ml, at least about 10⁶ CFUs/seed CFUs/gr or CFUs/ml, at least about 10⁷ CFUs/seed CFUs/gr or CFUs/ml, at least about 10⁸ CFUs/seed CFUs/gr or CFUs/ml, at least about 10⁹ CFUs/seed CFUs/gr or CFUs/ml.

According to a specific embodiment, the preparation is selected from the group consisting of a still culture, whole cultures stored stock of cells (particularly glycerol stocks), agar strip, stored agar plug in glycerol/water, freeze dried stock, and dried stocks such as lyophilisate dried onto filter paper or grain seed.

As used herein "a culture" refers to a fluid, pellet, scraping, dried sample, lyophilisate or a support, container, or medium such as a plate, paper, filter, matrix, straw, pipette or pipette tip, fiber, needle, gel, swab, tube, vial, particle, etc. that contains the deposited strain or the functional homolog thereof in an amount that exceeds that found in nature, as described hereinabove. In the present invention, an isolated culture of a microbial strain is a culture fluid or a scraping, pellet, dried preparation, lyophilisate, or a support, container, or medium that contains the microorganism, in the absence of other organisms.

According to a specific embodiment, the microbial strain or functional homolog thereof improves an agricultural trait of a cultivated plant heterologous to the deposited strain or the functionally homologous strain as compared to a control plant not treated with the microbial strain or the functionally homologous strain.

The term "control plant", "reference plant" refers to an agricultural plant or portion thereof of the same cultivar to which a treatment, formulation, composition or preparation as described herein is not administered/contacted. A reference agricultural plant therefore, is identical to the treated plant with the exception of the presence of the microbial strain as described herein and can serve as a control for detecting the effects of the microbial strain that is conferred to the plant. In some embodiments, the reference plant is an isoline plant and is referred to as a "control isoline plant" or "reference isoline plant".

The term "isoline" is a comparative term, relating to comparisons made among one or more groups of organisms that are substantially epigenetically and genetically identical and are grown in conditions which differ only in an experimental condition or treatment. In the present case the difference would be in the heterologous application of the microbial strain on a plant. Any differences between the plants derived from those seeds/leafs/plantlets when grown or stored in identical conditions may be attributed to the microbial treatment, thus forming an isoline comparison.

**In** some embodiments, a comparison is made between groups of organisms (e.g., plants) wherein each group includes at least 5 organisms, between 5 and 10 organisms, at least 10 organisms, between 10 and 100 organisms, for example, at least 100 organisms, between 100 and 300 organisms, at least 300 organisms, between 300 and 1,000 organisms, at least 1,000 organisms, between 1,000 and 3,000 organisms, at least 3,000 organisms, between 3,000 and 10,000 organisms, at least 10,000 organisms, between 10,000 and 30,000 organisms, at least 30,000 organisms, between 30,000 and 100,000 organisms, at least 100,000 organisms or more.

As used in here, the phrase "agricultural trait" refers to a characteristic of a plant that once improved, may lead to an increase in plant yield. For example, improved photosynthetic capacity may lead to an increased yield. As used in here, the phrase "response" refers to a measurable element of a plant that is used to determine if a plant trait is improved or not. For example, the plant trait photosynthetic capacity is determined as 'improved' if the chlorophyll level, as measured using the SPAD instrument (Knighton N. and Bugbee B., 2018, A Comparison of Opti-Sciences CCM-200 Chlorophyll Meter and the Minolta SPAD 502 Chlorophyll Meter. Crop Physiology Laboratory-Utah State University), is higher.

As used herein the phrase "plant yield" refers to the amount (e.g., as determined by weight or size) or quantity (numbers) of sealable tissues or organs produced per plant or per growing season. Hence, increased yield could affect the economic benefit one can obtain from the plant in a certain growing area and/or growing period.

Plant yield can be affected by other agricultural traits including, but not limited to, early vigor and biomass establishment, biomass accumulation up to VT, stem conductance, transpiration rate, photosynthetic capacity, reduced anthesis-silking interval (ASI), longer grain filling duration, increased assimilate partitioning, increased kernel number per plant, main ear size and kernel volume and weight. These agricultural traits can be measured by responses related to, but not limited to, plant biomass, plant growth rate, seed yield, seed or grain quantity, number of flowers (florets) per panicle (expressed as a ratio of number of filled seeds over number of primary panicles), harvest index, number of plants grown per area, number and size of harvested organs per plant and per area, number of plants per growing area (density), number of harvested organs in field, total leaf area, carbon assimilation and carbon partitioning (the distribution/ allocation of carbon within the plant), number of harvestable organs (e.g. seeds), seeds per pod, weight per seed and modified plant architecture such as increase stalk diameter, thickness or improvement of physical properties such as elasticity.

As used herein, the phrase "seed yield" refers to the number or weight of the seeds per plant, pod or spike weight, seeds per pod, or per growing area or to the weight of a single seed. Hence seed yield can be affected by seed dimensions (e.g., length, width, perimeter, area and/ or volume), number of (filled) seeds and seed filling rate and by seed oil content. Hence, increased seed yield per plant could affect the economic benefit one can obtain from the plant in a certain growing area and/ or growing time and increase in seed yield per growing area can be achieved by:
1. Increasing seed yield per plant, and/ or by
2. Increasing number of plants grown on the same given area and/ or by
3. Increasing harvest index (seed yield per the total biomass).

The term "seed" (also referred to as "grain" or "kernel") as used herein refers to a small embryonic plant enclosed in a covering called the seed coat (usually with some stored food), the product of the ripened ovule of gymnosperm and angiosperm plants which occurs after fertilization and some growth within the mother plant.

As used herein the phrase "plant biomass" refers to the amount (e.g., measured in grams of air-dry tissue) of a tissue produced from the plant in a growing season, which could also determine or affect the plant yield or the yield per growing area. An increase in plant biomass can be in the whole plant or in parts thereof such as aboveground (harvestable) parts, vegetative biomass, leaf size or area, leaf thickness, roots and seeds.

It should be noted that an increase in plant's dry weight, shoot dry weight, shoot fresh weight, vegetative dry weight, and/ or total dry matter per plant indicates an increased biomass as compared to a matching control plant under the same growth conditions.

As used herein the term "plant growth stages" refers to the plant phenology or development stages based on common methods. Knowledge of the plant growth process provides the means to enhance the crop (corn, wheat, etc.). Plant symptoms occurring during certain growth stages help the grower determine the cause and effect of a deficiency, disease or other crop problem and take timely measures. There are few common methods, all of them refer to number of leaves. For corn, wheat and *Brachypodium* we use the "Droopy" Leaf Method - Like the leaf collar method, this method of leaf staging begins with the short first leaf. Leaf counting then differs, though, by ending not with the uppermost leaf with a visible collar, but at that leaf that is at least 40 to 50 percent exposed from the whorl. In knee-high corn or older, the tip of this "indicator" leaf typically also "droops" or hangs down, hence the name "droopy" leaf method.

Vegetative Stages:
- VE (emergence)
- V1 (first leaf)
- V2 (second leaf)
- V3 (third leaf)
- V(n) (nth leaf)
- VT (tasseling)

Reproductive Stages:
- R1 (silking)
- R2 (blister)
- R3 (milk)
- R4 (dough)
- R5 (dent)
- R6 (physiological maturity)

Additionally or alternatively, the root biomass can be indirectly determined by measuring root coverage, root density and/or root length of a plant.

It should be noted that plants having a larger root coverage exhibit higher fertilizer (e.g., nitrogen) use efficiency and/or higher water use efficiency as compared to plants with a smaller root coverage.

As used herein the phrase "root coverage" refers to the total area or volume of soil or of any plant-growing medium encompassed by the roots of a plant.

According to some embodiments of the invention, the root coverage is the minimal convex volume encompassed by the roots of the plant.

It should be noted that since each plant has a characteristic root system, e.g., some plants exhibit a shallow root system (e.g., only a few centimeters below ground level), while others have a deep in soil root system (e.g., a few tens of centimeters or a few meters deep in soil below ground level), measuring the root coverage of a plant can be performed in any depth of the soil or of the plant-growing medium, and comparison of root coverage between plants of the same species (e.g., plant inoculated with bacteria of some embodiments of the invention and a control plant) should be performed by measuring the root coverage in the same depth.

As used herein the phrase "root length" refers to the total length of the longest root of a single plant.

As used herein the phrase "growth rate" refers to the increase in plant organ/tissue size per time (can be measured in cm² per day, cm/day or mm/day).

As used herein the phrase "photosynthetic capacity" (also known as "Aₘₐₓ") is a measure of the maximum rate at which leaves are able to fix carbon during photosynthesis. It is typically measured as the amount of carbon dioxide that is fixed per square meter per second, for example as µmol m⁻² sec⁻¹. Plants are able to increase their photosynthetic capacity by several modes of action, such as by increasing the total leaves area (e.g., by increase of leaves area, increase in the number of leaves, and increase in plant's vigor, e.g., the ability of the plant to grow new leaves along a time course) as well as by increasing the ability of the plant to efficiently execute carbon fixation in the leaves. Hence, the increase in total leaves area can be used as a reliable measurement parameter for photosynthetic capacity increment.

As used herein the phrase "plant vigor" refers to the amount (measured by weight) of tissue produced by the plant in a given time. Hence increased vigor could determine or affect the plant yield or the yield per growing time or growing area. **In** addition, early vigor (seed and/or seedling) results in improved field stand.

Improving early vigor is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigor. Where drill-seeding is practiced, longer mesocotyls and coleoptiles are important for good seedling emergence. The ability to engineer early vigor into plants would be of great importance in agriculture. For example, poor early vigor has been a limitation to the introduction of maize (*Zea mays* L.) hybrids based on Corn Belt germplasm in the European Atlantic.

As used herein the phrase "Harvest index" refers to the efficiency of the plant to allocate assimilates and convert the vegetative biomass into reproductive biomass such as fruit and seed yield.

Harvest index is influenced by yield component, plant biomass and indirectly by all tissues participant in remobilization of nutrients and carbohydrates in the plants such as stem width, rachis width and plant height. Improving harvest index will improve the plant reproductive efficiency (yield per biomass production) hence will improve yield per growing area. The Harvest Index can be calculated using: Grain yield per plant divided by the total dry matter per plant.

It should be noted that an increase in 1000 grain weight, plant height, inflorescence node number, grain number, spikelet's dry matter per plant, total grain yield per plant and/or rachis diameter in a transformed plant expressing an exogenous polynucleotide encoding the polypeptide of some embodiments of the invention indicates the ability of the polypeptide to increase the harvest index of the transformed plant as compared to a control, non-transformed plant, under the same growth conditions.

As used herein the phrase "Grain filling period" refers to the time in which the grain or seed accumulates the nutrients and carbohydrates until seed maturation (when the plant and grains/seeds are dried).

Grain filling period is measured as number of days from flowering/heading until seed maturation. Longer period of "grain filling period" can support remobilization of nutrients and carbohydrates that will increase yield components such as grain/seed number, 1000 grain/seed weight and grain/seed yield.

As used herein the phrase "heading" or "time to heading" which is interchangeably used herein, refers to the time from germination to the time when the first head immerges.

It should be noted that a shorter time to heading (i.e., a negative increment in the measured time to heading) in a plant enables the plant a longer time period for grain filling.

Thus, a shorter time to heading in plant inoculated with bacteria of some embodiments of the invention indicates the ability of the bacteria to increase the grain-filling period in the plant as compared to a control, under the same growth conditions.

As used herein the phrase "flowering" or "time to flowering" which is interchangeably used herein, refers to the time from germination to the time when the first flower is open.

As used herein the phrase "increasing early flowering" refers to increasing the ability of the plant to exhibit an early flowering as compared to a matching control plant (e.g., a non-inoculated plant under the same growth conditions). Additionally or alternatively, increasing early flowering of a population of plants indicates increasing the number or percentage of plants having an early flowering.

It should be noted that increasing the ability of the plant to exhibit an early flowering of a plant (i.e., a shorter time period between germination to the time in which the first flower opens) is advantageous since it enables the plant to produce more flowers, fruits, pods and seeds without changing plant maturity period, which eventually leads to increased biomass and yield of the plant.

It should be noted that increasing the ability of the plant to exhibit an early flowering along with a longer grain-filling period is advantageous to the plant since it supports a higher yield of the plant.

As used herein the phrase "plant height" refers to measuring plant height as indication for plant growth status, assimilates allocation and yield potential. In addition, plant height is an important trait to prevent lodging (collapse of plants with high biomass and height) under high density agronomical practice.

Plant height is measured in various ways depending on the plant species but it is usually measured as the length between the ground level and the top of the plant, e.g., the head or the reproductive tissue.

According to a specific embodiment, examples of an agricultural trait include, but are not limited to, biotic stress tolerance and abiotic stress tolerance.

According to a specific embodiment the agricultural traits include, but are not limited to, germination rate, disease resistance, heat tolerance, drought tolerance, water use efficiency, cold tolerance, salinity tolerance, metal tolerance, herbicide tolerance, chemical tolerance, nitrogen utilization, nutrient utilization, resistance to nitrogen stress, nitrogen fixation, pathogen resistance, insect resistance, yield, yield under water-limited conditions, grain weight, fruit weight, kernel moisture content, number of ears, number of kernels per ear, health enhancement, vigor, growth, photosynthetic capability, nutrition enhancement, altered protein content, altered oil content, biomass, root biomass, root length, root surface area, root architecture, shoot length, shoot height, shoot biomass, seed weight, seed carbohydrate composition, seed oil composition, number of pods, delayed senescence, stay-green, seed protein composition, dry weight of mature seeds, fresh weight of mature seeds, number of mature seeds per plant, number of flowers per plant, chlorophyll content, rate of photosynthesis, number of leaves, number of pods per plant, length of pods per plant, number of wilted leaves per plant, number of severely wilted leaves per plant, number of non-wilted leaves per plant, resistance to a fungal pathogen, resistance to a bacterial pathogen, resistance to a viral pathogen, resistance to a nematode, a detectable modulation in the level of a metabolite, a detectable modulation in gene expression, and a detectable modulation in the proteome, and combinations thereof.

It should be noted that an agricultural trait such as those described herein [e.g., yield, growth rate, biomass, vigor, harvest index, grain-filling period, flowering, heading, plant height, photosynthetic capacity, fertilizer use efficiency (e.g., nitrogen use efficiency), early flowering, grain filling period, harvest index, plant height] can be determined under stress (e.g., abiotic stress, nitrogen-limiting conditions) and/or non-stress (normal) conditions.

According to a specific embodiment, the agricultural trait is determined under drought conditions.

As used herein, the phrase "non-stress conditions" or "normal conditions" refers to the growth conditions (e.g., water, temperature, light-dark cycles, humidity, salt concentration, fertilizer concentration in soil, nutrient supply such as nitrogen, phosphorous and/or potassium), that do not significantly go beyond the everyday climatic and other abiotic conditions that plants may encounter, and which allow optimal growth, metabolism, reproduction and/or viability of a plant at any stage in its life cycle (e.g., in a crop plant from seed to a mature plant and back to seed again). Persons skilled in the art are aware of normal soil conditions and climatic conditions for a given plant in a given geographic location. It should be noted that while the non-stress conditions may include some mild variations from the optimal conditions (which vary from one type/species of a plant to another), such variations do not cause the plant to cease growing without the capacity to resume growth.

For example, normal conditions for growing corn include irrigation with about 452,000 liter water per 1000 square meters and fertilization with about 14 units nitrogen per 1000 square meters per growing season.

Normal conditions for growing cotton include irrigation with about 580,000 liter water per 1000 square meters and fertilization with about 24 units nitrogen per 1000 square meters per growing season.

Normal conditions for growing bean include irrigation with about 524,000 liter water per 1000 square meters and fertilization with about 16 units nitrogen per 1000 square meters per growing season.

The phrase "abiotic stress" as used herein refers to any adverse effect on metabolism, growth, reproduction and/or viability of a plant. Accordingly, abiotic stress can be induced by suboptimal environmental growth conditions such as, for example, salinity, osmotic stress, water deprivation, drought, flooding, freezing, low or high temperature, heavy metal toxicity, anaerobiosis, nutrient deficiency (e.g., nitrogen deficiency or limited nitrogen), atmospheric pollution or UV irradiation. The implications of abiotic stress are discussed in the Background section.

The phrase "abiotic stress tolerance" as used herein refers to the ability of a plant to endure an abiotic stress without suffering a substantial alteration in metabolism, growth, productivity and/or viability.

Plants are subject to a range of environmental challenges. Several of these, including salt stress, general osmotic stress, drought stress and freezing stress, have the ability to impact whole plant and cellular water availability. Not surprisingly, then, plant responses to this collection of stresses are related. Zhu et al. (2002) Ann. Rev. Plant Biol. 53: 247-273, noted that "most studies on water stress signaling have focused on salt stress primarily because plant responses to salt and drought are closely related and the mechanisms overlap". Many examples of similar responses and pathways to this set of stresses have been documented. For example, the CBF transcription factors have been shown to condition resistance to salt, freezing and drought (Kasuga et al. (1999) Nature Biotech. 17: 287-291). The Arabidopsis rd29B gene is induced in response to both salt and dehydration stress, a process that is mediated largely through an ABA signal transduction process (Uno et al. (2000) Proc. Natl. Acad. Sci. USA 97: 11632-11637), resulting in altered activity of transcription factors that bind to an upstream element within the rd29B promoter. In *Mesembryanthemum crystallinum* (ice plant), Patharker and Cushman have shown that a calcium-dependent protein kinase (McCDPK1) is induced by exposure to both drought and salt stresses (Patharker and Cushman (2000) Plant J. 24: 679-691). The stress-induced kinase was also shown to phosphorylate a transcription factor, presumably altering its activity, although transcript levels of the target transcription factor are not altered in response to salt or drought stress. Similarly, Saijo et al. demonstrated that a rice salt/drought-induced calmodulin-dependent protein kinase (OsCDPK7) conferred increased salt and drought tolerance to rice when overexpressed (Saijo et al. (2000) Plant J. 23: 319-327).

Exposure to dehydration invokes similar survival strategies in plants as does freezing stress (see, for example, Yelenosky (1989) Plant Physiol 89: 444-451) and drought stress induces freezing tolerance (see, for example, Siminovitch et al. (1982) Plant Physiol 69: 250-255; and Guy et al. (1992) Planta 188: 265-270). In addition to the induction of cold-acclimation proteins, strategies that allow plants to survive in low water conditions may include, for example, reduced surface area, or surface oil or wax production. In another example increased solute content of the plant prevents evaporation and water loss due to heat, drought, salinity, osmoticum, and the like therefore providing a better plant tolerance to the above stresses.

It will be appreciated that some pathways involved in resistance to one stress (as described above), will also be involved in resistance to other stresses, regulated by the same or homologous genes. Of course, the overall resistance pathways are related, not identical, and therefore not all genes controlling resistance to one stress will control resistance to the other stresses. Nonetheless, if a gene conditions resistance to one of these stresses, it would be apparent to one skilled in the art to test for resistance to these related stresses. Methods of assessing stress resistance are further provided in the Examples section which follows.

As used herein, the phrase "drought conditions" or "water limited conditions" refers to growth conditions with limited water availability. It should be noted that in assays used for determining the tolerance of a plant to drought stress the only stress induced is limited water availability, while all other growth conditions such as fertilization, temperature and light are the same as under normal conditions.

For example drought conditions for growing Brachypodium include irrigation with 240 milliliter at about 20% of tray filled capacity in order to induce drought stress, while under normal growth conditions trays irrigated with 900 milliliter whenever the tray weight reached 50% of its filled capacity (fertilization was applied equal to each treatment). Drought effect is between 10%-30% reduction, compared to control (normal growth conditions), in grain yield.

For example drought conditions for growing Wheat, Maize, Sorghum or Barley include normal irrigation (2-3 times a week with 250 milliliter at about 80% of filled capacity) up to VT. From VT stage cycles of moderate drought treatment (220 milliliter and re-irrigating (350 milliliter) were conducted whenever the soil reached 40% of its filled capacity, while under normal growth conditions trays were always irrigated with 350 milliliter (fertilization was applied equal to each treatment). Overall water administered was 40% less compared to plants grown in normal conditions.

As used herein the phrase "water use efficiency (WUE)" refers to the level of organic matter produced per unit of water consumed by the plant, *i.e.,* the dry weight of a plant in relation to the plant's water use, e.g., the biomass produced per unit transpiration.

As used herein the phrase "fertilizer use efficiency" (FUE) refers to the metabolic process (es) which lead to an increase in the plant's yield, biomass, vigor, and growth rate per fertilizer unit applied. The metabolic process can be the uptake, spread, absorbent, accumulation, relocation (within the plant) and use of one or more of the minerals and organic moieties absorbed by the plant, such as nitrogen, phosphates and/or potassium.

As used herein the phrase "fertilizer-limiting conditions" refers to growth conditions which include a level (e.g., concentration) of a fertilizer applied which is below the level needed for normal plant metabolism, growth, reproduction and/or viability.

As used herein the phrase "nitrogen use efficiency (NUE)" refers to the metabolic process (es) which lead to an increase in the plant's yield, biomass, vigor, and growth rate per nitrogen unit applied. The metabolic process can be the uptake, spread, absorbent, accumulation, relocation (within the plant) and use of nitrogen absorbed by the plant.

As used herein the phrase "nitrogen-limiting conditions" refers to growth conditions which include a level (e.g., concentration) of nitrogen (e.g., ammonium or nitrate) applied which is below the level needed for normal plant metabolism, growth, reproduction and/or viability.

Improved plant NUE and FUE is translated in the field into either harvesting similar quantities of yield, while implementing less fertilizers, or increased yields gained by implementing the same levels of fertilizers. Thus, improved NUE or FUE has a direct effect on plant yield in the field. Thus, the polynucleotides and polypeptides of some embodiments of the invention positively affect plant yield, seed yield, and plant biomass. In addition, the benefit of improved plant NUE will certainly improve crop quality and biochemical constituents of the seed such as protein yield and oil yield.

As used herein the term "trait" refers to a characteristic or quality of a plant which may overall (either directly or indirectly) improve the commercial value of the plant.

As used herein the term "increasing" or "improving" refers to at least about 2 %, at least about 3 %, at least about 4 %, at least about 5 %, at least about 10 %, at least about 15 %, at least about 20 %, at least about 30 %, at least about 40 %, at least about 50 %, at least about 60 %, at least about 70 %, at least about 80 %, increase in the trait [e.g., yield, seed yield, biomass, growth rate, vigor, photosynthetic capacity, early flowering, grain filling period, harvest index, plant height, abiotic stress tolerance, and/or nitrogen use efficiency] of a plant as compared to a control plant (e.g., isoline plant), *i.e.,* a plant inoculated with the microbial strain or functional homolog under the same (e.g., identical) growth conditions].

As used herein "cultivated plant", "crop plant", "agricultural plant", or "plant of agronomic importance", include plants that are cultivated by humans for food, feed, fiber, construction, fuel purposes and more. The term encompasses a whole plant, a grafted plant, ancestor(s) and progeny of the plants and plant parts (also referred to herein as "a portion"), including seeds, shoots, stems, roots (including tubers), rootstock, scion, and plant cells, tissues and organs. The plant may be in any form including suspension cultures, embryos, meristematic regions, callus tissue, leaves, gametophytes, sporophytes, pollen, and microspores. Plants that are particularly useful in the methods of the invention include all plants which belong to the superfamily Viridiplantae, in particular monocotyledonous and dicotyledonous plants including a fodder or forage legume, ornamental plant, food crop, tree, or shrub selected from the list comprising *Acacia spp., Acer spp., Actinidia spp., Aesculus spp., Agathis australis, Albizia amara*, *Alsophila tricolor, Andropogon spp., Arachis spp, Areca catechu, Astelia fragrans, Astragalus cicer, Baikiaea plurijuga*, *Betula spp., Brassica spp., Bruguiera gymnorrhiza*, *Burkea africana*, *Butea frondosa*, *Cadaba farinosa*, *Calliandra spp, Camellia sinensis, Canna indica, Cannabaceae, Cannabis, Cannabis sativa, Hemp, industrial Hemp, Capsicum spp., Cassia spp., Centroema pubescens, Chacoomeles spp., Cinnamomum cassia, Coffea arabica, Colophospermum mopane, Coronillia varia*, *Cotoneaster serotina*, *Crataegus spp., Cucumis spp., Cupressus spp., Cyathea dealbata*, *Cydonia oblonga*, *Cryptomeria japonica, Cymbopogon spp., Cynthea dealbata*, *Cydonia oblonga*, *Dalbergia monetaria*, *Davallia divaricata*, *Desmodium spp., Dicksonia squarosa*, *Dibeteropogon amplectens*, *Dioclea spp, Dolichos spp., Dorycnium rectum, Echinochloa pyramidalis, Ehraffia spp., Eleusine coracana*, *Eragrestis spp., Erythrina spp., Eucalypfus spp., Euclea schimperi*, *Eulalia villosa*, *Pagopyrum spp., Feijoa sellowlana*, *Fragaria spp., Flemingia spp, Freycinetia banksli, Geranium thunbergii, GinAgo biloba, Glycine javanica*, *Gliricidia spp, Gossypium hirsutum, Grevillea spp., Guibourtia coleosperma*, *Hedysarum spp., Hemaffhia altissima*, *Heteropogon contoffus*, *Hordeum vulgare, Hyparrhenia rufa, Hypericum erectum, Hypeffhelia dissolute, Indigo incamata*, *Iris spp., Leptarrhena pyrolifolia*, *Lespediza spp., Lettuca spp., Leucaena leucocephala*, *Loudetia simplex, Lotonus bainesli, Lotus spp., Macrotyloma axillare*, *Malus spp., Manihot esculenta*, *Medicago saliva, Metasequoia glyptostroboides, Musa sapientum, Nicotianum spp., Onobrychis spp., Ornithopus spp., Oryza spp., Peltophorum africanum*, *Pennisetum spp., Persea gratissima*, *Petunia spp., Phaseolus spp., Phoenix canariensis, Phormium cookianum, Photinia spp., Picea glauca*, *Pinus spp., Pisum sativam, Podocarpus totara*, *Pogonarthria fleckii, Pogonaffhria squarrosa*, *Populus spp., Prosopis cineraria*, *Pseudotsuga menziesii, Pterolobium stellatum*, *Pyrus communis, Quercus spp., Rhaphiolepsis umbellata*, *Rhopalostylis sapida*, *Rhus natalensis, Ribes grossularia*, *Ribes spp., Robinia pseudoacacia*, *Rosa spp., Rubus spp., Salix spp., Schyzachyrium sanguineum, Sciadopitys vefficillata*, *Sequoia sempervirens*, *Sequoiadendron giganteum*, *Sorghum bicolor, Spinacia spp., Sporobolus fimbriatus*, *Stiburus alopecuroides*, *Stylosanthos humilis, Tadehagi spp, Taxodium distichum, Themeda triandra*, *Trifolium spp., Triticum spp., Tsuga heterophylla*, *Vaccinium spp., Vicia spp., Vitis vinifera, Watsonia pyramidata*, *Zantedeschia aethiopica*, *Zea* mays, amaranth, artichoke, asparagus, broccoli, Brussels sprouts, cabbage, canola, carrot, cauliflower, celery, collard greens, flax, kale, lentil, oilseed rape, okra, onion, potato, rice, soybean, straw, sugar beet, sugar cane, sunflower, tomato, squash tea, maize, wheat, barley, rye, oat, peanut, pea, lentil and alfalfa, cotton, rapeseed, canola, pepper, sunflower, tobacco, eggplant, eucalyptus, a tree, an ornamental plant, a perennial grass and a forage crop. Alternatively algae and other non-Viridiplantae can be used for the methods of the present invention.

According to some embodiments of the invention, the cultivated plant is rice, maize, wheat, barley, peanut, potato, sesame, olive tree, palm oil, banana, soybean, sunflower, canola, sugarcane, alfalfa, millet, leguminosae (bean, pea), flax, lupinus, rapeseed, tobacco, poplar or cotton.

According to some embodiments of the invention the plant is a dicotyledonous plant.

According to some embodiments of the invention the plant is a monocotyledonous plant.

According to a specific embodiment the monocotyledonous plant includes a monocotyledonous species such as: maize (Zea mays), common wheat (Triticum aestivum), spelt (Triticum spelta), einkorn wheat (Triticum monococcum), emmer wheat (Triticum dicoccum), durum wheat (Triticum durum), Asian rice (Oryza sativa), African rice (Oryza glabaerreima), wild rice (Zizania aquatica, Zizania latifolia, Zizania palustris, Zizania texana), barley (Hordeum vulgare), Sorghum (Sorghum bicolor), Finger millet (Eleusine coracana), Proso millet (Panicum miliaceum), Pearl millet (Pennisetum glaucum), Foxtail millet (Setaria italica), Oat (Avena sativa), Triticale (Triticosecale), rye (Secale cereal), Russian wild rye (Psathyrostachys juncea), bamboo (Bambuseae), or sugarcane (e.g., Saccharum arundinaceum, Saccharum barberi, Saccharum bengalense, Saccharum edule, Saccharum munja, Saccharum officinarum, Saccharum procerum, Saccharum ravennae, Saccharum robustum, Saccharum sinense, or Saccharum spontaneum);

According to a specific embodiment, the dicotyledonous plant includes the dicotyledonous species such as: soybean (Glycine max), canola and rapeseed cultivars (Brassica napus), cotton (genus Gossypium), alfalfa (Medicago sativa), cassava (genus Manihot), potato (Solanum tuberosum), tomato (Solanum lycopersicum), pea (Pisum sativum), chick pea (Cicer arietinum), lentil (Lens culinaris), flax (Linum usitatissimum) or many varieties of vegetables.

According to a specific embodiment, the cultivated plant is of the *Gramineae* family.

As used herein "heterologous" refers to the relationship between the microbial strain and the plant (including plant parts as described hereinabove) or growth medium to which it has been applied. In a heterologous relation, the microbial strain or functional homolog is found in or on a plant or part thereof in a manner that is not naturally occurring. In some embodiments, such a manner is contemplated to include: the presence of the microbial strain or functional homolog; presence of the microbial strain or functional homolog in a different number, concentration, or amount; the presence of the microbial strain or functional homolog in or on a different plant part, tissue, cell type, or other physical location in or on the plant; the presence of the microbial strain or functional homolog at different time period, e.g. developmental phase of the plant or plant part, time of day, time of season, and combinations thereof. In some embodiments, plant growth medium is soil, a hydroponic apparatus, or artificial growth medium such as commercial potting mix. In some embodiments, the plant growth medium is soil in an agricultural field. In some embodiments, the plant growth medium is commercial potting mix. In some embodiments, the plant growth medium is an artificial growth medium such as germination paper. As a non-limiting example, if the plant has a microbial strain or functional homolog normally found in the root tissue but not in the leaf tissue, and the microbial strain or functional homolog is applied to the leaf, the microbial strain or functional homolog would be considered to be heterologously applied/contacted. As a non-limiting example, if the microbial strain or functional homolog is naturally found in the mesophyll layer of leaf tissue but is applied to the epithelial layer, the microbial strain or functional homolog would be considered to be heterologously applied/contacted. As a non-limiting example, a microbial strain or functional homolog is heterologously applied/contacted at a concentration that is at least 1.5 times, between 1.5 and 2 times, 2 times, between 2 and 3 times, 3 times, between 3 and 5 times, 5 times, between 5 and 7 times, 7 times, between 7 and 10 times, 10 times greater, or even greater than 10 times higher number, amount, or concentration than that which is naturally present. As a non-limiting example, a microbial strain or functional homolog is heterologously applied/contacted on a seedling if that microbial strain or functional homolog is normally found at the flowering stage of a plant and not at a seedling stage.

The microbial strains as described herein can be isolated as described in Table 1 of the Examples section which follows (that lists the source of the deposited microbes), Tables 2-58 above and the structural and functional characteristics of the microbial strains as described herein (e.g., Tables 46 and 60) may help in selecting the microbial strain of some embodiments of the invention.

Functional homologs may occur in nature without the intervention of man, e.g., by the mere propagation in culture. They can be also obtained by treatment with or by a variety of methods and compositions known to those of skill in the art. For example, the deposited strain may be treated with a chemical such as N-methyl-N'-nitro-N-nitrosoguanidine, ethylmethanesulfone, or by irradiation using gamma, x-ray, or UV-irradiation, or by other means well known to those practiced in the art, e.g., site directed mutagenesis, or by selection for a specific phenotype of interest such as described in Tables 2-58 in conjunction with the genetic structure selection elements as described above.

According to a specific embodiment, the microbial strain is genetically modified.

Methods of genetically modifying microbial strains are well known in the art and include, site directed mutagenesis and genome editing.

According to a specific embodiment, the microbial strain is naive (wild-type).

For research, development or agricultural applications (e.g., as described herein, improving an agricultural trait), the microorganisms as described herein need to be produced at small-, or large-scale.

Thus, according to an aspect of the invention there is provided a method of preparing an agricultural composition or preparation as described herein. The method comprises inoculating a microbial strain selected from the group consisting of:
(1) an EVO33432 strain, deposited as Accession Number 42921 at NCIMB or a functionally homologous strain;
(2) an EVO33410 strain, deposited as 42961 at NCIMB or a functionally homologous strain;
(3) an EVO33407 strain, deposited as Accession Number 42922 at NCIMB or a functionally homologous strain;
(4) an EVO33401 strain, deposited as Accession Number 42923 at NCIMB or a functionally homologous strain;
(5) an EVO33393 strain, deposited as Accession Number 42924 at NCIMB or a functionally homologous strain;
(6) an EVO33661 strain, deposited as Accession Number 42925 at NCIMB or a functionally homologous strain;
(7) an EVO33398 strain, deposited as Accession Number 42926 at NCIMB or a functionally homologous strain;
(8) an EVO33395 strain, deposited as Accession Number 42927 at NCIMB or a functionally homologous strain;
(9) an EVO33394 strain, deposited as Accession Number 42928 at NCIMB or a functionally homologous strain;
(10) an EVO32844 strain, deposited as Accession Number 42929 at NCIMB or a functionally homologous strain;
(11) an EVO32845 strain, deposited as Accession Number 42930 at NCIMB or a functionally homologous strain;
(12) an EVO33405 strain, deposited as Accession Number 42931 at NCIMB or a functionally homologous strain;
(13) an EVO32831 strain, deposited as Accession Number 42932 at NCIMB or a functionally homologous strain;
(14) an EVO33746 strain, deposited as Accession Number 42933 at NCIMB or a functionally homologous strain;
(15) an EVO33872 strain, deposited as Accession Number 42959 at NCIMB or a functionally homologous strain;
(16) an EVO33887 strain, deposited as Accession Number 42934 at NCIMB or a functionally homologous strain;
(17) an EVO11090 strain, deposited as Accession Number 42935 at NCIMB or a functionally homologous strain;
(18) an EVO33657 strain, deposited as Accession Number 42936 at NCIMB or a functionally homologous strain;
(19) an EVO33447 strain, deposited as Accession Number 42937 at NCIMB or a functionally homologous strain;
(20) an EVO33415 strain, deposited as Accession Number 42938 at NCIMB or a functionally homologous strain;
(21) an EVO40185 strain, deposited as Accession Number 42939 at NCIMB or a functionally homologous strain;
(22) an EVO32828 strain, deposited as Accession Number 42940 at NCIMB or a functionally homologous strain;
(23) an EVO32834 strain, deposited as Accession Number 42941 at NCIMB or a functionally homologous strain;
(24) an EVO32868 strain, deposited as Accession Number 42942 at NCIMB or a functionally homologous strain;
(25) an EVO33402 strain, deposited as Accession Number 42943 at NCIMB or a functionally homologous strain;
(26) an EVO40194 strain, deposited as Accession Number 42944 at NCIMB or a functionally homologous strain;
(27) an EVO32839 strain, deposited as Accession Number 42945 at NCIMB or a functionally homologous strain; and
(28) an EVO33441 strain, deposited as 42960 at NCIMB or a functionally homologous strain;
wherein the microbial strain or the functionally homologous strain improves an agricultural trait of a cultivated plant heterologous to the microbial strain or the functionally homologous strain as compared to a control plant not treated with the microbial strain or the functionally homologous strain, and wherein the microbial strain or the functionally homologous strain is present in the preparation at a concentration which exceeds that found in nature, into or onto a substratum and allowing the microbial strain or the functional homolog to grow at a temperature of 1-37 °C until obtaining a number of cells or spores of at least 10²-10³ per milliliter or per gram.

Cultures of the deposited strains or functional homologs may be prepared for use in compositions of the invention using standard static drying and liquid fermentation techniques known in the art. Growth is commonly effected in a bioreactor.

A bioreactor refers to any device or system that supports a biologically active environment. As described herein a bioreactor is a vessel in which microorganisms including the microorganism of the invention can be grown. A bioreactor may be any appropriate shape or size for growing the microorganisms. A bioreactor may range in size and scale from 10 mL (e.g., small scale) to liter's to cubic meters (e.g., large scale) and may be made of stainless steel, disposable material (e.g., nylon, plastic bags) or any other appropriate material as known and used in the art. The bioreactor may be a batch type bioreactor, a fed batch type or a continuous-type bioreactor (e.g., a continuous stirred reactor). For example, a bioreactor may be a chemostat as known and used in the art of microbiology for growing and harvesting microorganisms. A bioreactor may be obtained from any commercial supplier (See also Bioreactor System Design, Asenjo & Merchuk, CRC Press, 1995).

For small scale operations, a batch bioreactor may be used, for example, to test and develop new processes, and for processes that cannot be converted to continuous operations.

Microorganisms grown in a bioreactor may be suspended or immobilized. Growth in the bioreactor is generally under aerobic conditions at suitable temperatures and pH for growth. For the organisms of the invention, cell growth can be achieved at temperatures between 5-37 °C., with an exemplary temperature range selected from 15 to 30°C, 15 to 28 °C, 20 to 30°C, or 15 to 25 °C. The pH of the nutrient medium can vary between 4.0 and 9.0. For example, the operating range can be usually slightly acidic to neutral at pH 4.0 to 7.0, or 4.5 to 6.5, or pH 5.0 to 6.0. Typically, maximal cell yield is obtained in 20-72 hours after inoculation.

Optimal conditions for the cultivation of the microorganisms of this invention will, of course, depend upon the particular strain. However, by virtue of the conditions applied in the selection process and general requirements of most microorganisms, a person of ordinary skill in the art would be able to determine essential nutrients and conditions. The microorganisms would typically be grown in aerobic liquid cultures on media which contain sources of carbon, nitrogen, and inorganic salts that can be assimilated by the microorganism and supportive of efficient cell growth. Exemplary carbon sources are hexoses such as glucose, but other sources that are readily assimilated such as amino acids, may be substituted. Many inorganic and proteinaceous materials may be used as nitrogen sources in the growth process. Exemplary nitrogen sources are amino acids and urea but others include gaseous ammonia, inorganic salts of nitrate and ammonium, vitamins, purines, pyrimidines, yeast extract, beef extract, proteose peptone, soybean meal, hydrolysates of casein, distiller's solubles, and the like. Among the inorganic minerals that can be incorporated into the nutrient medium are the customary salts capable of yielding calcium, zinc, iron, manganese, magnesium, copper, cobalt, potassium, sodium, molybdate, phosphate, sulfate, chloride, borate, and like ions.

The culture can be a pure culture, whereby a single microbial strain is included or a mixed culture. This is of course pending the compliance of the microbial strains to co-exist and proliferate under the same culturing conditions. When needed, an antibiotic or other growth-restricting conditions can be employed during culturing to restrict the growth of other microorganisms (contaminants) not desired in the culture/co-culture e.g., temperature, essential nutrients and the like.

According to an alternative or an additional embodiment, the desired combination is produced following culturing, such as when the microbial strains do not share the same or optimal culturing conditions.

The ratio of each type of microorganism in the final product will depend on the desired agricultural trait to be achieved.

The identity of the microorganism(s) in the culture can be experimentally validated at the nucleic acid level, protein level, plant level (improving an agricultural trait of interest according to the Examples section which follows, e.g., Tables 7, 12, 18 and 27) or by using classical microbiology tools, e.g., streaking (e.g., with selection).

According to a specific embodiment, the composition or formulation, as further described hereinbelow, does not comprise more than 50, 30, 20 or 10 microbial strains.

According to a specific embodiment, the composition or preparation is soil-free.

According to some embodiments, also contemplated are compositions obtainable according to the methodology(s) described herein.

The culture or isolated preparation derived therefrom can be in a culture fluid, pellet, scraping, dried sample, lyophilisate, or a support, container, or medium such as a plate, paper, filter, matrix, straw, pipette or pipette tip, fiber, needle, gel, swab, tube, vial, particle, etc. that contains a single type of organism or no more than 10 species of organisms.

Alternatively or additionally, microbial strains of some embodiments of the present invention that comprise the microbial strains or cultures thereof can be in a variety of forms, including, but not limited to, still cultures, whole cultures, stored stocks of cells (particularly glycerol stocks), agar strips, stored agar plugs in glycerol/water, freeze dried stocks, and dried stocks such as lyophilisate dried onto filter paper or grain seeds.

According to an aspect of the invention there is provided a composition comprising the preparation as described herein and further comprising an agriculturally effective amount of a compound or composition selected from the group consisting of a fertilizer, an acaricide, a bactericide, a fungicide, an insecticide, a microbicide, a nematicide, a pesticide, a plant growth regulator, a rodenticide, a nutrient.

Also provided is a formulation comprising the preparation or composition of as described herein.

The compositions/formulations may further comprise a carrier. The carrier may be any one or more of a number of carriers that confer a variety of properties, such as increased stability, wettability, dispersability, etc. Wetting agents such as natural or synthetic surfactants, which can be nonionic or ionic surfactants, or a combination thereof can be included in a composition of the invention. Water-in-oil emulsions can also be used to formulate a composition that includes at least one isolated microorganism of the present invention (see, for example, U.S. Pat. No. 7,485,451, incorporated by reference herein). Suitable formulations that may be prepared include wettable powders, granules, gels, agar strips or pellets, thickeners, and the like, microencapsulated particles, and the like, liquids such as aqueous flowables, aqueous suspensions, water-in-oil emulsions, etc. The formulation may include grain or legume products (e.g., ground grain or beans, broth or flour derived from grain or beans), starch, sugar, or oil. The carrier may be an agricultural carrier. In certain preferred embodiments, the carrier is a seed, and the composition may be applied or coated onto the seed or allowed to saturate the seed.

In some embodiments, the agricultural carrier may be soil or plant growth medium. Other agricultural carriers that may be used include water, fertilizers, plant-based oils, humectants, or combinations thereof. Alternatively, the agricultural carrier may be a solid, such as diatomaceous earth, loam, silica, alginate, clay, bentonite, vermiculite, seed cases, other plant and animal products, or combinations, including granules, pellets, or suspensions. Mixtures of any of the aforementioned ingredients are also contemplated as carriers, such as but not limited to, pesta (flour and kaolin clay), agar or flour-based pellets in loam, sand, or clay, etc. Formulations may include food sources for the cultured organisms, such as barley, rice, or other biological materials such as seed, plant parts, sugar cane bagasse, hulls or stalks from grain processing, ground plant material ("yard waste") or wood from building site refuse, sawdust or small fibers from recycling of paper, fabric, or wood. Other suitable formulations will be known to those skilled in the art.

**In** the liquid form, e.g., solutions or suspensions, the microbial strain may be mixed or suspended in water or in aqueous solutions. Suitable liquid diluents or carriers include water, aqueous solutions, petroleum distillates, or other liquid carriers.

Solid compositions can be prepared by dispersing the microbial strain in and on an appropriately divided solid carrier, such as peat, wheat, bran, vermiculite, clay, talc, bentonite, diatomaceous earth, fuller's earth, pasteurized soil, and the like. When such formulations are used as wettable powders, biologically compatible dispersing agents such as non-ionic, anionic, amphoteric, or cationic dispersing and emulsifying agents can be used.

In a specific embodiment of the present invention, the composition/formulation may further include at least one chemical or biological fertilizer. The amount of at least one chemical or biological fertilizer employed can vary depending on the final formulation as well as the size of the plant and seed to be treated. Examples of biological fertilizers that are suitable for use herein according to the present invention for promoting plant growth and/yield include microbes, animals, bacteria, fungi, genetic material, plant, and natural products of living organisms.

A variety of chemical pesticides is apparent to one of skill in the art and may be used. Exemplary chemical pesticides include those in the carbamate, organophosphate, organochlorine, and prethroid classes. Also included are chemical control agents such as, but not limited to, benomyl, borax, captafol, captan, chorothalonil, formulations containing copper; formulations containing dichlone, dicloran, iodine, zinc; fungicides that inhibit ergosterol biosynthesis such as but not limited to blastididin, cymoxanil, fenarimol, flusilazole, folpet, imazalil, ipordione, maneb, manocozeb, metalaxyl, oxycarboxin, myclobutanil, oxytetracycline, PCNB, pentachlorophenol, prochloraz, propiconazole, quinomethionate, sodium aresenite, sodium DNOC, sodium hypochlorite, sodium phenylphenate, streptomycin, sulfur, tebuconazole, terbutrazole, thiabendazolel, thiophanate-methyl, triadimefon, tricyclazole, triforine, validimycin, vinclozolin, zineb, and ziram.

The formulation as used herein can refer also to a customary formulation in an effective amount to either the soil (i.e., in-furrow), a portion of the plant (i.e., drench) or on the seed before planting (i.e., seed coating or dressing). Customary formulations include solutions, emulsifiable concentrate, wettable powders, suspension concentrate, soluble powders, granules, suspension-emulsion concentrate, natural and synthetic materials impregnated with active compound, and very fine control release capsules in polymeric substances. In certain embodiments of the present invention, the microbial strains are formulated in powders that are available in either a ready-to-use formulation or are mixed together at the time of use. In either embodiment, the powder may be admixed with the soil prior to or at the time of planting.

Depending on the final formulation, one or more suitable additives can also be introduced to the compositions of the present invention. Adhesives such as carboxymethylcellulose and natural and synthetic polymers in the form of powders, granules or latexes, such as gum arabic, chitin, polyvinyl alcohol and polyvinyl acetate, as well as natural phospholipids, such as cephalins and lecithins, and synthetic phospholipids, can be added to the present compositions.

In an embodiment, the microbial strains are formulated in a single, stable solution, or emulsion, or suspension. For solutions, the active chemical compounds are typically dissolved in solvents before the microbial strain is added. Suitable liquid solvents include petroleum based aromatics, such as xylene, toluene or alkylnaphthalenes, aliphatic hydrocarbons, such as cyclohexane or paraffins, for example petroleum fractions, mineral and vegetable oils, alcohols, such as butanol or glycol as well as their ethers and esters, ketones, such as methyl ethyl ketone, methyl isobutyl ketone or cyclohexanone, strongly polar solvents, such as dimethylformamide and dimethyl sulphoxide. For emulsion or suspension, the liquid medium is water. In one embodiment, the chemical agent and the microbial strain are suspended in separate liquids and mixed at the time of application. In a preferred embodiment of suspension, the chemical agent and the microbial strain are combined in a ready-to-use formulation that exhibits a reasonably long shelf-life. In use, the liquid can be sprayed or can be applied foliarly as an atomized spray or in-furrow at the time of planting the crop. The liquid composition can be introduced in an effective amount on the seed (i.e., seed coating or dressing) or to the soil (i.e., in-furrow) before germination of the seed or directly to the soil in contact with the roots by utilizing a variety of techniques known in the art including, but not limited to, drip irrigation, sprinklers, soil injection or soil drenching. Optionally, stabilizers and buffers can be added, including alkaline and alkaline earth metal salts and organic acids, such as citric acid and ascorbic acid, inorganic acids, such as hydrochloric acid or sulfuric acid. Biocides can also be added and can include formaldehydes or formaldehyde-releasing agents and derivatives of benzoic acid, such as p-hydroxybenzoic acid.

The amount of the bacterial strain or functional homolog is sufficient to interact, colonize and/or localize in a cultivated plant treated with same.

Assays for interaction, colonization and localization are described further hereinbelow.

One of ordinary skill in the art would know how to calculate the concentration of the microbial strain or functional homolog.

According to a specific embodiment, the amount of the microbial strain or functional homolog thereof in the composition/formulation is as mentioned hereinabove.

According to a specific embodiment, the microbial strain(s) is about 2 % w/w/ to about 80 % w/w of the entire formulation/composition/preparation. According to another embodiment, the microbial strains(s) employed in the compositions is about 5 % w/w to about 65 % w/w or about 10% w/w to about 60% w/w by weight of the entire formulation/composition/preparation.

According to a specific embodiment, the preparation/composition/formulation provided herein is substantially stable. Thus, the microbial strain or functional homolog may be shelf-stable, where at least 0.01%, of the CFU or spores are viable after storage in desiccated form (i.e., moisture content of 30 % or less) for 1, 2, 3, 4, 5, 6, 7, 8, 9, 10 or greater than 10 weeks at 4 °C or at room temperature. Optionally, a shelf-stable formulation is in a dry formulation, a powder formulation, or a lyophilized formulation. In some embodiments, the formulation is formulated to provide stability for microbial strain or functional homolog. In one embodiment, the formulation is substantially stable at temperatures between about -20 °C and about 50 °C for at least about 1, 2, 3, 4, 5, or 6 days, or 1, 2, 3 or 4 weeks, or 1, 2, 3, 4, 5, 6, 7, 8, 9, 10, 11 or 12 months, or one or more years. In another embodiment, the formulation is substantially stable at temperatures between about 4 °C and about 37 °C for at least about 5, 10, 15, 20, 25, 30 or greater than 180 days.

According to a specific embodiment, the composition/formulation/ preparation is in a form selected from the group consisting of an emulsion, a colloid, a dust, a granule, a pellet, a powder, a spray, an emulsion, or a solution.

According to a specific embodiment, the composition/formulation/ preparation is in a form selected from the group consisting of a liquid, solid, semi-solid, gel or powder.

According to a specific embodiment the composition/formulation/ preparation may further comprise a stabilizer, a tackifier, a preservative, a carrier, a surfactant, an anticomplex agent and a combination thereof.

Once the composition/formulation/ preparation is obtained it can be used for treating plants for improving an agricultural trait of interest.

Thus, according to an aspect of the invention there is provided a method of treating a cultivated plant or portion thereof, said method comprising contacting the plant or portion thereof with the preparation, composition or formulation as described herein.

Also provided is a method of improving an agricultural trait of a cultivated plant, the method comprising:
(a) contacting the plant or portion thereof with an effective amount of the preparation, composition or formulation as described herein; and
(b) growing the plant or portion thereof; and
(c) selecting for the agricultural trait.

According to a specific embodiment, the contacting comprises contacting the plant's surrounding (e.g., soil, as further described hereinbelow).

According to a specific embodiment, the contacting is selected from the group consisting of spraying, immersing, coating, encapsulating, dusting.

According to a specific embodiment, the contacting comprises coating.

According to a specific embodiment, the microbial strain is present at a concentration of at least 100 CFU or spores per plant or portion thereof after said contacting.

According to a specific embodiment, the detection of the microbial strain or functional homologs at the level of detection of at least 100 CFU or spores.

According to a specific embodiment, the portion comprises a seed.

According to a specific embodiment, the portion comprises a seedling.

According to a specific embodiment, the portion comprises a cutting.

According to a specific embodiment, the portion comprises a rhizosphere.

According to a specific embodiment, the portion comprises a vegetative portion.

According to a specific embodiment, the portion comprises foliage.

According to a specific embodiment, the agricultural trait is selected from the group consisting of increased early vigor, increased biomass establishment, increased photosynthetic capacity, increased leaf transpiration rate, increased biomass accumulation up to VT, increased kernel number per plant, increased yield, increased stem conductance, increased assimilate partitioning, kernel volume, increased kernel weight, increased grain filling duration, increased main ear size and increased cob conductance.

According to a specific embodiment, the agricultural trait is selected from the group consisting of increased biomass, increased vigor, increased yield, increased resistance to abiotic stress, increased resistance to biotic stress and increased nitrogen utilization efficiency.

According to a specific embodiment, the agricultural trait is selected from the group consisting of increased root biomass, increased root length, increased height, increased shoot length, increased leaf number, increased water use efficiency, increased tolerance to low nitrogen stress, increased grain yield, increased photosynthetic rate, increased tolerance to drought, increased salt tolerance, increased resistance to nematode stress, increased resistance to a fungal pathogen, increased resistance to a bacterial pathogen and an increased resistance to a viral pathogen.

The preparation/composition/formulation can be applied in an amount effective to improve the agricultural trait of interest relative to that in an untreated control. The active constituents (e.g., microbial strains) are used in a concentration sufficient to enhance the growth of the target plant when applied to the plant. As will be apparent to a skilled person in the art, effective concentrations may vary depending upon various factors such as, for example, (a) the type of the plant or agricultural commodity; (b) the physiological condition of the plant or agricultural commodity; (c) the type of agricultural trait; (d) the stage of the plant; (e) plant as a whole or portion thereof (e.g., seed).

According to a specific embodiment, the contacting is preformed such that the concentration of the microbial strain is at least 100 CFU or spores per plant or portion thereof after the contacting (e.g., 1 hour after contacting).

Alternatively or additionally, the contacting is performed with at least 100 CFU or spores of the microbial strain.

According to a specific embodiment the preparation comprises at least about 100 CFU or spores, at least about 10² CFUs/seed CFUs/gr or CFUs/ml, at least about 10² CFUs/seed CFUs/gr or CFUs/ml, at least about 10³ CFUs/seed CFUs/gr or CFUs/ml, at least about 10⁴ CFUs/seed CFUs/gr or CFUs/ml, at least about 10⁵ CFUs/seed CFUs/gr or CFUs/ml, at least about 10⁶ CFUs/seed CFUs/gr or CFUs/ml, at least about 10⁷ CFUs/seed CFUs/gr or CFUs/ml, at least about 10⁸ CFUs/seed CFUs/gr or CFUs/ml, at least about 10⁹ CFUs/seed CFUs/gr or CFUs/ml.

The composition/preparation/formulation may be contacted with the cultivated plant or portion thereof plant using a variety of conventional methods such as dusting, coating, injecting, rubbing, rolling, dipping, spraying, or brushing, or any other appropriate technique which does not significantly injure the cultivated plant (or portion thereof) to be treated. According to a specific embodiment, contacting includes the inoculation of growth medium or soil with suspensions of microbial cells and the coating of plant seeds, seedlings or foliage with microbial cells and/or spores.

Typically, the compositions/formulations/preparations of the invention are chemically inert; hence they are compatible with substantially any other constituents of the application schedule. They may also be used in combination with plant growth affecting substances, such as fertilizers, plant growth regulators, and the like, provided that such compounds or substances are biologically compatible. They can also be used in combination with pesticides, herbicides, nematocides, fungicides, insecticides, acaricides, bactericides, microbicides, or combinations of any thereof. A mixture with other known active compounds, such as growth regulators, safeners and/or semiochemicals or any other agent that is described above in the context of the composition/formulation is also contemplated herein and not necessarily as part of the composition formulation that comprises the microbial strain.

According to a specific embodiment contacting is effected as a seed coating, a root treatment, or a foliar application. Each of which may comprise one or more microbial strains e.g., not more than 10 strains.

According to a specific embodiment, the plant or portion thereof is surface sterilized prior to contacting with the composition/formulation/preparation, especially for research applications.

In some embodiments, the composition/formulation/preparation can be applied to the plant or portion thereof, for example the plant seed, or by foliar application, and successful colonization can be confirmed by detecting the presence of the microbial strain within the plant. For example, after applying the composition/formulation/preparation to the seeds, high titers of the microbial strain can be detected in the roots and shoots of the plants that germinate from the seeds. In addition, significant quantities of the microbial strain can be detected in the rhizosphere of the plants. Therefore, in some embodiments, the microbial strain is heterologously disposed in an amount effective to colonize the plant. In some embodiments, colonization of the plant can be detected, for example, by detecting the presence of the microbial strain inside the plant. This can be accomplished by measuring the viability of the microbial strain after surface sterilization of the plant portion: microbial strain colonization results in an internal localization of the microbe, rendering it resistant to conditions of surface sterilization. The presence and quantity of the microbial strain can also be established using other means known in the art, for example, immunofluorescence microscopy using microbe specific antibodies, or fluorescence in situ hybridization. Alternatively, specific nucleic acid probes recognizing conserved sequences from the endophytes can be employed to amplify a region, for example by quantitative PCR, and correlated to CFUs by means of a standard curve.

According to other embodiments, the microbial strain is heterologously disposed, for example, on the surface of a plant portion of a cultivated plant, in an amount effective to be detectable in the mature agricultural plant. In some embodiments, the microbial strain is heterologously disposed in an amount effective to be detectable in an amount of at least about 100 CFU or spores, between 100 and 200 CFU or spores, at least about 200 CFU or spores, between 200 and 300 CFU or spores, at least about 300 CFU or spores, between 300 and 400 CFU or spores, at least about 500 CFU or spores, between 500 and 1,000 CFU or spores, at least about 1,000 CFU or spores, between 1,000 and 3,000 CFU or spores, at least about 3,000 CFU or spores, between 3,000 and 10,000 CFU or spores, at least about 10,000 CFU or spores, between 10,000 and 30,000 CFU or spores, at least about 30,000 CFU or spores, between 30,000 and 100,000 CFU or spores, at least about 100,000 CFU or spores, between 100,000 and 10⁶ CFU or spores at least about 10⁶ CFU or spores or more in the mature agricultural plant.

In some embodiments, the microbial strain is capable of colonizing particular tissue types of the plant. In some embodiments, the microbial strain is heterologously disposed on the plant portion in an amount effective to be detectable within a target tissue of the mature cultivated plant selected from a fruit, a seed, a leaf, or a root, or portion thereof. For example, the microbial strain can be detected in an amount of at least about 100 CFU or spores, between 100 and 200 CFU or spores, at least about 200 CFU or spores, between 200 and 300 CFU or spores, at least about 300 CFU or spores, between 300 and 400 CFU or spores, at least about 500 CFU or spores, between 500 and 1,000 CFU or spores, at least about 1,000 CFU or spores, between 1,000 and 3,000 CFU or spores, at least about 3,000 CFU or spores, between 3,000 and 10,000 CFU or spores, at least about 10,000 CFU or spores, between 10,000 and 30,000 CFU or spores, at least about 30,000 CFU or spores, between 30,000 and 100,000 CFU or spores, at least about 100,000 CFU or spores, between 100,000 and 10⁶ CFU or spores at least about 10⁶ CFU or spores or more in the mature cultivated plant.

According to some embodiments, the microbial strain described herein is capable of migrating/localizing from one tissue type to another. In some embodiments, the microbial strain that is coated onto the seed of a plant is capable, upon germination of the seed into a vegetative state, of localizing to a different tissue of the plant. For example, the microbial strain can be capable of localizing to any one of the tissues in the plant, including: the root, adventitious root, seminal root, root hair, shoot, leaf, flower, bud, tassel, meristem, pollen, pistil, ovaries, stamen, fruit, stolon, rhizome, nodule, tuber, trichome, guard cells, hydathode, petal, sepal, glume, rachis, vascular cambium, phloem, and xylem. In some embodiments, the microbial strain is capable of localizing to the root and/or the root hair of the cultivated plant. In other embodiments, the microbial strain is capable of localizing to the photosynthetic tissues, for example, leaves and shoots of the plant. In other cases, the microbial strain is localized to the vascular tissues of the plant, for example, in the xylem and phloem. In still another embodiment, the microbial strain is capable of localizing to the reproductive tissues (flower, pollen, pistil, ovaries, stamen, fruit) of the cultivated plant. In other embodiments, the microbial strain is capable of localizing to the root, shoots, leaves and reproductive tissues of the cultivated plant. In still another embodiment, the microbial strain colonizes a fruit or seed tissue of the cultivated plant. In still another embodiment, the microbial strain is able to colonize the plant such that it is present in the surface of the cultivated plant (i.e., microbial strain presence is detectably present on the plant exterior, or the episphere of the plant). In still other embodiments, the microbial strain is capable of localizing to substantially all, or all, tissues of the plant. In certain embodiments, the microbial strain is not localized to the root of a plant. In other cases, the microbial strain is not localized to the photosynthetic tissues of the plant.

In some embodiments, the microbial strain heterologously disposed on the plant element can be detected in the rhizosphere. In some embodiments, the rhizosphere-localized microbe can secrete compounds (such as siderophores or organic acids) that assist with nutrient acquisition by the plant. Therefore, in other embodiments, the microbial strain is heterologously disposed on the plant part in an amount effective to detectably colonize the soil environment surrounding the mature agricultural plant when compared with a reference agricultural plant. For example, the microbe can be detected in an amount of at least 100 CFU or spores/g DW, for example, at least 200 CFU or spores/g DW, at least 500 CFU or spores/g DW, at least 1,000 CFU or spores/g DW, at least 3,000 CFU or spores/g DW, at least 10,000 CFU or spores/g DW, at least 30,000 CFU or spores/g DW, at least 100,000 CFU or spores/g DW, at least 300,000 CFU or spores/g DW, or more, in the rhizosphere.

According to a specific embodiment, concomitantly or following contacting, the plant is allowed to grow or regenerated (in the case of a plant portion). For example, the plant or plant portions are placed in a medium that promotes plant growth. According to a specific embodiment, the medium that promotes plant growth is selected from the group consisting of: soil, hydroponic apparatus, and artificial growth medium. In some embodiments the plant portion is selected from the group consisting of seeds that are placed in the soil in rows, with substantially equal spacing between each seed within each row.

As used herein, the phrase "non-stress conditions" or "normal conditions" refers to the growth conditions (e.g., water, temperature, light-dark cycles, humidity, salt concentration, fertilizer concentration in soil, nutrient supply such as nitrogen, phosphorous and/or potassium), that do not significantly go beyond the everyday climatic and other abiotic conditions that plants may encounter, and which allow optimal growth, metabolism, reproduction and/or viability of a plant at any stage in its life cycle (e.g., in a crop plant from seed to a mature plant and back to seed again). Persons skilled in the art are aware of normal soil conditions and climatic conditions for a given plant in a given geographic location. It should be noted that while the non-stress conditions may include some mild variations from the optimal conditions (which vary from one type/species of a plant to another), such variations do not cause the plant to cease growing without the capacity to resume growth.

Following is a non-limiting description of non-stress (normal) growth conditions which can be used for growing the transgenic plants expressing the polynucleotides or polypeptides of some embodiments of the invention.

For example, normal conditions for growing sorghum include irrigation with about 452,000 liter water per 1000 square meters (1000 square meters) and fertilization with about 14 units nitrogen per 1000 square meters per growing season.

Normal conditions for growing cotton include irrigation with about 580,000 liter water per 1000 square meters (1000 square meters) and fertilization with about 24 units nitrogen per 1000 square meters per growing season.

Normal conditions for growing bean include irrigation with about 524,000 liter water per 1000 square meters (1000 square meters) and fertilization with about 16 units nitrogen per 1000 square meters per growing season.

Normal conditions for growing B. Juncea include irrigation with about 861,000 liter water per 1000 square meters (1000 square meters) and fertilization with about 12 units nitrogen per 1000 square meters per growing season.

The phrase "abiotic stress" as used herein refers to any adverse effect on metabolism, growth, reproduction and/or viability of a plant. Accordingly, abiotic stress can be induced by suboptimal environmental growth conditions such as, for example, salinity, osmotic stress, water deprivation, drought, flooding, freezing, low or high temperature, heavy metal toxicity, anaerobiosis, nutrient deficiency (e.g., nitrogen deficiency or limited nitrogen), atmospheric pollution or UV irradiation. The implications of abiotic stress are discussed in the Background section.

The phrase "abiotic stress tolerance" as used herein refers to the ability of a plant to endure an abiotic stress without suffering a substantial alteration in metabolism, growth, productivity and/or viability.

Plants are subject to a range of environmental challenges. Several of these, including salt stress, general osmotic stress, drought stress and freezing stress, have the ability to impact whole plant and cellular water availability. Not surprisingly, then, plant responses to this collection of stresses are related. Zhu (2002) Ann. Rev. Plant Biol. 53: 247-273 et al. note that "most studies on water stress signaling have focused on salt stress primarily because plant responses to salt and drought are closely related and the mechanisms overlap". Many examples of similar responses and pathways to this set of stresses have been documented. For example, the CBF transcription factors have been shown to condition resistance to salt, freezing and drought (Kasuga et al. (1999) Nature Biotech. 17: 287-291). The Arabidopsis rd29B gene is induced in response to both salt and dehydration stress, a process that is mediated largely through an ABA signal transduction process (Uno et al. (2000) Proc. Natl. Acad. Sci. USA 97: 11632-11637), resulting in altered activity of transcription factors that bind to an upstream element within the rd29B promoter. In Mesembryanthemum crystallinum (ice plant), Patharker and Cushman have shown that a calcium-dependent protein kinase (McCDPK1) is induced by exposure to both drought and salt stresses (Patharker and Cushman (2000) Plant J. 24: 679-691). The stress-induced kinase was also shown to phosphorylate a transcription factor, presumably altering its activity, although transcript levels of the target transcription factor are not altered in response to salt or drought stress. Similarly, Saijo et al. demonstrated that a rice salt/drought-induced calmodulin-dependent protein kinase (OsCDPK7) conferred increased salt and drought tolerance to rice when overexpressed (Saijo et al. (2000) Plant J. 23: 319-327).

Exposure to dehydration invokes similar survival strategies in plants as does freezing stress (see, for example, Yelenosky (1989) Plant Physiol 89: 444-451) and drought stress induces freezing tolerance (see, for example, Siminovitch et al. (1982) Plant Physiol 69: 250-255; and Guy et al. (1992) Planta 188: 265-270). In addition to the induction of cold-acclimation proteins, strategies that allow plants to survive in low water conditions may include, for example, reduced surface area, or surface oil or wax production. In another example increased solute content of the plant prevents evaporation and water loss due to heat, drought, salinity, osmoticum, and the like therefore providing a better plant tolerance to the above stresses.

It will be appreciated that some pathways involved in resistance to one stress (as described above), will also be involved in resistance to other stresses, regulated by the same or homologous genes. Of course, the overall resistance pathways are related, not identical, and therefore not all genes controlling resistance to one stress will control resistance to the other stresses. Nonetheless, if a gene conditions resistance to one of these stresses, it would be apparent to one skilled in the art to test for resistance to these related stresses. Methods of assessing stress resistance are further provided in the Examples section which follows.

As used herein, the phrase "drought conditions" refers to growth conditions with limited water availability. It should be noted that in assays used for determining the tolerance of a plant to drought stress the only stress induced is limited water availability, while all other growth conditions such as fertilization, temperature and light are the same as under normal conditions.

For example drought conditions for growing Brachypodium include irrigation with 240 milliliter at about 20% of tray filled capacity in order to induce drought stress, while under normal growth conditions trays irrigated with 900 milliliter whenever the tray weight reached 50% of its filled capacity.

As used herein the phrase "water use efficiency (WUE)" refers to the level of organic matter produced per unit of water consumed by the plant, *i.e.,* the dry weight of a plant in relation to the plant's water use, e.g., the biomass produced per unit transpiration.

As used herein the phrase "fertilizer use efficiency" refers to the metabolic process(es) which lead to an increase in the plant's yield, biomass, vigor, and growth rate per fertilizer unit applied. The metabolic process can be the uptake, spread, absorbent, accumulation, relocation (within the plant) and use of one or more of the minerals and organic moieties absorbed by the plant, such as nitrogen, phosphates and/or potassium.

As used herein the phrase "fertilizer-limiting conditions" refers to growth conditions which include a level (e.g., concentration) of a fertilizer applied which is below the level needed for normal plant metabolism, growth, reproduction and/or viability.

As used herein the phrase "nitrogen use efficiency (NUE)" refers to the metabolic process(es) which lead to an increase in the plant's yield, biomass, vigor, and growth rate per nitrogen unit applied. The metabolic process can be the uptake, spread, absorbent, accumulation, relocation (within the plant) and use of nitrogen absorbed by the plant.

As used herein the phrase "nitrogen-limiting conditions" refers to growth conditions which include a level (e.g., concentration) of nitrogen (e.g., ammonium or nitrate) applied which is below the level needed for normal plant metabolism, growth, reproduction and/or viability.

Improved plant NUE and FUE is translated in the field into either harvesting similar quantities of yield, while implementing less fertilizers, or increased yields gained by implementing the same levels of fertilizers. Thus, improved NUE or FUE has a direct effect on plant yield in the field. Thus, the polynucleotides and polypeptides of some embodiments of the invention positively affect plant yield, seed yield, and plant biomass. In addition, the benefit of improved plant NUE will certainly improve crop quality and biochemical constituents of the seed such as protein yield and oil yield.

Also provided is a cultivated plant or portion thereof having been treated with the preparation, composition or formulation as described herein.

Yet there is provided a composition comprising the preparation, composition, culture or formulation as described herein and a cultivated plant or a portion thereof, the plant or portion thereof being heterologous to the microbial strain or culture.

According to a specific embodiment, the portion comprises a seed, seedling or cutting.

According to a specific embodiment, the microbial strain coats the portion.

According to a specific embodiment, the microbial strain is present in the coat at a concentration of at least about 100 CFU or spores or spores, between 100 and 200 CFU or spores, at least about 200 CFU or spores, between 200 and 300 CFU or spores, at least about 300 CFU or spores, between 300 and 400 CFU or spores, at least about 500 CFU or spores, between 500 and 1,000 CFU or spores, at least about 1,000 CFU or spores, between 1,000 and 3,000 CFU or spores, at least about 3,000 CFU or spores, between 3,000 and 10,000 CFU or spores, at least about 10,000 CFU or spores, between 10,000 and 30,000 CFU or spores, at least about 30,000 CFU or spores, between 30,000 and 100,000 CFU or spores, at least about 100,000 CFU or spores, between 100,000 and 10⁶ CFU or spores at least about 10⁶ CFU or spores or more per seed (coat).

Methods of qualifying the presence of the microbial strain are described herein throughout the disclosure and are well known to those of skills in the art.

Also provided herein is an article of manufacture which comprises the composition/preparation/formulation with or without a heterologous plant element e.g., seed as described herein.

According to a specific embodiment, the article of manufacture is selected from the group consisting of: bottle, jar, ampule, package, vessel, bag, box, bin, envelope, carton, container, silo, shipping container, truck bed, and case.

Also provided herein is a method of processing a cultivated plant or portion thereof to a processed product of interest, the method comprising:
(a) providing the cultivated plant or portion thereof with the heterogonous microbial strain as described herein;
(b) subjecting said cultivated plant or portion thereof to a processing procedure so as to obtain the processed product.

According to a specific embodiment, the processing procedure is selected from the group consisting of cutting, chopping, grinding, milling, shredding, homogenizing, or pressing.

Embodiments of the invention further relate to processed products generated according to the present teachings in which the DNA of the microbial strain and optionally that of the plant are included, however in most cases neither the plant nor the microbial strain are in a viable condition.

The processed product may this comprise DNA unique for the cultivated plant or portion thereof and to the microbial strain and which can be detected by, for example, deep-sequencing.

According to a specific embodiment, the processed product is selected from the group consisting of a flour, a syrup, a meal, an oil, a film, a packaging, a construction material, a paper, a nutraceutical product, a pulp, an animal feed, a fish fodder, a bulk material for industrial chemicals, a cereal product and a processed human-food product.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is understood that any Sequence Identification Number (SEQ ID NO) disclosed in the instant application can refer to either a DNA sequence or an RNA sequence, depending on the context where that SEQ ID NO is mentioned, even if that SEQ ID NO is expressed only in a DNA sequence format or an RNA sequence format.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### SOURCING OF MICROBIAL STRAINS WITH PLANT BIOSTIMULATORY ACTIVITY

This example describes the source from where the microbial strains were isolated prior to screening for plant bio-stimulatory activity. Sourcing is guided by one or more of the following assumptions:
1. The inventors assume that the plant microbiome is enriched with plant beneficial microbes that co-evolved with plants and developed a mutualistic interaction with plants (Bulgarelli, D., Schlaeppi, K., Spaepen, S., Ver Loren van Themaat, E., Schulze-Lefert, P. 2013. Structure and functions of the bacterial microbiota of plants. Annu. Rev. Plant Biol. 64:807-838).
2. The inventors assume that plants growing in the wild are dependent on functions provided by their microbiome for survival and reproduction. In contrast, domesticated plants are nurtured by farmers and therefore do not need the full extent of microbiome functions and therefore are not a good source for beneficial microbial strains (Philippot, L., Raaijmakers, J.M., Lemanceau, P., and van der Putten, W.H. 2013. Going back to the roots: the microbial ecology of the rhizosphere. Nat. Rev. Microbiol. 11:789-799).
3. The inventors also assume that microbial strains that provide plants with functions that alleviate drought stress are found in climatic zones, habitats and niches in which plant experience water deficiency such as arid and semi-arid climatic zones and sandy soil habitats.
4. The inventors also assume that the microbiomes of plants evolutionarily related to the target plant (*Zea maize*) such as various cereal plants including C4 and C3 plants, are enriched with microbial strains that can also interact, colonize and provide beneficial functions to the target plant.
5. In addition, the inventors assume that native plants to Israel such as wheat and other native wild cereals, co-evolved with the local microbial diversity to exploit the functional diversity available for their survival and reproduction, and therefore are a better source for microbial strains with plant bio-stimulatory activity than non-native plants.

According to some embodiments of the invention, microbial strains were sourced from several sources combining one or more of the above assumptions. Table 1 presents the microbial strains described in this invention and their source.

### Experimental procedures

Sampling expeditions were carried out during the years 2014-2016. Source plants were sampled from various relevant habitats across Israel as described in Table 1. Source plants were removed from soil by digging carefully around plant roots using ethanol-sterilized shovels up to the depth of 20-30 cm below ground. Roots were then vigorously shaken to remove soil particles loosely attached to the roots. The microbiome compartments of the source plant were then separated in the sampling site using ethanol-sterilized pruning shears, into root (including the rhizosphere: the soil remain attached to the root), stem, leaf and grains and stored in separate sterile tubes at 4°C for further processing in the bacteriological laboratory. In the laboratory, the rhizosphere was separated from the root by immersing the root with sterile PBS [per liter: 8 gr sodium chloride (NaCl), 0.2 gr potassium chloride (KCl), 1.42 gr disodium phosphate (Na₂HPO₄) and 0.24 gr potassium phosphate (KH₂PO₄), pH7.4] and shaking for 30 min at 200 rounds per minute (RPM). Thereafter, the root was removed (transferred carefully to a new 50 ml Falcon tube). Microbes released from the root into PBS were serially diluted and the various dilutions were plated onto several bacteriological soil growth media such as, but not limited to R2G [per liter: 0.5 g proteose peptone, 0.5 g casamino acids, 0.5 g yeast extract, 0.5 g dextrose, 0.5 g soluble starch, 0.3 g dipotassium phosphate (K₂HPO₄), 0.05 g, magnesium sulfate (MgSO₄•7H₂O), 0.3 g sodium pyruvate and 8 g gelrite as a gelling agent], PDG (per liter: 4 g potato extract, 20 g dextrose and 8 g gelrite), TSA (per liter: 17 g tryptone, 3 g soytone, 2.5 g dextrose, 5 g NaCl, 2.5 g K₂HPO₄ and 15 g agar), PCA (per liter: 5 g peptone, 2.5 g yeast extract, 1 g glucose and 15 gr agar), LB (per liter: 10 g tryptone, 5 g yeast extract, 10 g NaCl and 15 g agar), LGI [per liter: 5 g sucrose, 0.2 g dipotassium phosphate (K₂HPO₄), 0.6 g monopotassium phosphate (KH₂PO₄), 0.2 g magnesium sulfate (MgSO₄•7H₂O), 0.02 g calcium chloride (CaCl₂•2H₂O), 0.002 g sodium molybdate (Na₂MoO₄•2H₂O), 2 ml bromthymol blue solution (0.5% in 0.2N KOH), 4 ml Fe(III) EDTA (1.64%), 1 ml vitamin solution (per 10 ml: 10 mg biotin, 20 mg pyridoxol) and 8 g gelrite, pH6.0], HVG [per liter: 4 g disodium phosphate (Na₂HPO₄), 1.7 g potassium chloride (KCl), 1 g magnesium sulfate (MgSO₄•7H₂O), 1 g iron sulfate (FeSO₄), 0.02 g calcium carbonate (CaCO₃), 1 g humic acid, 1.5 g calcium chloride (CaCl₂) and 9 g gelrite], PD3 [4 g tryptone, 2 g phytone, 1 g sodium citrate, 1 g disodium succinate, 0.01 g hemin chloride, 1 g magnesium sulfate (MgSO₄•7H₂O), 1.5 g dipotassium phosphate (K₂HPO₄), 1 g monopotassium phosphate (KH₂PO₄), 2 g potato starch and 9 g gelrite)].

Isolated colonies that appeared on plates after 24-72 hours of growth at 28 °C, in the dark, were further picked and re-isolated on a new R2G plate before storage in a R2A broth supplemented with 25 % glycerol at -80°C. Isolates were identified to the strain level by whole genome sequencing using Illumina MiSeq sequencing platform or to the specie level by Sanger sequencing of the 16S-rRNA gene with the universal primers 16S_27F and 16S_1492R (see Example 6 for details).

**Table 1**

| ***The 28 microbial strains described according to some embodiments of the invention and their sourcing data **** | | | | | | |
|---|---|---|---|---|---|---|
| **Microbial strain number** | **Source plant** | **Plant type** | **Evolutionary relatedness to corn** | **Source tissue** | **Climatic growth area** | **Source GPS Coordinates** |
| EVO11090 | *Zea maize* | commercial plant | C4 cereal plant * | stem endosphere | Mediterranean climate | 31°52'58.9"N |
| | | | | | | 34°50'36.4"E |
| EVO32828 | *Triticum dicoccoides* | commercial plant | C3 cereal plant * | rhizosphere | Mediterranean climate | 32°53'46.7"N |
| | | | | | | 35°46'40.3"E |
| EVO32831 | *Triticum dicoccoides* | commercial plant | C3 cereal plant * | rhizosphere | Mediterranean climate | 32°53'46.7"N |
| | | | | | | 35°46'40.3"E |
| EVO32834 | *Atriplex halimus* | wild plant * | non-cereal plant | rhizoplane | arid climate * | 30°52'33.2"N |
| | | | | | | 34°47'08.1"E |
| EVO32839 | *Triticum dicoccoides* | commercial plant | C3 cereal plant * | rhizosphere | Mediterranean climate | 32°53'46.7"N |
| | | | | | | 35°46'40.3"E |
| EVO32844 | *Triticum aestivum* | commercial plant | C3 cereal plant * | root endosphere | Mediterranean climate | 31°36'42.7"N |
| | | | | | | 34°54'18.6"E |
| EVO32845 | *Triticum aestivum* | commercial plant | C3 cereal plant * | spike phyllosphere | Mediterranean climate | 31°36'42.7"N |
| | | | | | | 34°54'18.6"E |
| EVO32868 | *Aegilops sharonesis* | wild plant * | C3 cereal plant * | rhizosphere | Mediterranean climate | 31°47'55.5"N |
| | | | | | | 34°40'16.4"E |
| EVO33393 | *Atriplex halimus* | wild plant * | non-cereal plant | rhizosphere | arid climate * | 30°52'33.2"N |
| | | | | | | 34°47'08.1"E |
| EVO33394 | *Atriplex halimus* | wild plant * | non-cereal plant | rhizosphere | arid climate * | 30°52'33.2"N |
| | | | | | | 34°47'08.1"E |

| **Microbial strain number** | **Source plant** | **Plant type** | **Evolutionary relatedness to corn** | **Source tissue** | **Climatic growth area** | **Source GPS Coordinates** |
|---|---|---|---|---|---|---|
| EVO33395 | *Aegilops sharonesis* | wild plant * | C3 cereal plant * | spike phyllospher e | Mediterranean climate | 31°47'55.5"N |
| | | | | | | 34°40'16.4"E |
| EVO33398 | *Triticum dicoccoides* | commercial plant | C3 cereal plant * | rhizosphere | Mediterranean climate | 32°53'46.7"N |
| | | | | | | 35°46'40.3"E |
| EVO33401 | *Triticum aestivum* | landrace | C3 cereal plant * | seed coat | Mediterranean climate | 31°36'42.7"N |
| | | | | | | 34°54'18.6"E |
| EVO33402 | *Aegilops sharonesis* | wild plant * | C3 cereal plant * | spike phyllospher e | Mediterranean climate | 31°47'55.5"N |
| | | | | | | 34°40'16.4"E |
| EVO33405 | *Triticum aestivum* | commercial plant | C3 cereal plant * | rhizosphere | Mediterranean climate | 31°36'42.7"N |
| | | | | | | 34°54'18.6"E |
| EVO33407 | *Triticum aestivum* | commercial plant | C3 cereal plant * | root endosphere | Mediterranean climate | 31°36'42.7"N |
| | | | | | | 34°54'18.6"E |
| EVO33410 | *Aegilops sharonesis* | wild plant * | C3 cereal plant * | root endosphere | Mediterranean climate | 31°47'55.5"N |
| | | | | | | 34°40'16.4"E |
| EVO33415 | *Aegilops sharonesis* | wild plant * | C3 cereal plant * | root endosphere | Mediterranean climate | 31°47'55.5"N |
| | | | | | | 34°40'16.4"E |
| EVO33432 | *Saccharum spontaneum* | wild plant * | C4 cereal plant * | root endosphere | semi-arid climate * | 31°29'39.9"N |
| | | | | | | 34°46'44.1"E |
| EVO33441 | *Aegilops sharonesis* | wild plant * | C3 cereal plant * | root endosphere | Mediterranean climate * | 31°47'55.5"N |
| | | | | | | 34°40'16.4"E |
| EVO33447 | *Sorghum halepense* | wild plant * | C4 cereal plant * | rhizosphere | semi-arid climate * | 31°19'54.5"N |
| | | | | | | 34°40'30.9"E |
| EVO33657 | *Aegilops sharonesis* | wild plant * | C3 cereal plant * | root endosphere | Mediterranean climate | 31°47'55.5"N |
| | | | | | | 34°40'16.4"E |
| EVO33661 | *Sorghum halepense* | wild plant * | C4 cereal plant * | rhizosphere | semi-arid climate * | 32°35'46.7"N |
| | | | | | | 35°32'53.5"E |
| EVO33746 | *Lotus peregrinus* | wild plant * | Non-cereal plant | leaf endosphere | Mediterranean climate | 32°43'16.2"N |
| | | | | | | 35°09'30.6"E |
| EVO33872 | *Imperata cylindrica* | wild plant * | C4 cereal plant * | stem endosphere | semi-arid climate * | 32°27'38.2"N |
| | | | | | | 35°31'21.7"E |
| EVO33887 | *Zea maize* | commercial plant | C4 cereal plant * | leaf endosphere | Mediterranean climate | 31°52'58.9"N |
| | | | | | | 34°50'36.4"E |
| EVO40185 | *Zea maize* | commercial plant | C4 cereal plant * | rhizosphere | Mediterranean climate | 31°49'04.7"N |
| | | | | | | 34°48'44.7"E |
| EVO40194 | *Sorghum halepense* | wild plant * | C4 cereal plant * | rhizoplane | Mediterranean climate | 32°50'12.5"N |
| | | | | | | 35°30'11.0"E |

| | | | | | | |
|---|---|---|---|---|---|---|
| ****Asterisk-marked cells represent criteria on the basis of which microbial strains were sourced.*** | | | | | | |

### EXAMPLE 2

### M1: A HIGH THROUGHPUT CORN VEGETATIVE GREENHOUSE ASSAY

This example is a description of experiments and results providing proofs that microbial strains, dare endowed with the ability to improve vegetative plant traits when applied to the environment of the seed during sowing as a co-seed application (as explained below). The inventors produced these results using a High-Throughput (HTP) greenhouse-screening assay designated M1. M1 is a plant trait assay testing the ability of microbial strains to improve pre-defined vegetative plant traits in the greenhouse. In this assay the inventors discovered microbial strains that improve the following plant traits in *Zea maize* (corn) plants grown under a moderate drought stress (plants were provided with 50 % less water than plants grown under normal water treatment, aim to produce up to 30% reduction in shoot dry weight):
1) "Early vigor and biomass establishment".
2) "Stem conductance".
3) "Photosynthetic capacity".
4) "Leaf transpiration".
Microbial strains were applied to seeds using a co-seed application. "Co-seed application" refers to an application of microbial cells by pipetting of 1 ml of tap water containing microbial cells at a concentration of 10⁷-10⁹ CFUs/ ml, directly on a seed placed in a hole in the soil.

### Experimental Procedures

Isolated microbial strains were grown as a lawn on two R2G plates for 2 days on 28 °C in the dark. R2G is a bacteriological growth medium composed per liter of: 0.5 g proteose peptone, 0.5 g casamino acids, 0.5 g yeast extract, 0.5 g dextrose, 0.5 g soluble starch, 0.3 g dipotassium phosphate (K₂HPO₄), 0.05 g, magnesium sulfate (MgSO₄•7H₂O), 0.3 g sodium pyruvate and 8 g gelrite as a gelling agent. Cells were then collected from the plates and suspended in 20 ml of tap water. Cell concentration in each suspension was determined using serial dilution in sterile phosphate buffered saline (PBS, composed per liter of: 8 gr sodium chloride (NaCl), 0.2 gr potassium chloride (KCl), 1.42 gr disodium phosphate (Na₂HPO₄) and 0.24 gr potassium phosphate (KH₂PO₄), pH7.4) and plating on R2G plates and counting and calculating Colony Forming Units (CFUs) after 2 days of growth on 28 °C in the dark.

In the greenhouse, 1.8 liter pots were filled up with agricultural field soil. Seeds of a commercial corn hybrid (Pioneer 37N01) were placed in finger-made holes in each pot and 1 ml of cell suspension (10⁷-10⁹ CFU/ ml) was dispensed on top of each seed (a procedure called co-seed, as explained above). The holes were carefully covered and the pots were irrigated to allow germination. Each strain was tested in four pot replicates (*n* = 4; one plant per pot). After seedlings emergence, plants were grown under moderate drought stress (50 % less water than plants grown under normal water treatment, aim to reduce up to 30% reduction in shoot dry weight) up to the stage of five leaves (V5). During growth, plant responses that represent the target plant traits were measured (see Table 7). Plant height and lower stem width were measured once or twice a week (starting from week 2 post sowing). Chlorophyll level, using SPAD units or quantum yield, was measured 3 times along the experiment and the total shoot fresh and/or dry weight and final lower stem width were measured once at experiment completion. Leaf temperature was measured 3 times along the experiment. Plant height growth rate and lower stem width growth rates were calculated from 5-6 sequential measurements.

### Measured responses in M1 High Throughput (HTP) corn trait assay:

**1) Plant height [cm]** - Plants were characterized for height once or twice a week at 5-6 time points during growth period. At each time point, plants were measured for their height using a measuring tape. Plant height was measured from ground level to the top of the longest leaf.
**2) Plant height growth rate [cm/ day]** - A calculation from plant height [cm] measurements. Rate is calculated by dividing the change in plant height over that time period by the time interval.
**3) Lower stem width [mm]** - Plants were characterized for stem width once or twice a week at 5-6 time points during growth period. The diameter of the stem was measured in the lower internode. **Final lower stem width [mm]** - The last lower stem width measurement.
**4) Lower stem width growth rate [mm/ day]** - A calculation from stem width [cm] measurements. Rate is calculated by dividing the change in stem width over that period by the time interval.
**5) SPAD [SPAD units] -** Chlorophyll content was determined using a Minolta SPAD 502 chlorophyll meter and measurement was performed at three time points during the growth period. SPAD meter readings were done on young fully developed leaf. Seven measurements per leaf were taken per plot.
**6) Quantum yield [Fv/ Fm] -** Photosystem II efficiency was measured using the FluorPen-100 fluorometer (Photon System Instruments) at three time points during the growth period. Quantum yield readings were done on young fully developed leaf.
**7) Leaf temperature [°C]** - Leaf temperature was measured at vegetative stages using Fluke IR thermometer 568 device. Measurements were done on a fully developed leaf.
**8) Shoot dry weight (DW) [gr]** - At the end of the experiment (~V5), the above ground plant material was harvested and weighed after 48 hours of drying at 70 °C.

**Table 2**

| ***Microbial strains that improve responses indicative of one or more of the corn trait "Early vigor and biomass establishment" and "Stem conductance", in M1 high throughput corn trait assay. In the list are microbial strains that passed the screen successfully with a minimum of one response improved significantly (2-tails t-test, p-value < 0.2) compared to the non-inoculated control Statistically significant improved responses are marked by an asterisk. Sequential measurement of the same response at different time points are shown as Response_# (for example Lowe Stem width _2).*** | | | | | | | | |
|---|---|---|---|---|---|---|---|---|
| **Microbial strain number** | **Lower Stem width growth rate** | | **Lower Stem width 1** | | **Lower Stem width_2** | | **Lower Stem width 3** | |
| | **% Improvem ent** | **p-value** | **% Improve ment** | **p-value** | **% Improvem ent** | **p-value** | **% Improve ment** | **p-value** |
| EVO32834 | ND | ND | 7.00% * | 0.17 * | 1.00% | 0.881 | 34.00% * | 0.011 * |
| EVO32839 | 11.00% * | 0.109* | ND | ND | ND | ND | 41.00% * | 0.002 * |
| EVO32844 | 32.00% * | 0.112 * | 38.00% * | 0.002 * | 38.00% * | 0.001 * | 8.60% * | 0.016 * |
| EVO33393 | 59.00% * | 0.04 * | 12.00% * | 0.164 * | ND | ND | 8.90% * | 0.015 * |
| EVO33394 | 15.20% | 0.87 | 13.50% * | 0.006 * | 20.00% * | 0.011 * | 3.40% * | 0.079 * |
| EVO33395 | 4.00% | 0.559 | 7.50% * | 0.038 * | 7.20% | 0.287 | 10.00% * | 0.177 * |
| EVO33398 | 15.90% | 0.716 | 1.90% | 0.147 * | 3.20% | 0.55 | 32.00% * | 0.015 * |
| EVO33401 | ND | ND | ND | ND | 6.00% | 0.42 | 10.70% * | 0.008 * |
| EVO33402 | 40.00% * | 0.051 * | 10.00% | 0.432 | 25.00% * | 0.025 * | 3.10% * | 0.119 * |
| EVO33405 | 33.00% * | 0.108 * | 4.00% | 0.731 | 13.00% | 0.233 | 10.10% * | 0.009 * |
| EVO33407 | 22.10% | 0.389 | -0.80% | 0.252 | 11.30% * | 0.123 * | 20.00% * | 0.124 * |
| EVO33410 | 22.30% | 0.523 | 1.60% * | 0.114 * | -3.60% | 0.964 | 13.50% * | 0.003 * |
| EVO33415 | 50.00% * | 0.107 * | -7.00% | 0.236 | -3.00% | 0.643 | 15.80% * | 0.001 * |
| EVO33432 | 25.00% | 0.276 | 2.50% * | 0.128 * | 7.80% | 0.255 | 25.00% * | 0.06 * |
| EVO33441 | 15.2% | 0.757 | -3.4% | 0.397 | -3.5% | 0.844 | 8.6% * | 0.016 * |
| EVO33447 | 27.80% * | 0.191 * | 8.20% * | 0.031 * | 11.40% * | 0.121 * | ND | ND |
| EVO33657 | 24.50% | 0.293 | 10.20% * | 0.017 * | ND | ND | 2.60% * | 0.097 * |
| EVO33661 | 24.10% | 0.308 | 19.60% * | 0.001 * | 10.90% * | 0.134 * | -3.00% | 0.68 |
| EVO40194 | 49% * | 0.086 * | 13% | 0.131 | ND | ND | 31% * | 0.015 * |

| | | | | | | | | |
|---|---|---|---|---|---|---|---|---|
| ND= NO DATA | | | | | | | | |

**Table 3**

| ***Microbial strains that improve responses indicative of the corn trait "Early vigor and biomass establishment" and "Stem conductance", in M1 high throughput corn trait assay. In the list are microbial strains that passed the screen successfully with a minimum of one response improved significantly (2-tails t-test, p-value < 0.2) compared to the non-inoculated control Statistically significant improved responses are marked by an asterisk. Sequential measurement of the same response at different time points are shown as Response_# (for example Lower Stem width_6).*** | | | | | | |
|---|---|---|---|---|---|---|
| **Microbial strain number** | **Lower Stem width 4** | | **Lower Stem width 5** | | **Lower Stem width 6** | |
| | **% Improvement** | **p-value** | **% Improvement** | **p-value** | **% Improvement** | **p-value** |
| EVO32844 | 35.00% * | 0.008 * | 34.00% * | 0.024 * | ND | ND |
| EVO33393 | 41.00% * | 0.001 * | 28.00% * | 0.076 * | 46.00% * | 0.009 * |
| EVO33394 | 10.40% * | 0.065 * | 7.50% | 0.359 | ND | ND |
| EVO33402 | 36.00% * | 0.007 * | 29.00% * | 0.056 * | ND | ND |
| EVO33405 | 24.00% * | 0.071 * | 23.00% * | 0.125 * | ND | ND |
| EVO33407 | 9.80% * | 0.079 * | 12.90% * | 0.11 * | ND | ND |
| EVO33410 | 12.50% * | 0.091 * | 8.70% | 0.257 | ND | ND |
| EVO33415 | 9.00% | 0.412 | 7.00% | 0.571 | 32.00% * | 0.119 * |
| EVO33432 | 7.40% * | 0.154 * | 15.60% * | 0.053 * | ND | ND |
| EVO33447 | 10.70% * | 0.059 * | 19.70% * | 0.015 * | ND | ND |
| EVO33657 | 7.20% * | 0.159 * | 14.90% * | 0.064 * | ND | ND |
| EVO33661 | 14.20% * | 0.018 * | 21.20% * | 0.008 * | ND | ND |
| EVO40194 | 25.00% * | 0.054 * | 33.00% * | 0.039 * | 35.00% * | 0.045 * |

**Table 4**

| ***Microbial strains that improve responses indicative of the corn trait "Early vigor and biomass establishment", in M1 high throughput corn trait assay. In the list are microbial strains that passed the screen successfully with a minimum of one response improved significantly (2-tails t-test, p-value < 0.2) compared to the non-inoculated control. Statistically significant improved responses are marked by an asterisk. Sequential measurement of the same response at different time points are shown as Response_# (for example Plant height_1)*** | | | | | | | | |
|---|---|---|---|---|---|---|---|---|
| **Microbial strain number** | **Plant height growth rate** | | **Plant height_1** | | **Plant height_2** | | **Plant height_3** | |
| | **% Improve ment** | **p-value** | **% Improve ment** | **p-value** | **% Improvem ent** | **p-value** | **% Improvemen t** | **p-value** |
| EVO32844 | 23.00% | 0.347 | 12.00% | 0.218 | 14.00% * | 0.164 * | 18.00% * | 0.064 * |
| EVO33393 | 13.00% | 0.439 | 46.00% * | 0.003 * | ND | ND | 28.00% * | 0.02 * |
| EVO33394 | -8.20% | 0.524 | 14.00% * | 0.076 * | 8.20% * | 0.031 * | 4.90% * | 0.074 * |
| EVO33395 | -9.90% | 0.689 | 15.10% * | 0.054 * | 10.30% * | 0.01 * | 4.10% * | 0.108 * |
| EVO33398 | -11.60% | 0.871 | 5.80% | 0.486 | 5.20% * | 0.122 * | 4.90% * | 0.074 * |
| EVO33402 | -3.00% | 0.895 | 17.00% * | 0.076 * | 23.00% * | 0.031 * | 23.00% * | 0.018 * |
| EVO33405 | 19.00% | 0.445 | 9.00% | 0.337 | 19.00% * | 0.076 * | 25.00% * | 0.011 * |
| EVO33407 | -7.00% | 0.415 | 15.10% * | 0.054 * | 5.20% * | 0.122 * | 3.30% * | 0.153 * |
| EVO33410 | -11.80% | 0.559 | 10.10% | 0.295 | -1.00% | 0.837 | 3.80% * | 0.162 * |
| EVO33415 | 85.00% * | 0.033 * | -6.00% | 0.24 | 1.00% | 0.869 | -2.00% | 0.814 |
| EVO33447 | -6.80% | 0.399 | 12.80% * | 0.105 * | 4.10% * | 0.18 * | 1.60% | 0.283 |
| EVO33661 | ND | ND | 9.30% | 0.246 | 5.20% * | 0.122 * | 5.70% * | 0.05 * |
| EVO40194 | 39.00% * | 0.051 * | 23.00% * | 0.135 * | ND | ND | 23.00% * | 0.056 * |

**Table 5**

| ***Microbial strains that improve responses indicative of the corn trait "Early vigor and biomass establishment", in M1 high throughput corn trait assay. In the list are microbial strains that passed the screen successfully with a minimum of one response improved significantly (2-tails t-test, p-value < 0.2) compared to the non-inoculated control. Statistically significant improved responses are marked by an asterisk. Sequential measurement of the same response at different time points are shown as Response_# (for example Plant height_5).*** | | | | | | | | |
|---|---|---|---|---|---|---|---|---|
| **Microbial strain number** | **Plant height 4** | | **Plant height_5** | | **Plant height 6** | | **Shoot DW** | |
| | **% Improve -ment** | **p-value** | **% Improvement** | **p-value** | **% Improvement** | **p-value** | **% Improvement** | **p-value** |
| EVO32844 | 16.00% * | 0.176 * | 18.00% | 0.268 | ND | ND | 135.00% * | 0.01 * |
| EVO33393 | 25.00% * | 0.003 * | 36.00% * | 0.003 * | 32.00% * | 0.043 * | 88.00% * | 0.062 * |
| EVO33394 | 1.30% * | 0.04 * | 2.20% * | 0.12 * | ND | ND | 10.30% * | 0.056 * |
| EVO33395 | ND | ND | 2.70% * | 0.096 * | ND | ND | 8.60% * | 0.079 * |
| EVO33398 | -8.70% | 0.851 | -1.10% | 0.361 | ND | ND | 4.70% * | 0.162 * |
| EVO33402 | 8.00% | 0.498 | 5.00% | 0.742 | ND | ND | 76.00% * | 0.142 * |
| EVO33405 | 9.00% | 0.43 | 17.00% | 0.295 | ND | ND | 94.00% * | 0.072 * |
| EVO33407 | ND | ND | 3.20% * | 0.077 * | ND | ND | 15.50% * | 0.018 * |
| EVO33415 | 17.00% * | 0.136 * | 30.00% * | 0.051 * | 27.00% * | 0.13 * | 103.00% * | 0.044 * |
| EVO33432 | -4.00% | 0.288 | -1.60% | 0.42 | ND | ND | 10.10% * | 0.059 * |
| EVO33441 | 0.0% | 0.071 | 1.6% | 0.147 | ND | ND | 7.4% * | 0.100 * |
| EVO33447 | ND | ND | 2.20% * | 0.12 * | ND | ND | 16.50% * | 0.014 * |
| EVO33657 | ND | ND | -9.70% | 0.42 | ND | ND | 10.60% * | 0.053 * |
| EVO33661 | 2.00% * | 0.03 * | 3.80% * | 0.061 * | ND | ND | 19.10% * | 0.007 * |
| EVO40194 | 19.00% * | 0.023 * | 38.00% * | 0.002 * | 40.00% * | 0.012 * | 108.00% * | 0.023 * |

**Table 6**

| ***Microbial strains that improve responses indicative of the corn traits "Photosynthetic capacity" and "Leaf transpiration rate", in M1 high throughput corn trait assay. In the list are microbial strains that passed the screen successfully with a minimum of one response improved significantly (2-tails t-test,** p-**value < 0.2) compared to the non-inoculated. Statistically significant improved responses are marked by an asterisk.*** | | | | | | |
|---|---|---|---|---|---|---|
| **Microbial strain number** | **Quantum Yield** | | **SPAD** | | **Leaf temperature** | |
| | **% Improvement** | **p-value** | **% Improvement** | **p-value** | **% Improvement** | **p-value** |
| EVO33393 | ND | ND | 15.00% * | 0.013 * | ND | ND |
| EVO33401 | 3.00% * | 0.197 * | 0.00% | 0.932 | 4.00% * | 0.128 * |
| EVO33405 | ND | ND | 24.00% * | 0.046 * | ND | ND |
| EVO33415 | ND | ND | 15.00% * | 0.14 * | ND | ND |
| EVO33657 | ND | ND | 11.70% * | 0.174 * | ND | ND |
| EVO40194 | ND | ND | 17.00% * | 0.005 * | ND | ND |

**Table 7**

| ***Allocation of M1 responses to specific traits*** | | |
|---|---|---|
| # | **Responses** | **Traits** |
| 1 | Plant height [cm] | Early vigor and biomass establishment |
| 2 | Plant height growth rate [cm/ day] | Early vigor and biomass establishment |
| 3 | Lower stem width [mm] | Early vigor and biomass establishment, Stem conductance |
| 4 | Lower stem width growth [mm/ day] | Early vigor and biomass establishment |
| 5 | SPAD [SPAD units] | Photosynthetic capacity |
| 6 | Quantum yield [Fv/ Fm] | Photosynthetic capacity |
| 7 | Leaf temperature (°C) | Leaf transpiration rate |
| 8 | Dry weight per plant [gr] | Early vigor and biomass establishment |

### Discussion

Listed in Tables 2-6 are 19 microbial strains that improve one or more of the above plant traits in the M1 assay. Some microbial strains, based on the combined results of all modules (see Examples 3 and 4), were selected to be tested under field conditions, and successfully improved plant traits under field conditions.

Table 7 lists the plant responses measured in the M1 traits assay and their allocation to plant traits. These results indicate that microbial strains that improve pre-defined plant traits in the M1 trait assay, can improve also plant traits and plant tolerance to water stress in the field, resulting with a potential increase in yield that could affect the economic benefit one can obtain from the plant in a certain growing area and/or growing time. The majority of the microbial strains improve the responses plant height and/or lower stem width and their respective growth rates and/or shoot dry/fresh weight, all are measures of the plant trait "Early vigor and biomass establishment". Some microbial strains improve the responses SPAD, quantum yield or leaf temperature that represent the plant traits "Photosynthetic capacity" and "Leaf transpiration rate". Some microbial strains improve multiple responses. For example, Microbial strain EVO32844 significantly improved lower stem width growth rate and shoot dry weight by 32 % and 135 % respectively, compared to the non-inoculated control; Microbial strain EVO33661 significantly improved lower stem width growth rate, plant height growth rate and shoot dry weight by 44 %, 71 % and 109 % respectively, compared to the non-inoculated control.

### EXAMPLE 3

### BD: HIGH THROUGHPUT MODEL PLANT YIELD GREENHOUSE ASSAY

This example relates to a description of experiments and results providing additional proofs that microbial strains according to some embodiments of the invention improve plant traits when applied to the environment of the seed during sowing. In this example, described are microbial strains that improve plant traits of the monocot cereal model plant *Brachypodium distachyon* under moderate drought growth conditions (25 % less water than plants grown under normal water treatment). As used in here, the phrase *"Brachypodium"* refers to *Brachypodium distachyon.* The inventors used *Brachypodium* as a model plant for corn and wheat that can be operated in a high throughput manner to screen for microbial strains that improve vegetative and reproductive plant traits. As used in here, the phrase "BD" refers to the high throughput yield assay performed using *Brachypodium* model plant. The BD yield assay tests the ability of microbial strains to improve pre-defined vegetative and reproductive plant traits in the greenhouse, when applied as a co-seed application after sowing. *Brachypodium* has several features that qualify it as a model plant for biostimulants discovery, such as compact physical stature, a short lifecycle, the ability to self-pollinate and simple growth requirements. The inventors developed the BD assay to cost-effectively screen for microbial strains that improve the vegetative and reproductive plant traits:
1) "Stem conductance".
2) "Longer grain-filling duration".
3) "Kernel volume and weight".
4) "Biomass accumulation up to VT".
5) "Increased assimilate partitioning".
6) "Increased kernel number per plant".
7) "Increased yield".

### Experimental procedures

Microbial strains were prepared for the assay by growing them as lawns on four R2G plates [per liter: 0.5 g proteose peptone, 0.5 g casamino acids, 0.5 g yeast extract, 0.5 g dextrose, 0.5 g soluble starch, 0.3 g dipotassium phosphate (K₂HPO₄), 0.05 g, magnesium sulfate (MgSO₄•7H₂O), 0.3 g sodium pyruvate and 8 g gelrite as a gelling agent)] for 2 days at 28 °C, in the dark. Cells were then scraped off the plates and suspended in 200 ml of tap water. Cell concentration in each suspension was determined using a serial dilution in sterile phosphate buffered saline (PBS) and plating on R2G plates and counting and calculating colony-forming units (CFUs) after 2 days of growth. In parallel, *Brachypodium* seeds were sown into 19-22 mm wide and 35 mm high pit germination plugs (Growtech). The plugs were placed in black plastic trays and flooded with water, until plugs were saturated. The trays were placed in a refrigerator where they undergone cold treatment for 3 days at 4°C. Then, the trays were placed in bottom-sealed boxes, 1 tray of 40 plugs per box. The whole 200 ml of the microbial strain cell suspension were then poured over the plugs. Overflow gathered on the bottom of the box was collected by 25 ml pipette dispenser and re-applied to the plugs until all plugs were completely soaked with the microbial suspension. As a non-inoculated control, seeded plugs were treated with tap water only. The trays were then placed in the greenhouse for hardening under mist conditions until the seeds had germinated (1 week). Plugs with emerging seedlings were planted in 3.1 liter planters, in rows of 6 plugs per planter with a total of 6 planter replicates per each tested Microbial strain (n=6). Plants were grown under moderate drought stress (25 % less water than plants grown under normal water treatment) up to grain maturity. Plant responses to the microbial strains were measured in heading (spikelet emergence) and at harvest (seed maturation), covering the vegetative and reproductive traits listed in Table 12. Microbial strains are consider to successfully pass the experiment if they improve at least one plant response over the non-inoculated control with *p*-value < 0.2 (2-tails t-test).

### Measured responses in BD HTP Brachypodium yield assay:

**1. Plant height [cm]** - Plant height was measured using a measuring type at harvest, from ground level to the spikelet base of the longest spikelet of each plant.
**2. Vegetative dry weight [gr]** - The weight of the above ground vegetative plant material of the four central plant of each plot, without the spikelets, after 48 hours of drying in an oven in 70°C, divided by the number of measures plant per plot (four).
**3. Total dry mater per plant [gr]** - A calculation of spikelets dry weight [gr] plus vegetative dry weight [gr] per plot, divided by the number of measured plants per plot (four).
**4. Spikelets dry weight [gr]** - The weight spikelets of the four central plant of each plot, after 48 hours of drying in an oven in 70°C, divided by the number of measures plant per plot (four).
**5. Total grains yield per plant [gr]** - The weight of the grains from the dry spikelets per plot, divided by the number of plants per plot.
**6. Grain number** - The number of the grains from the dry spikelets per plot as from an image, divided by the number of plants per plot.
7. **Peduncle thickness [mm]** - Peduncle (the middle of the internode below the first spikelet) dimeter was measured using micro caliber.
**8. Rachis dimeter [mm]** - Rachis (the node above the first spikelet) dimeter was measured at harvest using micro caliber.
**9. Harvest index per plant** - Grain yield per plant divided by the total dry matter per plant.
**10. 1,000-grain weight [gr]** - A calculation of total grain yield per plant [gr] divided by the grain number, multiply by 1000.
**11. Number of days to heading [days]** - Number of days to heading was calculated as the number of days from sowing until 50 % of plants in the plot arrive heading (emergence of the first head).
**12. Grain filling duration [days]** - The number of days to reach maturity stage subtracted by the number of days to reach heading stage. Maturity stage is defined as the changing in the color of spikelets from green to yellow in 50 % of plants in a plot.

**Table 8**

| ***Microbial strains that improve responses indicative of the Brachypodium traits "Biomass accumulation up to VT" in BD High Throughput Brachypodium yield assay. In the list are microbial strains that passed the screen successfully with a minimum of one response improved significantly (2-tails t-test, p-value < 0.2) compared to the non-inoculated control. Statistically significant improved responses are marked by an asterisk.*** | | | | | | |
|---|---|---|---|---|---|---|
| **Microbial strain number** | **Plant height** | | **Vegetative dry weight** | | **Total dry mater per plant** | |
| | **% Improvement** | **p-value** | **% Improvement** | **p-value** | **% Improvement** | **p-value** |
| EVO32828 | 2.00% | 0.752 | 13.00% | 0.277 | 14.00% * | 0.107 * |
| EVO32834 | 3.20% | 0.498 | 35.90% * | 0.001 * | 21.70% * | 0.037 * |
| EVO32839 | 4.30% | 0.325 | 18.00% * | 0.109 * | 16.30% * | 0.067 * |
| EVO32844 | 4.60% | 0.371 | 22.70% * | 0.053 * | 9.50% | 0.323 |
| EVO32845 | 8.00% * | 0.091 * | 13.00% * | 0.184 * | 15.00% * | 0.046 * |
| EVO33393 | 10.00% * | 0.036 * | 12.00% | 0.231 | 14.00% * | 0.058 * |
| EVO33395 | 5.00% | 0.21 | 15.00% * | 0.175 * | 14.00% * | 0.059 * |
| EVO33401 | 7.00% * | 0.164 * | 19.00% * | 0.062 * | 17.00% * | 0.025 * |
| EVO33402 | 4.00% | 0.395 | 24.00% * | 0.019 * | 11.00% * | 0.17 * |
| EVO33410 | 7.00% | 0.316 | 21.00% * | 0.146 * | 25.00% * | 0.041 * |
| EVO33661 | 2.00% | 0.491 | 12.00% | 0.204 | 10.00% * | 0.167 * |
| EVO33746 | 1.40% | 0.768 | 15.60% * | 0.046 * | 8.70% * | 0.175 * |
| EVO33887 | 6.00% * | 0.197 * | 2.00% | 0.800 | 7.00% | 0.460 |

**Table 9**

| ***Microbial strains that improve responses indicative of the Brachypodium traits "Increased kernel number per plant" and "Increased yield" in BD High Throughput Brachypodium yield assay. In the list are microbial strains that passed the screen successfully with a minimum of one response improved significantly (2-tails t-test, p-value < 0.2) compared to the non-inoculated control Statistically significant improved responses are marked by an asterisk.*** | | | | | | |
|---|---|---|---|---|---|---|
| **Microbial strain number** | **Spikelets dry weight** | | **Total Grains yield per plant** | | **Grain number** | |
| | **% Improvement** | **p-value** | **% Improvement** | **p-value** | **% Improvement** | **p-value** |
| EVO11090 | ND | ND | 14.00% * | 0.114 * | 11.00% * | 0.152 * |
| EVO32828 | 15.00% * | 0.125 * | 13.00% | 0.249 | 14.00% * | 0.163 * |
| EVO32831 | 24.00% * | 0.087 * | 32.00% * | 0.041 * | 26.00% * | 0.068 * |
| EVO32839 | 14.50% * | 0.116 * | 13.20% * | 0.199 * | 16.80% * | 0.087 * |
| EVO32844 | ND | ND | ND | ND | 0.90% | 0.939 |
| EVO32845 | 17.00% * | 0.024 * | 16.00% * | 0.071 * | 17.00% * | 0.029 * |
| EVO32868 | 16.30% * | 0.121 * | 19.40% * | 0.11 * | 22.70% * | 0.047 * |
| EVO33887 | ND | ND | 18.00% * | 0.151 * | 21.00% * | 0.086 * |
| EVO33393 | 17.00% * | 0.023 * | 19.00% * | 0.037 * | 17.00% * | 0.037 * |
| EVO33395 | ND | ND | ND | ND | 14.00% * | 0.096 * |
| EVO33398 | 16.00% * | 0.121 * | 6.00% | 0.572 | 4.00% | 0.696 |
| EVO33401 | 15.00% * | 0.116 * | 15.00% | 0.224 | 14.00% | 0.201 |
| EVO33410 | 29.00% * | 0.041 * | 30.00% * | 0.06 * | 23.00% * | 0.103 * |
| EVO33441 | 12.0% | 0.223 | 19.0% | 0.091 | 11.0% | 0.225 |
| EVO33872 | ND | ND | 23.00% * | 0.030 * | 16.00% * | 0.090 * |
| EVO40185 | ND | ND | 14.00% | 0.207 | 15.00% * | 0.139 * |
| EVO40194 | ND | ND | 11.00% * | 0.188 * | 10.00% | 0.219 |

**Table 10**

| ***Microbial strains that improve responses indicative of the Brachypodium traits "Stem conductance" and "Increased assimilate partitioning" in BD High Throughput Brachypodium yield assay. In the list are microbial strains that passed the screen successfully with a minimum of one response improved significantly (2-tails t-test, p-value < 0.2) compared to the non-inoculated control Statistically significant improved responses are marked by an asterisk.*** | | | | | | |
|---|---|---|---|---|---|---|
| **Microbial strain number** | **Peduncle thickness** | | **Rachis width** | | **Harvest index** | |
| | **% Improvement** | **p-value** | **% Improvement** | **p-value** | **% Improvement** | **p-value** |
| EVO11090 | ND | ND | ND | ND | 13.00% * | 0.117 * |
| EVO32831 | ND | ND | ND | ND | 18.00% * | 0.057 * |
| EVO32844 | 24.70% * | 0.107 * | 28.50% * | 0.076 * | ND | ND |
| EVO32868 | -2.30% | 0.877 | 4.50% | 0.778 | 9.20% * | 0.144 * |
| EVO33398 | 20.00% * | 0.187 * | 22.00% * | 0.178 * | 3.00% | 0.699 |
| EVO33441 | ND | ND | ND | ND | 13.0% * | 0.093 * |
| EVO33872 | 0.00% | 0.977 | ND | ND | 13.00% * | 0.079 * |
| EVO33887 | 6.00% | 0.487 | 1.00% | 0.877 | 11.00% * | 0.097 * |
| EVO40185 | 23.00% * | 0.013 * | 26.00% * | 0.048 * | 9.00% | 0.269 |
| EVO40194 | ND | ND | ND | ND | 22.00% * | 0.003 * |

**Table 11**

| ***Microbial strains that improve responses indicative of the Brachypodium traits "Kernel volume and weight" and "Longer grain filling duration" in BD High Throughput Brachypodium yield assay. In the list are microbial strains that passed the screen successfully with a minimum of one response improved significantly (2-tails t-test, p-value < 0.2) compared to the non-inoculated control. Statistically significant improved responses are marked by an asterisk.*** | | | | | | |
|---|---|---|---|---|---|---|
| **Microbial strain number** | **1000 grain weight** | | **Number of days heading** | | **Grain fill duration** | |
| | **% Improvement** | **p-value** | **% Improvement** | **p-value** | **% Improvement** | **p-value** |
| EVO32828 | -1.00% | 0.813 | -7.00% * | 0.062 * | 9.00% * | 0.01 * |
| EVO32831 | 8.00% * | 0.025 * | -10.00% * | 0.003 * | 6.00% * | 0.153 * |
| EVO32834 | 2.50% | 0.598 | -1.60% | 0.529 | 11.10% * | 0.003 * |
| EVO33394 | 8.00% * | 0.094 * | -13.00% * | 0 * | 5.00% * | 0.146 * |
| EVO33398 | 2.00% | 0.656 | -5.00% * | 0.11 * | 1.00% | 0.794 |
| EVO33405 | 1.00% | 0.806 | -3.00% * | 0.1 * | 0.00% | 0.919 |
| EVO33410 | 7.00% * | 0.065 * | -7.00% * | 0.046 * | 6.00% * | 0.132 * |
| EVO33432 | 1.00% | 0.909 | -6.00% * | 0.044 * | 2.00% | 0.521 |
| EVO33441 | 6.0% | 0.298 | -11.0% * | 0.000 * | 3.0% | 0.378 |
| EVO33661 | 2.00% | 0.728 | -9.00% * | 0.003 * | 5.00% * | 0.148 * |
| EVO33746 | 12.70% * | 0.054 * | 2.00% | 0.413 | 1.40% | 0.734 |
| EVO33872 | 6.00% * | 0.116 * | -2.00% | 0.888 | -1.00% | 0.943 |
| EVO33887 | -1.00% | 0.797 | ND | ND | 8.00% * | 0.017 * |
| EVO40194 | 1.00% | 0.707 | -9.00% * | 0.007 * | 0.00% | 0.993 |

**Table 12**

| ***Allocation of BD assay measured plant responses to specific plant traits.*** | | |
|---|---|---|
| # | **Plant responses** | **Plant traits** |
| 1 | Plant height [cm] | Biomass accumulation up to VT |
| 2 | Vegetative dry weight [gr] | Biomass accumulation up to VT |
| 3 | Total dry mater per plant [gr] | Biomass accumulation up to VT |
| 4 | Spikelets dry weight [gr] | Increased kernel number per plant and increased yield |
| 5 | Total grains yield per plant [gr] | Increased yield |
| 6 | Grain number | Increased kernel number per plant |
| 7 | Peduncle thickness [mm] | Stem conductance |
| 8 | Rachis dimeter [mm] | Stem conductance |
| 9 | Harvest Index | Increased assimilate partitioning |
| 10 | 1000 grain weight | Kernel volume and weight |
| 11 | Number of days to heading [days] | Longer grain filling duration |
| 12 | Grain filling period (days) | Longer grain filling duration |

### Discussion

Listed in Tables 8-11 are 24 microbial strains that improve one or more of the above plant traits in the BD trait and yield assay. Some microbial strains were selected, based on combined results from all screening assays (see Examples 2 and 4), to be tested under field conditions and were proven to improve plant traits also under field conditions (see Example 5). These results indicate that microbial strains that improve pre-defined plant traits in the BD trait and yield assay, can improve plant traits and plant tolerance to water stress in the field, resulting with an increased yield that could affect the economic benefit one can obtain from the plant in a certain growing area and/or growing time. Table 12 presents plant responses measured in the BD traits and yield assay and their allocation to specific plant traits. Among the 24 microbial strains listed in Tables 8-11, are 16 microbial strains that are also listed in Tables 2-6. These 16 microbial strains passed successfully both M1 and BD assays indicating that the majority of the microbial strains can improve the performance of multiple plant species (*Zea maize* and *Brachypodium distachyon*) and may exhibit similar plant genetic stability when applied as a seed treatment to other plant species. Several of the overlapping microbial strains improve the only plant trait that is measured in both assays ("Stem conductance"), an indication of similar functions provided to different plant species. Similarly, shoot biomass related responses measures in the M1 assay (dry weight per plant) and in the BD assay (vegetative dry weight and total dry mater per plant), were improved by similar microbial strains indicating again of similar functions provided to different plant species, in this case, functions that represent the plant traits "Early vigor and biomass accumulation" (M1) and "Biomass accumulation up to VT" (BD). The BD trait and yield assay is mostly complementary to the M1 trait assay. It addresses many new plan traits that are not addressed by the M1 trait assay. These traits are reproductive traits that provide data regarding the impact of the microbial strain on grain production.

### EXAMPLE 4

### M2: LOW-THROUGHPUT (LTP) CORN TRAIT/ YIELD ASSAY

This example is a description of experiments and results providing additional proofs that microbial strains of some embodiments of the invention improve plant traits when applied to the environment of the seed during sowing. In this example, the inventors describe microbial strains that improve responses related to the following corn traits:
1) "Early vigor and biomass establishment".
2) "Stem conductance".
3) "Photosynthetic capacity".
4) "Biomass accumulation up to VT".
5) "Main ear size".
7) "Increased yield".

The inventors produced these results using a Low-Throughput (LTP) greenhouse-screening assay designated M2. As used in here, the phrase "M2" refers to a plant trait and yield assay testing the ability of microbial strains to improve pre-defined vegetative and reproductive plant traits in the greenhouse, when applied as a co-seed application.

### Experimental procedures

In the M2 trait and yield assay, microbial strains were tested in five replicates, each replicate consisted of six plants, each co-seeded with 1 ml of microbial strain suspension and grown in 50-liter planters under moderate drought conditions (25% less water than plants grown under normal water treatment) up to grain harvest. microbial strains were grown in the laboratory as a lawn on six R2G plates for 2 days on 28°C in the dark. Cells were collected from plates and suspended in 100 ml tap water. Cell concentration in each suspension was determined using serial dilution in sterile phosphate buffered saline (PBS) and plating on R2G plates and counting and calculating colony forming units (CFUs) after 2 days of growth. In the greenhouse, 50-liter planters were filled with agricultural field soil. Corn seeds (Pioneer 37N01 or Limagrain LG3713) were co-seeded into soil with 1 ml of cell suspension (~10⁸ CFU/ ml). As a non-inoculated control, seeds were treated with tap water only. Each microbial strain treatment was tested in 5 replicates (five planters; n *=* 5). Post germination, plants were grown under moderate drought stress (25% less water than the normal water treatment of the technical controls) up to the stage of seed maturation. Vegetative and reproductive responses were measured during growth including, plant height and lower stem width that were measured once every two weeks up to the stage of 8 leaves (V8, starting from week 2 post sowing). SPAD measurements were taken at 3 time points during vegetative growth, total shoot dry weight was measured upon harvest. Additional responses were measured after harvest including ear dry weight and grain yield per plant. microbial strains were considered to successfully pass the experiment if they improved at least one plant response in comparison to the non-inoculated control with *p*-value < 0.2 (2-tails t-test).

### Measured responses in M2 LTP corn trait/yield assay:

**1. Plant height [cm]** - Plant height was measured once every 2 weeks at five time points up to V8. At each time point, the four central plants of each plot were measured using a measuring tape starting from ground level to the top of the longest leaf.
**2. Plant height growth rate** [cm/ **day]** - A calculation from plant height [cm] measurements. Rate is calculated by dividing the change in plant height over that time period by the time interval.
**3. Lower stem width [mm]** - Lower stem width was measured once every 2 weeks at five time points up to V8 and once more in flowering (VT). At each time point, the diameter of the stem in the lower internode of the four central plants of each plot was measured.
**4. Lower stem width growth rate [mm/ day] -** A calculation from lower stem width [cm] measurements. Rate is calculated by dividing the change in stem width over that period by the time interval.
**5. SPAD [SPAD units] -** Chlorophyll content was determined using a Minolta SPAD 502 chlorophyll meter and measurement was performed at three time points during the growth period. SPAD meter readings were done on young fully developed leaf. Seven measurements per leaf were taken per plant.
**6. Vegetative dry weight per plant [gr] -** The weight of the above ground vegetative plant material of the four central plant of each plot, without the ears, after 48 hours of drying in an oven in 70 °C, divided by the number of measures plant per plot (four).
**7. Main ear dry weight per plant [gr]** - The weight of the ears removed from the four central plant of each plot after 48 hours of drying in an oven in 70 °C, divided by the number of plants per plot (four).
**8. Total dry matter per plant [gr]** - Vegetative dry weight per plot + ears dry weight per plot, divided by the number of measured plants per plot (four).
**9. Main ear grain yield per plant [gr] -** The weight of the grains manually removed from the dry main ears.

**Table 13**

| ***Microbial strains that improve responses indicative of the corn trait "Early vigor and biomass accumulation" in M2 Low Throughput corn trait*/*yield assay. In the list are microbial strains that passed the screen successfully with a minimum of one response improved significantly (2-tails t-test, p-value < 0.2) compared to the non-inoculated control. Statistically significant improved responses are marked by an asterisk.*** | | | | | | | |
|---|---|---|---|---|---|---|---|
| **Microbial strain number** | **Corn variety** | **Plant height_4** | | **Plant height_5** | | **Plant height growth rate** | |
| | | **% Improvement** | **p-value** | **% Improvement** | **p-value** | **% Improvement** | **p-value** |
| EVO32845 | LG3713 | 9% * | 0.0516 * | **4%** | 0.445 | 3% | 0.7915 |
| EVO33398 | LG3713 | 8% * | 0.0714 * | 6% | 0.2819 | 11% | 0.3655 |
| EVO33405 | LG3713 | 12% * | 0.009 * | 9% * | 0.1013 * | 17% * | 0.1624 * |

**Table 14**

| ***Microbial strains that improve responses indicative of the corn traits "Early vigor and biomass accumulation" and "Stem conductance" in M2 Low Throughput corn trait*/*yield assay. In the list are microbial strains that passed the screen successfully with a minimum of one response improved significantly (2-tails t-test, p-value < 0.2) compared to the non-inoculated control Statistically significant improved responses are marked by an asterisk.*** | | | | | |
|---|---|---|---|---|---|
| **Microbial strain number** | **Corn variety** | **Lower Stem width** | | **Lower Stem width growth rate** | |
| | | **% Improvement** | **p-value** | **% Improvement** | **p-value** |
| EVO32845 | LG3713 | 9% * | 0.09 * | 25% * | 0.0811 * |
| EVO33398 | LG3713 | 11% * | 0.0351 * | 29% * | 0.0406 * |
| EVO33405 | LG3713 | 11% * | 0.1199 * | 24% * | 0.0927 * |

**Table 15**

| ***Microbial strains that improve responses indicative of the corn traits "Biomass accumulation up to VT" in M2 low throughput corn trait*/*yield assay. In the list are microbial strains that passed the screen successfully with a minimum of one response improved significantly (2-tails t-test, p-value < 0.2) compared to the non-inoculated control. Statistically significant improved responses are marked by an asterisk.*** | | | | | |
|---|---|---|---|---|---|
| **Microbial strain number** | **Corn variety** | **Vegetative dry weight per plant** | | **Total dry matter per plant** | |
| | | **% Improvement** | **p-value** | **% Improvement** | **p-value** |
| EVO32839 | 37N01 | 16% * | 0.0821 * | 22% * | 0.0156 * |
| EVO32845 | 37N01 | 16% * | 0.0151 * | 10% * | 0.0732 * |
| EVO32845 | LG3713 | 43% * | 0.173 * | 6% | 0.3661 |
| EVO33398 | 37N01 | 55% * | 0.0255 * | 2% | 0.8664 |
| EVO33398 | LG3713 | 46% * | 0.1443 * | 2% | 0.7844 |
| EVO33405 | 37N01 | 50% * | 0.04 * | ND | ND |
| EVO33405 | LG3713 | 49% * | 0.1238 * | ND | ND |

**Table 16**

| ***Microbial strains that improve responses indicative of the corn trait "Photosynthetic capacity" in M2 low throughput corn trait*/*yield assay. In the list are microbial strains that passed the screen successfully with a minimum of one response improved significantly (2-tails t-test, p-value < 0.2) compared to the non-inoculated control. Statistically significant improved responses are marked by an asterisk.*** | | | |
|---|---|---|---|
| **Microbial strain number** | **Corn variety** | **SPAD** | |
| | | **% Improvement** | **p-value** |
| EVO32834 | 37N01 | 6% * | 0.0558 * |
| EVO33405 | 37N01 | 9% * | 0.1019 * |
| EVO33405 | LG3713 | 7% * | 0.1948 * |

**Table 17**

| ***A microbial strain that improve responses indicative of the corn traits "Main ear size" and "Increased kernel number per plant and yield" and "Increased yield" in M2 low throughput corn trait*/*yield assay.*** | | | | | |
|---|---|---|---|---|---|
| ***In the list are microbial strains that passed the screen successfully with a minimum of one response improved significantly (2-tails t-test, p-value < 0.2) compared to the non-inoculated control.*** | | | | | |
| ***Statistically significant improved responses are marked by an asterisk.*** | | | | | |
| **Microbial strain number** | **Corn variety** | **Main ear dry weight per plant** | | **Main ears grain yield per plant** | |
| | | **% Improvement** | **p-value** | **% Improvement** | **p-value** |
| EVO32839 | 37N01 | 34% * | 0.0013 * | 27% * | 0.0084 * |

**Table 18**

| ***Allocation of M2 plant responses to specific plant traits*** | | |
|---|---|---|
| # | **Responses** | **Traits** |
| 1 | Plant height [cm] | Early vigor and biomass establishment |
| 2 | Plant height growth rate [cm/ day] | Early vigor and biomass establishment |
| 3 | Lower stem width [mm] | Early vigor and biomass establishment, Stem conductance |
| 4 | Lower stem width [mm/ day] | Early vigor and biomass establishment |
| 5 | SPAD [SPAD units] | Photosynthetic capacity |
| 6 | Vegetative dry weight per plant [gr] | Biomass accumulation up to VT |
| 7 | Main ear dry weight per plant [gr] | Main ear size |
| 8 | Total dry matter per plant [gr] | Biomass accumulation up to VT |
| 9 | Main ear grain yield per plant [gr] | Increased yield |

### Discussion

Listed in Tables 13-17 are 5 microbial strains that improve one or more of the above plant traits in the M2 trait and yield assay and were selected based on the overall results obtained from all screening assay, to be tested under field conditions and pass the field test successfully. Table 18 describes the plant responses and traits improved by the microbial strains in this assay and the allocation of plant responses to specific plant traits. These results indicate that microbial strains that improve pre-defined plant traits in the M2 trait and yield assay, can improve plant traits and plant tolerance to water stress in the field, resulting with an increased yield that could affect the economic benefit one can obtain from the plant in a certain growing area and/or growing time. The M2 trait and yield assay tests the ability of microbial strains to improve both vegetative and reproductive plant responses and plant traits in corn plants growing in populations (6 plants together), under moderate drought condition in the greenhouse up to seed maturation. Improvement of vegetative responses and traits such as "Early vigor and biomass establishment", "Stem conductance" and "Photosynthetic capacity" are all evidence for functions that the microbial strains provide to the plant that improve the tolerance of the plant to water stress. Improvement of reproductive traits is an evidence that the early impact and improvement on plant development has an influence on the reproduction on the plant. All of the five microbial strains listed also in Tables 8-11 among the microbial strains that successfully passed the BD assay. Four of the microbial strains listed (EVO33398, EVO33405, EVO32839 and EVO32834) appear in Tables 2-6 among the microbial strains that successfully passed the M1 screen module. These four microbial strains passed the three M1, BD and M2 screening assays successfully. Three microbial strains improved the trait "Biomass accumulation up to VT" in two different corn genetic background (37N01 and LG3713). These results indicate that microbial strains described in this invention can improve multiple plant traits (vegetative and reproductive) in multiple plant species and genetic varieties and may improve similarly other plant species and varieties used in agriculture.

### EXAMPLE 5

### F: FIELD TRAIT AND YIELD ASSAY

This example is a description of field experiments and results providing additional proofs that microbial strains of some embodiments of the invention improve plant traits when applied to the seed as a seed coat prior to sowing as a single microbial strain seed coat treatment. In this example, the inventors validated the efficacy of the most promising microbial strains discovered in the M1, BD and M2 screening assay in field experiments. In these experiments, microbial strains were applied as seed coats and tested for the ability to improve pre-defined target plant traits under moderate drought treatment applied between the stage of flowering (VT) and the harvest (H):
1) "Early vigor and biomass establishment".
2) "Stem conductance".
3) "Leaf transpiration rate".
4) "Photosynthetic capacity".
5) "Maintain total biomass under stress".
6) "Main ear size".
7) "Kernel volume and weight".
8) "Cob conductance".
9) "Increased yield".

### Experiment procedures

All microbial strains that significantly improve the pre-defined target plant traits in any of the M1, BD and M2 screening assay participated in the nomination for validation in field experiments. Nominated microbial strains exhibited one of the following criteria:
1) microbial strains that passed successfully in multiple screening assays (M1, BD and/or M2).
2) microbial strains that passed successfully the same screening assay multiple times.
3) microbial strains that consistently improved the same plant traits across screening assays (M1, BD and/or M2).
Microbial strains were grown as a lawn on five R2A plates each [per liter: 0.5 g proteose peptone, 0.5 g casamino acids, 0.5 g yeast extract, 0.5 g dextrose, 0.5 g soluble starch, 0.3 g dipotassium phosphate (K₂HPO₄), 0.05 g, magnesium sulfate (MgSO₄•7H₂O), 0.3 g sodium pyruvate and 8 g gelrite as a gelling agent] for 2 days at 28 °C in the dark. The bacteria were then collected and suspended in 20 ml sterile R2A broth (with the gelrite) supplemented with 25 % glycerol and kept on -80°C until use. Two weeks before field sowing, each strain was cultured on 25 R2G plates and regrown for 2 days at 28 °C in the dark. Cells were then harvested and suspended in 50 ml tap water supplemented with 2% Carboxy Methyl Cellulose (CMC) serving as a gluing agent. This suspension was mixed with corn seeds (Pioneer 37N01, 1498 and 2088 and Limagrain 3713) in a ratio of 1 part of suspension (in gr) for every 20 parts of seeds (in gr). As a control, seeds were incubated with the water-CMC solution only. The mixture was shaken gently for 10 min and thereafter the seeds were separated and dried on a paper towel in a biological cabinet for few hours. The average number of CFUs on seeds was measured 3 days before sowing to insure a titer of >10⁵ CFUs/ seed by vigorously mixing five coated seeds in PBS to release bacteria from coat, serially diluting the sample, plating the dilutions on R2G plates and enumerated CFUs 2 days after.

### Sowing and plant growth:

Coated corn seeds (with bacteria and control) were sown using a manual seed planter. Untreated seeds were used as a second control that was not treated with neither bacteria, nor water-CMC solution. The planter box was cleaned between seed batches using 70 % ethanol and rinsing with tap water to eliminate ethanol traces. Each combination of seed with microbial strain coat including the control was sown in six replicated plots (n=6) in a random blocks statistical design. Each plot was comprised of two paralleled rows of 4 meters each, in a density of 10 seeds/ per meter (total of 80 seeds per plot, 480 seeds total per each treatment with a microbial strain coat). Plants were grown under commercial fertilization and irrigation protocol (between 30 to 40 m³ water per 1000 m² every week with 4 kg of Nitrogen per 1000 m²) up to VT (flowering), when moderate drought treatment was applied (25% less water than commercial protocol). In order to discover microbial strains that improve vegetative and reproductive pre-defined plant responses and plant traits in the field, plant responses and yield parameters were measured during the experiments.

### Measured Responses in field corn trait assay:

**1) Lower stem width [mm]** - Plants were characterized for lower stem width once every two weeks at five time points during growth period. The diameter of the stem was measured in the lower internode.
**2) Middle stem width [mm]** - Measurement of the width in the middle of the internode below the main ear with a caliper was take at VT (flowering).
**3) Leaf temperature [°C] -** Measurement of leaf temperature at R2 using FLUKE 568 IR thermometer from leaf above the main ear.
**4) Quantum yield [Fv/ Fm] -**
**5) SPAD [SPAD units]** - Chlorophyll content was determined using a Minolta SPAD 502 chlorophyll meter. SPAD meter readings were done on young fully developed leaf. Seven measurements per leaf were taken per plot.
**6) Vegetative dry weight per plant [gr] -** The weight after 48 hours drying at 70 °C, of the above ground vegetative plant material without the ears per plot divided by the number of plant per plot.
**7) Total dry matter per plant [gr]** - The weight after 48 hours drying at 70 °C, of the whole plants (vegetative and reproductive parts) per plot, divided by the number of plants per plot.
**8) Vegetative dry weight per area** - Vegetative dry weight of plants from known harvest area (eg. 1.5 m²)
**9) Total dry matter per area** - Yield and vegetative dry weight of plants from known harvest area
**10) Main ears dry weight per plant [gr]** - The weight after 48 hours drying st 70 °C, of the ears per plot, divided by the number of plants per plot.
**11) Main ear area [cm²]** - Main ears from 15 plants per plot were photographed in a controlled light environment using interchangeable lens digital cameras Canon DSLR EOS700D and the area of the ears was calculated from the images using proprietary algorithms that were developed using the Java open source software named ImageJ developed by NIH. The average area of a single ear was calculated by dividing the total area by the number of ears in the images.
**12) Main ear width [cm] -** Main ears from 15 plants per plot were photographed in a controlled light environment using interchangeable lens digital cameras Canon DSLR EOS700D and the width of the ears was calculated from the images using proprietary algorithms that were developed using the Java open source software named ImageJ developed by NIH. The average width of a single ear was calculated by dividing the total width by the number of ears in the image.
**13) Main ear length [cm] -** Main ears from 15 plants per plot were photographed in a controlled light environment using interchangeable lens digital cameras Canon DSLR EOS700D and the length of the ears was calculated from the images using proprietary algorithms that were developed using the Java open source software named ImageJ developed by NIH. The average length of a single ear was calculated by dividing the total width by the number of ears in the image.
**14) Main ear row num -** the average manual count of rows from 15 ears per plot
**15) 1,000 grains weight [gr]** - The ratio between the main ear grain yield per plant, divided by the number of grains per plant, multiply by 1,000.
**16) Grain area [cm²]** - A sample of ~200 grain was photographed in a controlled light environment using interchangeable lens digital cameras Canon DSLR EOS700D and their area was calculated from the image using proprietary algorithms that were developed using the Java open source software named ImageJ developed by NIH. The average area of a single grain was calculated by dividing the total area by the number of grains in the image.
**17) Cob width [cm] -** Main ears from 15 plants per plot were threshed, grains and cobs were separated, cobs photographed in a controlled light environment using interchangeable lens digital cameras Canon DSLR EOS700D and the width of the cobs was calculated from the images using proprietary algorithms that were developed using the Java open source software named ImageJ developed by NIH. The average width of a single cob was calculated by dividing the total width by the number of cobs in the image.
**18) Main ear grain yield per plant [gr]** - The weight of the grains that were manually removed from the main ears per plot, divided by the number of plants per plot.
**19) Total grain yield per plant [gr] -** The weight of the grains that were manually removed from the main and secondary ears per plot, divided by the number of plants per plot
**20) Bushels per acre** - conversion of grain yield per plot (in kg per area unit) harvest by combine, to bushels per acre.

**Table 19**

| ***Microbial strains that improve responses indicative of the corn traits "Early vigor and biomass establishment" and "Stem conductance" in the F field trait and yield assay. In the list are microbial strains that passed the experiment successfully with a minimum of one response improved significantly (2-tails t-test, p-value < 0.2) compared to the non-inoculated control.*** | | | | | |
|---|---|---|---|---|---|
| ***Statistically significant improved responses are marked by an asterisk.*** | | | | | |
| **Microbial strain number** | **Variety** | **Lower stem width** | | **Middle stem width** | |
| | | **% Improvement** | **p-value** | **% Improvement** | **p-value** |
| EVO32844 | 37N01 | 5% * | 0.134 * | 12% * | 0.0023 * |
| EVO32845 | 37N01 | 1% | 0.7856 | 7% * | 0.0645 * |
| EVO33398 | 37N01 | 0% | 0.9654 | 7% * | 0.044 * |
| EVO33402 | 37N01 | ND | ND | 8% * | 0.0409 * |
| EVO33405 | 37N01 | 2% | 0.4646 | 4% * | 0.1741 * |
| EVO33441 | 37N01 | 3% | 0.5406 | 7% * | 0.1545 * |
| EVO33661 | 37N01 | -1% | 0.8404 | 9% * | 0.0241 * |

**Table 20**

| ***Microbial strains that improve responses indicative of the corn traits "Photosynthetic capacity" and "Leaf transpiration rate" in the F field trait and yield assay. In the list are microbial strains that passed the experiment successfully with a minimum of one response improved significantly (2-tails t-test, p-value < 0.2) compared to the non-inoculated control. Statistically significant improved responses are marked by an asterisk.*** | | | | | | | |
|---|---|---|---|---|---|---|---|
| **Microbial strain number** | **Variety** | **Leaf temperature** | | **Quantum yield** | | **SPAD** | |
| | | **% Improvement** | **p-value** | **% Improvement** | **p-value** | **% Improvement** | **p-value** |
| EVO32844 | 37N01 | 1% | 0.4513 | 2% * | 0.1183 * | ND * | ND * |
| EVO33405 | 37N01 | 2% * | 0.1336 * | 0% | 0.7055 | ND | ND |
| EVO33872 | 37N01 | 2% * | 0.068 * | ND | ND | ND | ND |
| EVO33872 | 1498 | ND | ND | ND | ND | 2% * | 0.03 * |
| EVO33872 | 2088 | 2% * | 0.17 * | ND | ND | ND | ND |
| EVO40194 | 37N01 | 2% * | 0.134 * | ND | ND | ND | ND |
| EVO40185 | 37N01 | 3% * | 0.036 * | ND | ND | ND | ND |

**Table 21**

| ***Microbial strains that improve responses indicative of the corn trait "Maintain total biomass under stress" in the F field trait and yield assay. In the list are microbial strains that passed the experiment successfully with a minimum of one response improved significantly (2-tails t-test, p-value < 0.2) compared to the non-inoculated control Statistically significant improved responses are marked by an asterisk.*** | | | | | |
|---|---|---|---|---|---|
| **Microbial strain number** | **Variety** | **Vegetative dry weight per plant** | | **Total dry matter per plant** | |
| | | **% Improvement** | **p-value** | **% Improvement** | **p-value** |
| EVO32844 | 37N01 | 22% * | 0.016 * | 23% * | 0.0024 * |
| EVO32845 | 37N01 | 15% * | 0.082 * | 11% * | 0.1 * |
| EVO33398 | 37N01 | 15% * | 0.0435 * | 11% * | 0.0465 * |
| EVO33405 | 37N01 | 8% | 0.2079 | 10% * | 0.0742 * |
| EVO33661 | 37N01 | 6% | 0.4267 | 11% * | 0.1178 * |
| EVO40185 | 37N01 | 1% | 0.812 | 12% * | 0.054 * |

**Table 22**

| ***Microbial strains that improve responses indicative of the corn trait "Maintain total biomass under stress" in the F field trait and yield assay. In the list are microbial strains that passed the experiment successfully with a minimum of one response improved significantly (2-tails t-test, p-value < 0.2) compared to the non-inoculated control Statistically significant improved responses are marked by an asterisk.*** | | | | | |
|---|---|---|---|---|---|
| **Microbial strain number** | **Variety** | **Vegetative dry weight per area** | | **Total dry matter per area** | |
| | | **% Improvement** | **p-value** | **% Improvement** | **p-value** |
| EVO33872 | 2088 | 13% * | 0.01 * | 7% * | 0.00 * |

**Table 23**

| ***Microbial strains that improve responses indicative of the corn trait "Main ear size" in F field trait and yield assay. In the list are microbial strains that passed the experiment successfully with a minimum of one response improved significantly (2-tails t-test, p-value < 0.2) compared to the non-inoculated control. Statistically significant improved responses are marked by an asterisk.*** | | | | | | | |
|---|---|---|---|---|---|---|---|
| **Microbial strain number** | **Variety** | **Main ear dry weight per plant** | | **Main ear width** | | **Main ear area** | |
| | | **% Improvement** | **p-value** | **% Improvement** | **p-value** | **% Improvement** | **p-value** |
| EVO32844 | 37N01 | 12% * | 0.075 * | 4% * | 0.0733 * | 7% * | 0.1676 * |
| EVO33398 | 37N01 | 11% * | 0.1088 * | 3% * | 0.0808 * | 4% | 0.2586 |
| EVO33402 | 37N01 | 10% * | 0.1006 * | 3% * | 0.0497 * | 5% * | 0.1313 * |
| EVO33405 | 37N01 | 10% * | 0.1965 * | ND | ND | ND | ND |
| EVO33661 | 37N01 | 19% * | 0.043 * | 4% * | 0.0542 * | 8% * | 0.1293 * |
| EVO33872 | 37N01 | 9% * | 0.146 * | 1% | 0.496 | 0% | 0.8 |
| EVO33872 | 1498 | ND | ND | ND | ND | 4% * | 0.04 * |
| EVO40185 | 37N01 | 19% * | 0.005 * | 2% * | 0.108 * | 8% * | 0.031 * |

**Table 24**

| ***Microbial strains that improve responses indicative of the corn trait "Main ear size" in F field trait and yield assay. In the list are microbial strains that passed the experiment successfully with a minimum of one response improved significantly (2-tails t-test, p-value < 0.2) compared to the non-inoculated control Statistically significant improved responses are marked by an asterisk.*** | | | | | |
|---|---|---|---|---|---|
| **Microbial strain number** | **Variety** | **Main ear length** | | **Main ear row number** | |
| | | **% Improvement** | **p-value** | **% Improvement** | **p-value** |
| EVO33872 | 1498 | 3% * | 0.01 * | 1% | 0.02 |

**Table 25**

| ***Microbial strains that improve responses indicative of the corn trait "Kernel volume and weight" and "Cob conductance" in F field trait and yield assay. In the list are microbial strains that passed the experiment successfully with a minimum of one response improved significantly (2-tails t-test, p-value < 0.2) compared to the non-inoculated control. Statistically significant improved responses are marked by an asterisk.*** | | | | | | | |
|---|---|---|---|---|---|---|---|
| **Microbial strain number** | **Variety** | **1000 grains weight** | | **Grain area** | | **Cob width** | |
| | | **% Improvement** | **p-value** | **% Improvement** | **p-value** | **% Improvement** | **p-value** |
| EVO32844 | 37N01 | 0% | 0.8158 | 0% | 0.9816 | 4% * | 0.0073 * |
| EVO32845 | 37N01 | 5% * | 0.1867 * | 4% * | 0.1122 * | 1% | 0.3125 |
| EVO33398 | 37N01 | 7% * | 0.0818 * | 4% * | 0.0961 * | 3% * | 0.0133 * |
| EVO33402 | 37N01 | 7% * | 0.0878 * | 4% * | 0.0836 * | 2% * | 0.0571 * |
| EVO33405 | 37N01 | 6% * | 0.1238 * | 0% | 0.9203 | 2% * | 0.0599 * |
| EVO33661 | 37N01 | 1% | 0.8659 | 0% | 0.9504 | 3% * | 0.0161 * |
| EVO40185 | 37N01 | 3% | 0.463 | 5% * | 0.121 * | 4% * | 0.059 * |

**Table 26**

| ***Microbial strains that improve responses indicative of the corn trait "Increased yield" in F field trait and yield assay. In the list are microbial strains that passed the experiment successfully with a minimum of one response improved significantly (2-tails t-test, p-value < 0.2) compared to the non-inoculated control. Statistically significant improved responses are marked by an asterisk.*** | | | | | | | |
|---|---|---|---|---|---|---|---|
| **Microbial strain number** | **Variety** | **Main ear grain yield per plant** | | **Total grain yield per plant** | | **Bushels per acre** | |
| | | **% Improvement** | **p-value** | **% Improvement** | **p-value** | **% Improvement** | **p-value** |
| EVO32845 | 37N01 | 10% * | 0.0937 * | 12% * | 0.1664 * | ND | ND |
| EVO33398 | 37N01 | 7% | 0.2076 | 11% * | 0.1039 * | ND | ND |
| EVO33402 | 37N01 | 9% * | 0.1458 * | 12% * | 0.1341 * | ND | ND |
| EVO33405 | 37N01 | 3% | 0.6204 | 9% | 0.2172 | ND | ND |
| EVO33661 | 37N01 | 10% * | 0.1281 * | 18% * | 0.0346 * | ND | ND |
| EVO33872 | 37N01 | 9% * | 0.193 * | 10% * | 0.113 * | ND | ND |
| EVO33872 | 1498 | ND | ND | ND | ND | 12% * | 0.01 * |
| EVO33872 | 2088 | ND | ND | ND | ND | 8% | 0.43 |
| EVO40185 | 37N01 | 17% * | 0.015 * | 24% * | 0.001 * | ND | ND |

**Table 27**

| ***Allocation of F responses to specific plant traits.*** | | |
|---|---|---|
| # | **Responses** | **Plant traits** |
| 1 | Lower stem width [mm] | Early vigor and biomass establishment, Stem conductance |
| 2 | Middle stem width [mm] | Stem conductance |
| 3 | Leaf temperature [°C] | Leaf transpiration rate |
| 4 | Quantum yield [Fv/ Fm] | Photosynthetic capacity |
| 5 | SPAD | Photosynthetic capacity |
| 6 | Vegetative dry weight per plant [gr] | Maintain total biomass under stress |
| 7 | Total dry matter per plant [gr] | Maintain total biomass under stress |
| 8 | Vegetative DW per area | Maintain total biomass under stress |
| 9 | Total dry matter per area | Maintain total biomass under stress |
| 10 | Main ear dry weight per plant [gr] | Main ear size |
| 11 | Main ear area [cm²] | Main ear size |
| 12 | Main ear width [cm] | Main ear size |
| 13 | Ear Length [cm] | Main ear size |
| 14 | Ear row num (main) | Main ear size |
| 15 | 1000 grain weight [gr] | Kernel volume and weight |
| 16 | Grain area [cm²] | Kernel volume and weight |
| 17 | Cob width [cm] | Cob conductance |
| 18 | Main ear grain yield per plant [gr] | Increased yield |
| 19 | Total grain yield per plant [gr] | Increased yield |
| 20 | Bushels per acre | Increased yield |

### Discussion

Listed in Tables 19-26 are 13 microbial strains that improve one or more of the above plant traits (Table 27) in the F trait and yield assay (field experiment). Table 27 describes the plant responses and traits improved by those microbial strains in this experiment and the allocation of plant responses to specific plant traits. Among these microbial strains, 7 improve the trait "Increased yield" resulting with an increase in the economic profit one can obtain from the plant treated with those microbial strains. These results strongly suggest that microbial strains that have not yet been tested under field conditions are likely to improve plant production under field conditions. Consistent improvement of similar plant traits across experimental systems (M1, BD, M2 and F) by microbial strains is a strong indication for their ability to improve these traits and yield across location, years, seasons, crops and agriculture practices.

### EXAMPLE 6

### NICHE PREFERENCE-BASED CLUSTERING OF MICROBIAL STRAINS

In this example, Microbial strains were clustered together into groups based on their niche preference. The inventors assume that plant microbiome-derived microbial strains with plant bio-stimulatory activity, co-evolved with plants and therefore have a high preference to colonize plant tissues in comparison to the surrounding soil. Microbial strains inoculated to the seed environment and have the ability to colonize the plant root, can express plant beneficial functions in, on and/or close to the plant root, functions that for example provide protection from plant pathogens and pests or improve nutrients such as nitrogen, phosphorus and/or sulfurous bioavailability near the plant.

The inventors tested the niche preference of microbial strains. Two different niches were addressed:
1) Rhizosphere - As used herein, the "Rhizosphere" is the soil left attached to rood surface after vigorous shaking of the root and removal of the loosely attached soil. The properties of that soil are directly influenced by the activity of the plant root (Hiltner. L. 1904. Über neuere erfahrungen und probleme auf dem gebiete der bodenbakteriologie unter besonderer berücksichtigung der gründüngung und brache. Arb DLG 98:59-78).
2) Bulk soil - As used herein, the "Bulk soil" is the soil away from plant. In the present example, Bulk soil was sampled from a plant growth compartment that was not sown with seed.

### Experimental Procedures

Rifampicin resistant microbial strains were raised by cultivating a suspension of about 10⁹ cells/ ml on R2G plate supplemented with 50 µg/ mL rifampicin. After 2 days on 28 °C in the dark, few resistant colonies were pooled together to produce a rifampicin resistance culture. This culture was further grown and finally suspended in tap water at a concentration of 10⁹ CFU/ ml. One ml of cell suspension was then mixed into 25 grams of field soil placed in a well of a germination tray with or without a corn seed (*n*=3 for each). Soil samples of ~200 mg were used to determine the initial concentration of the rifampicin resistant Microbial strain at the beginning of the experiment (T=0 days). To enumerate cell number in soil, samples were collected from each well into a 2 ml sterile Eppendorf tube and the number of CFUs/ gr of soil was determined by plating of serial dilutions of each sample (done in sterile phosphate buffer saline (PBS)) on R2G plates supplemented with 50 µg/ mL rifampicin. The remains of the soil samples were dried for 2 days in an oven (60 °C) and weighed to allow calculation of CFUs/ gr soil. The germination tray was watered to allow seed germination and kept in the greenhouse for 17 days. On T=17 days, the wells were resampled for bulk-soil samples and plant roots. The rhizosphere soil was separated from each root by washing with sterile PBS and rifampicin resistant colonies in both soil and rhizosphere samples were determined using the same procedure as in T=0.

Niche preference index was calculated as [CFUs/ gr rhizosphere soil (T = 17 days)] / [CFUs/ gr soil (T = 17 days)], in which higher ratio (>2) and significant difference (2-tails t-test, p-value < 0.2) between the results in the presence and absence of growing plants are evidence of preference of the rhizosphere over life in the bulk soil and vice versa.

**Table 28**

| ***Niche preference index of microbial strains. Shown are isolates with preference of rhizosphere niche over the bulk soil. In the list are microbial strains that their abundance in the rhizosphere is significantly greater (2-tails t-test, p-value < 0.2) compared to their abundance in the bulk-soil.*** | | |
|---|---|---|
| **Microbial strain number** | **Niche preference index** | **p-value** |
| EVO32828 | 17.7 | 0.171 |
| EVO32828 | 7.1 | 0.047 |
| EVO32834 | 27.8 | 0.122 |
| EVO32845 | 99.1 | 0.001 |
| EVO32845 | 58 | 0.006 |
| EVO33393 | 21.8 | 0.115 |
| EVO33394 | 46.9 | 0.123 |
| EVO33395 | 341.9 | 0.009 |
| EVO33402 | 993.4 | 0.027 |
| EVO33405 | 3682.6 | 0.142 |
| EVO33405 | 187.3 | 0.002 |
| EVO33407 | 193 | 0.059 |
| EVO33410 | 731.3 | 0.02 |
| EVO33415 | 9.3 | 0.091 |
| EVO33441 | 11.4 | 0.071 |
| EVO33661 | 43.8 | 0.071 |

### Discussion

Listed in Table 28 are 13 microbial strains that significantly prefer the rhizosphere niche over the bulk soil niche. These microbial strains are adapted to the plant environment and are rhizosphere competent (Ghirardi, S., Dessaint, F., Mazurier, S., Corberand, T., Raaijmakers, J.M., Meyer, J.M., Dessaux, Y., and Lemanceau, P. 2012. Identification of traits shared by rhizosphere-competent strains of fluorescent pseudomonads. Microb. Ecol. 64:725-37), and while they stimulate plant growth (as evidenced in examples 2-5), they obtain nutrients and possibly other benefits from the plant in an established mutualistic symbiotic interaction. Therefore, the inventors claim that plant beneficial microbial strains described in this invention, need to be able to colonize the plant microbiome in order to exert their beneficial activity on the plant. The rhizosphere is the first plant microbiome compartment encountered by soil introduced microbial strains. They must be adapted to either colonize the rhizosphere or travel through the rhizosphere toward inner plant microbiome compartments such as the rhizoplane or the plant endosphere. Therefore, their abundance in the rhizosphere should be greater than in the surrounding bulk-soil due to migration to the rhizosphere and/or proliferation in the rhizosphere.

### EXAMPLE 7

### FUNCTIONS-BASED CLUSTERING OF MICROBIAL STRAINS

In this example, the inventors cluster together microbial strains with similar plant beneficial functions. Improvements of plant traits by application of a microbial strain from the lists present in this invention are all evidences for functions that the microbial strains provide to the plant. These functions make the plant more tolerant to the water stress, and allow the plant maintaining improved growth rate and development under fluctuating water availability with lesser stress damage and senesces symptoms. Such functions are functions that improve nutrient availability to the plant, nutrient such as, but not limiting to, nitrogen, phosphorous and sulfur, when diffusion of molecules containing such elements is impaired due to the reduction in water activity in the plant surrounding soil.

### Nitrogen fixation

Nitrogen is a nutrient that limits the growth and productivity of non-leguminous plants and is the most limiting factor in maize production (McCarty, G., and Meisinger, J. 1997.Effects of N fertilizer treatments on biologically active N pools in soils under plow and no tillage. Biol. Fertil. Soils 24,406-412.). Diazotrophs (refers here to nitrogen fixing bacteria) were previously found to interact with plants either in the rhizosphere or endosphere (Reinhold-Hurek, B., and Hurek, T. 1998. Life in grasses: diazotrophic endophytes. Trends Microbiol. 6:139-144; Wakelin, S.A., and Ryder, M.H. 2004. Plant growth-promoting inoculants in Australian agriculture. Crop Manag. 3:1-5). Given the ability of diazotrophs to fix nitrogen, some strains may relieve N-deficiencies where there is inadequate application of N fertilizers. Therefore, the inventors tested a microbial strains for the ability to fix nitrogen assuming that it a key-function that allows them to improve plant production.

### Experimental procedure

Microbial strains were grown on R2G plates for 48 hours on 28 °C, in the dark. Several individual colonies of each strains were pooled together and suspended in one ml of sterile phosphate buffered saline (PBS). Hundred-microliter aliquots of cell suspensions were inoculated into 5 ml sterile NFb medium (per liter: 5 g DL-malic acid, 0.5 g K₂HPO₄, 0.2 g MgSO₄•7H₂O, 0.1 g NaCl, 0.02 g CaCl₂•2H₂O, 2 ml 0.5% Bromthymol blue solution in 0.2M KOH. Adjust pH to 6.5 and add 1.8 g of agar. Autoclave at 121°C for 15 minutes. Add 1 ml filter sterilized vitamin solution [10 mg Biotin and 20 mg Pyridoxol dissolved in 100 ml distilled water], 2 ml micronutrients solution [0.4 g CuSO₄ • 5H₂O, 0.12 g ZnSO₄•7H₂O, 1.4 g H₃BO₃, 1 g Na₂MoO₄•2H₂O, 1.5 g MnSO₄•H₂O] and 4 ml iron solution [Fe(III) EDTA (1.64% solution]; Hartmann, A., and Baldani, J.I. 2006. The genus Azospirillum. In: Dworkin, M., Falkow, S., Rosenberg, E., Schleifer, K.H., Stackebrandt, E., eds. The Prokaryotes: A handbook on the biology of bacteria: Proteobacteria: Alpha and Beta subclass. Springer Science + Business Media, New York: 3ed. v 5:115-140) in 15 ml test tubes. The cultures were incubated for 7 days on 28°C without shaking and thereafter tested for the existence of a pellicle (evidence of growth). If growth was observed, the pellicle forming culture was re-inoculated into fresh medium, incubated again on 28°C for 7 days and if a pellicle was formed again, the strain was considered as nitrogen fixation positive.

**Table 29**

| ***Nitrogen fixing microbial strains.*** | |
|---|---|
| **Microbial strain number** | **N-fixation** |
| EVO32845 | positive |
| EVO33393 | positive |
| EVO33394 | positive |
| EVO33401 | positive |
| EVO33402 | positive |
| EVO33407 | positive |
| EVO33432 | positive |
| EVO33447 | positive |

### Discussion

Listed in Table 29 are 8 microbial strains that can use atmospheric nitrogen as a sole source of nitrogen. Such microbial strains are nitrogen fixers (diazotrophs). This ability to provide nitrogen to plant is already long exploited in the growth of leguminous plants such as soybean where diazotrophs bacteria such as *Rhizobium* species becoming established inside the root in a symbiotic structures called root nodules and are dependent on the host plant for nitrogen fixation (they cannot independently fix nitrogen). Many other microbial strains that do not form root nodules are known to be able to fix nitrogen in-planta, and can be used to increase bioavailability of nitrogen to plant and reduce the amount of fertilizer farmers apply to field. By that, diazotrophs can increase the economical profit one can obtain from a certain growth area or growth season.

### Phosphate solubilization

Phosphorus is the second important key element after nitrogen as a mineral nutrient in terms of quantitative plant requirement. Although abundant in soils, in both organic and inorganic forms, its availability is restricted as it occurs mostly in insoluble forms (Illmer, P.A., Barbato, A., and Schinner, F. 1995. Solubilization of hardly soluble AlPO4 with P-solubilizing microorganisms. Soil Biol. Biochem. 27:260-270). Poor availability or deficiency of phosphorus (P) markedly reduces plant size and growth. To satisfy crop nutritional requirements, P is usually added to soil as chemical P fertilizer that has various long term impacts on the environment and plants can use only a small amount of it that is rapidly becomes fixed in soils. In this regards phosphate solubilizing microbial strains are eco-friendly means for P nutrition of crops (Sharma, S.B., Sayyed, R.Z., Trivedi, M.H., and Gobi, T.A. 2013. Phosphate solubilizing microbes: sustainable approach for managing phosphorus deficiency in agricultural soils. Springerplus 2:587). Therefore, the inventors tested microbial strains for their ability to solubilize phosphate, assuming it is a key-function that allows them to improve plant production.

### Experimental procedure

Microbial strains were grown on R2G plates for 48 hours on 28 °C, in the dark. Several individual colonies of each strains were pooled together and suspended in one ml of sterile phosphate buffered saline (PBS). Hundred-microliter aliquots of cell suspensions were inoculated into 5 ml sterile Tricalcium Phosphate media plates (per liter: 10 g D-Glucose, 0.37 g NH₄NO₃, 0.84 g MgSO₄•7H₂O, 0.3 g NaCl, 5 mg FeCl₃, 0.7 g Ca₃O₈P₂, 8 gr gerite and 1,000 ml double distilled water). The plates were than incubated on 28°C for 2-5 days in the dark and visually inspected daily for the appearance of transparent zones around the colonies - an evidence of solubilization of the mineral calcium phosphorus that is in the medium.

**Table 30**

| ***Phosphate solubilizing microbial strains.*** | |
|---|---|
| **Microbial strain number** | **P-solubilization** |
| EVO32831 | positive |
| EVO32845 | positive |
| EVO33398 | positive |
| EVO33401 | positive |
| EVO33402 | positive |
| EVO33405 | positive |
| EVO33407 | positive |
| EVO33661 | positive |

### Discussion

Listed in Table 30 are 8 microbial strains that can solubilize and assimilate phosphate sequestered in a none soluble form as calcium phosphate. There several pathways through which microbial strains can solubilize phosphate (Sharma, S.B., Sayyed, R,Z., Trivedi, M.H., and Gobi, T.A. 2013. Phosphate solubilizing microbes: sustainable approach for managing phosphorus deficiency in agricultural soils. Springerplus 2:587) and increase the bioavailability of phosphate to plant and by that improve plant productivity. In addition, such microbial strains that can provide phosphate to plant, can reduce use of chemical fertilizers by farmers. Together, their action can increase the economical profit one can obtain from a certain area or growth season.

### ACC degradation

Microbial strains modulate the level of the phytohormone ethylene by consuming the ethylene precursor 1-aminocyclopropane-1-carboxylate (ACC) as a nitrogen source using the enzyme 1-aminocyclopropane-1-carboxylate deaminase (ACCd). The hormone ethylene is an important modulator of normal plant growth and development in plants and is a key feature in the response of plants to a wide range of stresses. Many aspects of the growth of plant tissues such as roots, stems, leaves, flowers and fruits, as well as all stages of plant development are affected by ethylene. Ethylene synthesis is affected by a number of different factors including temperature, light, gravity, nutrition, the presence and level of other plant hormones, and the presence of various types of biological stress to which the plant may be subjected. Regarding a plant's response to stress, an increased level of ethylene is typically formed in response to the presence of metals, organic and inorganic chemicals, temperature extremes, too much or too little water, ultraviolet light, insect damage, nematode damage, phytopathogens (both fungi and bacteria), and mechanical wounding. By decreasing ACC levels in plants, ACCd-producing microbial strains decrease plant ethylene levels, which when present in high concentrations can lead to plant growth inhibition or even death. By that, microbial strains promote plant growth even in the _{presence} of various environmental stresses (abiotic or biotic) like drought stress (Glick, B. 2014. Bacteria with ACC deaminase can promote plant growth and help to feed the world. Microbiol. Res. 169:30-39).

### Experimental procedure

Microbial strains were grown on R2G plates for 48 hours on 28 °C, in the dark. Several individual colonies of each strains were pooled together and suspended in one ml of sterile phosphate buffered saline (PBS). Ten-microliter aliquots of cell suspensions were spotted onto three different assay agar plates:
1) Modified DF minimal salts medium with no nitrogen source used as a negative control (per liter: 2 g glucose, 2 g gluconic acid, 2 g citric acid, 4 g KH₂PO₄, 6 g Na₂HPO₄, 0.2 g MgSO₄•7H₂O, 12 gr agar, 990 ml distilled water and 10 ml micronutrient solution [per liter: 0.2 g CaCl₂, 0.2 g FeSO₄•7H₂O, 15 mg H₃BO₃, 20 mg ZnSO₄•7H₂O, 10 mg Na₂MoO₄, 10 mg KI, 1- mg NaBr, 10 mg MnCl₂, 5 mg COCl₂, 5 mg CuCl₂, 2 mg AlCl₃, 2 mg NiSO₄ and 1 lit distilled water]; Dworken, M., and Foster, J. 1958. Experiments with some microorganisms which utilize ethane and hydrogen. J. Bacteriol. 75: 592-601).
2) Modified DF minimal salts medium supplemented with 2 gr/ liter (NH4)₂SO₄ as nitrogen source used as a positive control.
3) Modified DF minimal salts medium supplemented with 0.3 gr ACC as nitrogen source employed here to test the ability of Microbial strain to utilize ACC as a sole nitrogen source. The plates were incubated at 28 °C for 72 h. microbial strains were considered as ACC degraders if exhibited growth on the plates supplemented with ACC (#3) but not on the negative control plates (#1).

**Table 31**

| ***ACC degrading microbial strains.*** | |
|---|---|
| **Microbial strain number** | **ACC degradation** |
| EVO33393 | positive |
| EVO33398 | positive |
| EVO33402 | positive |
| EVO33447 | positive |
| EVO33661 | positive |

### Discussion

Listed in Table 31 are 5 microbial strains that can use ACC, the precursor of the plant hormone ethylene, as a sole nitrogen source. These microbial strains probably express the enzyme ACCd that reduces the levels of ACC in the plant and consequently, the level of ethylene, that when present in high concentrations can lead to plant growth inhibition or even death. By that, microbial strains promote plant growth even when the plant normally produce high levels of ethylene, for example, when challenged by various environmental stresses (abiotic or biotic) like drought stress.

### EXAMPLE 8

### BIOFILM FORMATION ABILITY-BASED CLUSTERING OF MICROBIAL STRAINS

In this example, the inventors cluster microbial strains based on their ability to physically interact with surfaces to form complex multicellular assemblies known as biofilms and aggregates. Biofilms are microbial-preferred state of existence in which communities gain enhanced defenses and multiple mechanisms of survival that enhance their fitness. Microbials in biofilm also gain access to resources and niches that require critical mass and cannot effectively be utilized by free-living isolated cells. One impotent property of biofilm is the ability to retain moisture that can protect against water deprivation during desiccation or osmotic stress. Moisture trapping is achieved via different polymers of sugars called exopolysaccharides (EPS). Plant-associated microbials sense physical and chemical cues present in the rhizosphere (for example root surface, root polysaccharides and root exudates) and in response switch from a motile free-living physiology to an adhesive physiology allowing them to attach to surfaces (Ramey, B.E, Koutsoudis, M., von Bodman, S.B., and Fuqua, C. 2004. Biofilm formation in plant-microbe associations. Curr. Opin. Microbiol. 7:602-609). Biofilms can be established on various surfaces including plant roots and soil particles in the rhizosphere, sometimes resulting in "wet sleeves" around and along roots and cementing of soil particles that can improve crop productivity and the physiochemical properties of soil (Qurashi, A.W., and Sabri, A.N. 2012. Bacterial exopolysaccharide and biofilm formation stimulate chickpea growth and soil aggregation under salt stress. Braz. J. Microbiol. 43:1183-1191). In addition, biofilm-forming in a rhizosphere exposed to desiccation was reported to be higher than that formed under non-stressful conditions (Bogino, P., Abod, A., Nievas, F., and Giordano, W. 2013. Water-limiting conditions alter the structure and biofilm-forming ability of bacterial multispecies communities in the alfalfa rhizosphere. PLoS One 8(11):e79614). These evidences suggest that an impotent feature through which microbial strains may improve plant production under drought conditions is via biofilm formation.

### Experiment procedures

Microbial strains were revived from a glycerol vile kept in -80°C by streaking them onto R2G plates and growing for 48 hours on 28°C, in the dark. Cells were collected into sterile phosphate-buffered saline (PBS) and the cell suspension optical density as measured at 600 nm (OD600; using a spectrophotometer Infinite M200 PRO) was adjusted to 0.5. 10 µl aliquots were then inoculated into wells of a 96 wells sterile plate already containing 200 µl of R2A liquid broth (4 technical repeats per strain). In each plate 16 wells were supplemented with PBS only as a negative control. The plates were then sealed and incubated without shaking for 48 hours on 28°C, in the dark. The liquid medium with planktonic culture was then carefully removed and the wells were filled with 200-µl crystal violet (0.5% w/v) solution. After 15 minutes of incubation in room temperature (RT) the crystal violet solution was removed, the plated were washed several times with water to remove crystal violet traces and finally air-dried. In order to quantify the biofilm formed on the walls of each well, the biofilm formed on the walls of the wells were immersed with 200 µl of 70% ethanol for 15 minutes to allow release of the crystal violet from the attached biomass. Relative levels of crystal violet dissolved in ethanol in each well, which is indicative of relative levels of attached biomass, were measured at 570 nm using plate reader (Infinite M200 PRO). Each strain was tested in at least three independent experiments. Microbial strains were considered as producing biofilm if the measured OD (570 nm) was significantly higher than the OD (570 nm) of the negative control wells (2-tails t-test, p-value<0.2).

**Table 32**

| ***Biofilm formation by microbial strains.*** | | | |
|---|---|---|---|
| **Microbial strain number** | **Averaged OD (570 nm)** | **Microbial strain/ Negative control** | **p-Value** |
| EVO32844 | 0.439 | 2.28 | 1.53E-01 * |
| EVO32845 | 1.074 | 5.56 | 7.16E-22 * |
| EVO33393 | 1.047 | 5.42 | 1.22E-20 * |
| EVO33394 | 0.899 | 4.66 | 5.35E-10 * |
| EVO33395 | 2.172 | 11.25 | 2.16E-90 * |
| EVO33398 | 2.568 | 13.3 | 4.30E-82 * |
| EVO33401 | 0.893 | 4.63 | 7.51E-10 * |
| EVO33402 | 0.932 | 4.82 | 8.58E-11 * |
| EVO33405 | 1.314 | 6.81 | 1.39E-22 * |
| EVO33407 | 3.454 | 17.89 | 7.99E-94 * |
| EVO33410 | 1.29 | 6.68 | 1.02E-21 * |
| EVO33432 | 0.675 | 3.49 | 2.16E-05 * |
| EVO33441 | 1.231 | 6.38 | 1.17E-19 * |
| EVO33661 | 1.679 | 8.7 | 1.09E-37 * |
| Negative control | 0.193 | 1 | NA |

### Discussion

Listed in Table 32 are 14 microbial strains able to attached and form biofilm on the surface of wells of a 96-wells microliter plate. This data suggests that microbial strains described in this invention are able of forming biofilm on plant surfaces such as the root and on rhizosphere soil particles, establish a stable colony on the proximity of the plant to serve as a basis for the observed mutualistic interaction. The inventors claim that the majority of the microbial strains that form mutual interaction with crop plants and improve plant productivity, have a life stage involve attachment and/ aggregation on and near the plant.

### EXAMPLE 9

### METABOLIC CAPACITY-BASED CLUSTERING OF MICROBIAL STRAINS

### Experimental procedures

Microbial strains were streaked onto R2G plates and grown for 48 hours on 28 °C in the dark. Several colonies were then collected and suspended in 10 ml Inoculation Fluid IF-A (Biolog cat 72401, lot 16OCT061) in a sterile 50 ml tube. Turbidity was measured at wavelength of 590 nm using a plate reader (Infinite M200 PRO) and adjusted to the range of 0.0013-0.007 using Inoculation Fluid (equivalent to 0.004-0.02 in a 1 cm cuvette). Hundred-microliter aliquots of the cell suspension were then distributed into all of the 96 wells of a GEN-III plate (Biolog cat 1030, lot 3012061). The plate was incubated on 28 °C for 48 hours, along which (at 3, 6, 24, 30 and 48 hours) the development of a purple indicator color in each well was measured at a wavelengths of 590 nm and 750 nm. Strain performances (carbon source utilization and chemical resistance and sensitivity) were according to manufacture instructions (www(dot)biolog(dot)com/pdf/milit/00P%20185rA%20GEN%20III%20MicroPlate%20IFU%20 Mar2008.pdf).

### Tables 33-41

Microbial strains clustering based on their carbon source utilization ability. "+" refers to ability to grow with the specified nutrient as a sole source of carbon, "-" refers to inability to grow with specified nutrient as a sole source of carbon. Marked by an asterisk are the positive results.

**Table 33**

| **Microbial strain number** | **L-Aspartic Acid** | **L-Alanine** | **L-Glutamic Acid** | **N-Acetyl-D-Glucosamine** | **D-Gluconic Acid** | **L-Malic Acid** | **Glycerol** |
|---|---|---|---|---|---|---|---|
| EVO32845 | +* | + * | + * | + * | + * | + * | + * |
| EVO33393 | + * | + * | + * | + * | + * | + * | + * |
| EVO33398 | + * | + * | + * | + * | + * | + * | + * |
| EVO33401 | + * | + * | + * | + * | + * | + * | + * |
| EVO33402 | + * | + * | + * | + * | + * | + * | + * |
| EVO33405 | + * | + * | + * | + * | + * | + * | + * |
| EVO33407 | + * | + * | + * | + * | + * | + * | + * |
| EVO33410 | + * | + * | + * | + * | + * | + * | + * |
| EVO33441 | + * | + * | + * | + * | + * | + * | + * |
| EVO33661 | + * | + * | + * | + * | + * | + * | + * |

**Table 34**

| **Microbial strain number** | **Acetic Acid** | **D-Galactose** | **D-Mannose** | **Alpha-D-Glucose** | **D-Fructose** | **D-Mannitol** | **L- Lactic Acid** |
|---|---|---|---|---|---|---|---|
| EVO32845 | + * | + * | + * | + * | + * | + * | + * |
| EVO33393 | + * | + * | + * | + * | + * | - | + * |
| EVO33398 | + * | + * | + * | + * | + * | + * | + * |
| EVO33401 | + * | + * | + * | + * | + * | + * | + * |
| EVO33402 | + * | + * | + * | + * | + * | + * | + * |
| EVO33405 | + * | + * | + * | + * | + * | + * | + * |
| EVO33407 | + * | + * | + * | + * | + * | + * | + * |
| EVO33410 | + * | + * | + * | + * | + * | + * | + * |
| EVO33441 | + * | + * | + * | + * | + * | + * | + * |
| EVO33661 | + * | + * | + * | + * | + * | + * | + * |

**Table 35**

| **Microbial strain number** | **Alpha-Hydroxy-D,L-Butyric Acid** | **Mucic Acid** | **D-Saccharic Acid** | **Glucuronamid e** | **Formic Acid** | **Citric Acid** | **myo-Inositol** |
|---|---|---|---|---|---|---|---|
| EVO32845 | - | + * | + * | + * | + * | + * | + * |
| EVO33393 | + * | + * | + * | + * | + * | - | + * |
| EVO33398 | + * | + * | + * | + * | + * | + * | - |
| EVO33401 | + * | + * | + * | + * | + * | + * | + * |
| EVO33402 | + * | + * | + * | + * | + * | + * | + * |
| EVO33405 | + * | + * | + * | + * | + * | + * | + * |
| EVO33407 | + * | + * | + * | + * | + * | + * | + * |
| EVO33410 | + * | + * | + * | - | + * | + * | + * |
| EVO33441 | + * | + * | + * | + | + * | + * | + * |
| EVO33661 | + * | + * | + * | + * | + * | + * | + * |

**Table 36**

| **Microbial strain number** | **D-Glucuronic Acid** | **L-Serine** | **D-Fucose** | **L-Arginine** | **Inosine** | **D-Sorbitol** | **D-Fructose-6-PO4** |
|---|---|---|---|---|---|---|---|
| EVO32845 | + * | + * | + * | + * | + * | - | + * |
| EVO33393 | + * | + * | + * | + * | - | - | + * |
| EVO33398 | - | + * | + * | + * | + * | - | + * |
| EVO33401 | + * | - | - | - | - | - | + * |
| EVO33402 | + * | + * | + * | + * | - | + * | + * |
| EVO33405 | + * | + * | + * | + * | + * | + * | + * |
| EVO33407 | + * | + * | + * | + * | + * | + * | - |
| EVO33410 | + * | + * | + * | + * | + * | + * | + * |
| EVO33441 | + * | + * | + * | + * | + * | + * | + * |
| EVO33661 | + * | + * | + * | + * | + * | + * | + * |

**Table 37**

| **Microbial strain number** | **Methyl Pyruvate** | **Pectin** | **Acetoacetic Acid** | **D-Trehalose** | **D-Melibiose** | **Glycyl-L-Proline** | **D-Glucose-6-PO4** |
|---|---|---|---|---|---|---|---|
| EVO32845 | + * | + * | + * | + * | + * | + * | + * |
| EVO33393 | + * | + * | + * | + * | + * | - | + * |
| EVO33398 | + * | - | - | + * | + * | + * | - |
| EVO33401 | + * | + * | + * | + * | + * | + * | + * |
| EVO33402 | + * | - | - | + * | - | + * | + * |
| EVO33405 | + * | + * | + * | - | - | - | - |
| EVO33407 | - | - | - | + * | - | - | - |
| EVO33410 | + * | - | - | + * | + * | + * | + * |
| EVO33441 | - | - | - | + * | + * | + * | + * |
| EVO33661 | - | + * | + * | + * | - | + * | - |

**Table 38**

| **Microbial strain number** | **D-Cellobiose** | **Gentiobiose** | **Beta-Methyl-D-Glucoside** | **D-Salicin** | **D-Maltose** | **L-Rhamnose** | **D-Turanose** |
|---|---|---|---|---|---|---|---|
| EVO32845 | + * | + * | + * | + * | + * | + * | - |
| EVO33393 | + * | + * | + * | + * | + * | + * | + * |
| EVO33398 | - | - | - | - | - | - | - |
| EVO33401 | + * | + * | + * | + * | + * | + * | + * |
| EVO33402 | - | - | - | - | - | + * | - |
| EVO33405 | - | - | - | - | - | - | - |
| EVO33407 | - | - | - | - | - | - | - |
| EVO33410 | + * | + * | + * | + * | + * | + * | - |
| EVO33441 | + * | + * | + * | + * | + * | + * | + |
| EVO33661 | - | - | - | - | - | - | - |

**Table 39**

| **Microbial strain number** | **D-Raffin ose** | **Stach yose** | **Alpha-D-Lactose** | **N-Acetyl-D-Galactosamine** | **N-Acetyl-Beta-D-Mannosamine** | **Dex trin** | **Bromo-Succinic Acid** |
|---|---|---|---|---|---|---|---|
| EVO32845 | + * | - | - | - | - | + * | + * |
| EVO33393 | - | - | - | + * | + * | + * | - |
| EVO33398 | - | - | - | - | - | - | + * |
| EVO33401 | + * | + * | + * | + * | + * | + * | - |
| EVO33402 | - | - | + * | + * | - | + * | + * |
| EVO33405 | - | - | - | - | - | - | - |
| EVO33407 | - | - | - | - | - | - | - |
| EVO33410 | + * | - | + * | - | + * | + * | - |
| EVO33441 | + * | + * | + * | + * | + * | + * | - |
| EVO33661 | - | - | - | - | - | - | + * |

**Table 40**

| **Microbial strain number** | **D-Lactic Acid Methyl Ester** | **Quinic Acid** | **D-Arab itol** | **Propio nic Acid** | **Alpha-KetoGlutaric Acid** | **L-Fuc ose** | **Gamma-Amino-Butryric Acid** | **Twe en 40** |
|---|---|---|---|---|---|---|---|---|
| EVO32845 | - | + * | - | - | - | + * | - | + * |
| EVO33393 | + * | + * | + * | + * | + * | + * | + * | + * |
| EVO33398 | - | + * | + * | + * | + * | + * | + * | + * |
| EVO33401 | - | + * | - | - | - | - | + * | - |
| EVO33402 | - | + * | + * | + * | + * | + * | + * | + * |
| EVO33405 | - | + * | + * | + * | + * | + * | + * | + * |
| EVO33407 | - | + * | + * | + * | + * | - | + * | + * |
| EVO33410 | - | + * | + * | + * | + * | - | + * | + * |
| EVO33441 | - | + * | + * | + * | + * | + * | - * | - * |
| EVO33661 | - | + * | + * | + * | + * | + * | + * | + * |

**Table 41**

| **Microbial strain number** | **Alpha-HydroxyButyric Acid** | **Gel atin** | **D-Malic Acid** | **3-Methyl Glucose** | **N-Acetyl Neuraminic Acid** | **Alpha-Keto-Butyric Acid** | **D-Aspartic Acid** |
|---|---|---|---|---|---|---|---|
| EVO32845 | + * | - | - | + * | - | - | - |
| EVO33393 | - | + * | + * | + * | + * | + * | - |
| EVO33398 | + * | - | - | - | - | + * | - |
| EVO33401 | - | + * | - | - | + * | - | - |
| EVO33402 | + * | - | + * | - | - | + * | - |
| EVO33405 | + * | - | - | - | - | - | - |
| EVO33407 | - | - | - | - | - | - | - |
| EVO33410 | + * | - | + * | + * | - | + * | + * |
| EVO33441 | - * | + * | + * | + * | - | - * | - * |
| EVO33661 | - | - | + * | - | - | - | + * |

### Discussion

Listed in Tables 33-41 are ten microbial strains and the carbon sources they can utilize as sole sources of carbon. There are 12 carbon sources that all of the tested microbial strains were capable of utilizing. Among these are several amino acids (L-Aspartic Acid, L-Alanine and L-Glutamic Acid), several sugars (D-Galactose, D-Mannose, D-Fructose and Alpha-D-Glucose), and organic acids (D-Gluconic Acid, Malic Acid and Acetic Acid), all of which were previously reported to be deposit into the rhizosphere in a process called rhizodeposition (Doornbos, R.F., van Loon, L.C., and Bakker, P.A.H.M. 2012. Impact of root exudates and plant defense signaling on bacterial communities in the rhizosphere. A review. Agron. Sustain. Dev. 32: 227-243), indicating that all of the microbial strains tested here are adapted to exploit plant exudates for their growth. Other metabolic similarities are indication of functional overlap between microbial strains resulting with similar adaption and niche preference.

### Tables 42-45

Microbial strains clustering based on their chemical resistance. "+" refers to resistance to the chemical/ chemical condition and "-" refers to sensitivity to the chemical/ chemical condition. Marked by an asterisk are the positive results.

**Table 42**

| **Microbial strain number** | **Niaproof 4** | **Troleandomyci n** | **4% NaCl** | **1% NaCl** | **Lincomyci n** | **Rifamycin SV** |
|---|---|---|---|---|---|---|
| EVO32844 | - | - | - | + * | + * | + * |
| EVO32845 | + * | + * | + * | + * | + * | + * |
| EVO33393 | + * | + * | + * | + * | + * | + * |
| EVO33394 | - | - | - | + * | - | - |
| EVO33395 | - | - | + * | + * | - | - |
| EVO33398 | - | + * | + * | + * | + * | + * |
| EVO33401 | - | + * | + * | + * | - | + * |
| EVO33402 | - | - | - | - | + * | + * |
| EVO33405 | + * | + * | + * | + * | + * | + * |
| EVO33407 | + * | + * | + * | + * | + * | + * |
| EVO33410 | + * | + * | + * | + * | + * | + * |
| EVO33432 | - | - | + * | + * | + * | + * |
| EVO33441 | + * | + * | + * | + * | + * | + * |
| EVO33661 | + * | + * | + * | + * | + * | + * |

**Table 43**

| **Microbial strain number** | **1% Sodium Lactate** | **pH 6** | **Potassium Tellurite** | **8% NaCl** | **Guanidine HCl** | **Lithium Chloride** |
|---|---|---|---|---|---|---|
| EVO32844 | + * | + * | + * | - | - | - |
| EVO32845 | + * | + * | - | + * | + * | + * |
| EVO33393 | + * | + * | + * | - | - | - |
| EVO33394 | + * | + * | + * | - | - | + * |
| EVO33395 | + * | + * | + * | + * | - | + * |
| EVO33398 | + * | + * | + * | - | + * | - |
| EVO33401 | + * | + * | - | - | - | - |
| EVO33402 | + * | + * | - | - | - | - |
| EVO33405 | + * | + * | + * | - | + * | + * |
| EVO33407 | + * | + * | + * | - | - | - |
| EVO33410 | + * | + * | + * | + * | + * | + * |
| EVO33432 | + * | + * | + * | + * | - | + * |
| EVO33441 | + * | + * | + * | + * | + * | + * |
| EVO33661 | + * | + * | + * | - | - | + * |

**Table 44**

| **Microbial strain number** | **pH 5** | **Vancomyc in** | **D-Serine** | **Tetrazolium Blue** | **Tetrazolium Violet** | **Sodium Bromate** |
|---|---|---|---|---|---|---|
| EVO32844 | - | - | + * | + * | + * | - |
| EVO32845 | + * | + * | + * | + * | + * | - |
| EVO33393 | - | - | - | - | - | - |
| EVO33394 | - | - | - | - | - | - |
| EVO33395 | - | - | + * | - | + * | + * |
| EVO33398 | + * | + * | - | + * | + * | + * |
| EVO33401 | - | + * | - | + * | + * | - |
| EVO33402 | + * | + * | - | + * | + * | - |
| EVO33405 | + * | + * | + * | + * | + * | - |
| EVO33407 | + * | + * | - | + * | + * | - |
| EVO33410 | + * | + * | - | + * | + * | + * |
| EVO33432 | + * | + * | - | + * | + * | - |
| EVO33441 | + * | + * | - | + * | + * | + * |
| EVO33661 | + * | + * | - | + * | + * | - |

**Table 45**

| **Microbial strain number** | **Nalidixic Acid** | **Sodium Butyrate** | **Minocycline** | **Fusidic Acid** | **Aztreonam** |
|---|---|---|---|---|---|
| EVO32844 | - | - | - | - | + * |
| EVO32845 | - | - | - | - | - |
| EVO33393 | - | - | - | - | - |
| EVO33394 | + * | - | - | - | + * |
| EVO33395 | + * | - | - | - | - |
| EVO33398 | + * | - | + * | + * | + * |
| EVO33401 | - | - | - | - | - |
| EVO33402 | - | - | - | - | - |
| EVO33405 | + * | - | + * | + * | + * |
| EVO33407 | - | - | - | + * | + * |
| EVO33410 | + * | + * | + * | + * | + * |
| EVO33432 | - | - | - | - | - |
| EVO33441 | + * | + * | + * | + * | + * |
| EVO33661 | + * | - | - | + * | + * |

### Discussion

Chemical resistance and sensitivity similarities between microbial strains described in this invention is another indication of functional overlap between microbial strains resulting with similar adaptions and niche preferences. In this example, microbial strains are clustered by both their resistance to a chemical and sensitivity to a chemical. For example, most tested microbial strains are sensitive to sodium butyrate.

**Table 46**

| ***List of Isolates*** | | | |
|---|---|---|---|
| **Isolate ID Number** | **Organism** | **16S SEQ ID NOs** | **Deposit Accession Number** |
| EVO11090 | Bacillus sp. | 1 | 42935 |
| EVO32828 | Pseudomonas sp. | 2;3 | 42940 |
| EVO32831 | Acinetobacter sp. | 4 | 42932 |
| EVO32834 | Microbacterium sp. | 5;6 | 42941 |
| EVO32839 | Pseudoxanthomonas sp. | 7 | 42945 |
| EVO32844 | Chryseobacterium sp. | 8 | 42929 |
| EVO32845 | Erwinia sp. | 9;10 | 42930 |
| EVO32868 | Pseudomonas sp. | 11 | 42942 |
| EVO33393 | Pseudomonas sp. | 12;13 | 42924 |
| EVO33394 | Arthrobacter sp. | 14 | 42928 |
| EVO33395 | Kocuria sp. | 15 | 42927 |
| EVO33398 | Pseudomonas sp. | 16 | 42926 |
| EVO33401 | Erwinia sp. | 17 | 42923 |
| EVO33402 | Paraburkholderia sp. | 18 | 42943 |
| EVO33405 | Pseudomonas sp. | 19;20 | 42931 |
| EVO33407 | Pseudomonas sp. | 21;22 | 42922 |
| EVO33410 | Flavobacterium sp. | 23 | 42961 |
| EVO33415 | Acidovorax sp. | 24 | 42938 |
| EVO33432 | Bacillus sp. | 25 | 42921 |
| EVO33441 | Enterobacter sp. | 26;27 | 42960 |
| EVO33447 | Variovorax sp. | 28;29 | 42937 |
| EVO33657 | Acidovorax sp. | 30 | 42936 |
| EVO33661 | Pseudomonas sp. | 31 | 42925 |
| EVO33746 | Bacillus sp. | 32 | 42933 |
| EVO33872 | Curtobacterium sp. | 33 | 42959 |
| EVO33887 | Paenibacillus sp. | 34 | 42934 |
| EVO40185 | Bacillus sp. | 35 | 42939 |
| EVO40194 | Bacillus sp. | 36 | 42944 |

### EXAMPLE 10

### 16S-RRNA MICROBIAL STRAIN IDENTIFICATION

### Experimental procedures

In the present invention, 16S-rRNA sequences were obtained by either:
1) Polymerase Chain Reaction (PCR; Mullis, K.B., Erlich, H.A., Arnheim, N., Horn, G.T., Saiki, R.K, Less, S.J.S. 1987. Process for amplifying, detecting, and/or-cloning nucleic acid sequences. U.S. Patent 4,683,195) using the universal primers 16S_27F (AGAGTTTGATCMTGGCTCAG, SEQ ID NO: 169) and 16S_1492R (TACGGYTACCTTGTTACGACTT, SEQ ID NO: 170) (Eden, P.A., Schmidt, T.M., Blakemore, R.P., and Pace, N.R. 1991. Phylogenetic analysis of Aquaspirillum magnetotacticum using polymerase chain reaction-amplified 16S rRNA-specific DNA. Int. J. Syst. Bacteriol. 41:324-325) followed by Sanger sequencing (Sanger, F., and Coulson, A.R. 1975. A rapid method for determining sequences in DNA by primed synthesis with DNA polymerase. J. Mol. Biol. 94:441-448) of the amplified fragments using the primers 16S_27F and 16S_1492R, and the additional primers 16S-5151F (GTGCCAGCMGCCGCGGTAA, SEQ ID NO: 171) and 16S_970R (CCGTCAATTCMTTTRAGTTT, SEQ ID NO: 172).
2) Extraction of 16S-rRNA sequences from the assembly of microbial strains genome sequences (Evogene proprietary pipeline) using MOTHUR tool (bioinformatics tool for analyzing 16S-rRNA gene sequences; Schloss, P.D., Westcott, S.L., Ryabin, T., Hall, J.R., Hartmann, M., Hollisteret, E.B., al. 2009. Introducing mothur: open-source, platform-independent, community-supported software for describing and comparing microbial communities. Appl. Environ. Microbiol. 75:7537-7541) and SILVA database (a comprehensive on-line resource for quality checked and aligned ribosomal RNA sequence data; Pruesse, E., Quast, C., Knittel, K., Fuchs, B.M., Ludwig, W., Peplies, J., and Glöckner, F.O. 2007. SILVA: a comprehensive online resource for quality checked and aligned ribosomal RNA sequence data compatible with ARB. Nucl. Acids Res. 35:7188-7196) as a reference.

Obtained 16S-RNA sequences were clustered (grouped) to Operational Taxonomic Units (OTUs) using nucleotide-based local alignment search tool (BLASTN; Altschul, S., Gish, W., Miller, W., Myers, E.W., and Lipman, D.J. 1990. Basic local alignment search tool. J. of Mol. Biol. 215:403-410).

### EXAMPLE 11

### W: WHEAT FIELD TRAIT AND YIELD ASSAY

Field experiments and results are provided substantiating that microbial strains according to some embodiments of the invention improve wheat traits when applied to the seed as a single microbial strain as a seed coat prior to sowing. Specifically tested were pre-defined target plant traits under moderate drought treatment applied between the stage of flowering (VT) and the harvest (H):
1) "Early vigor and biomass establishment"
2) "Photosynthetic capacity"
3) "Maintain total biomass under stress"
4) "Leaf transpiration rate"
5) "Stem conductance"
6) "Increased assimilate partitioning"
7) "Increased kernel number per plant"
8) "Kernel volume and weight"
9) "Increased yield"

### Experiment procedures:

### Microbial strains and seed coating:

Microbial strains were grown as a lawn on five R2G plates each [per liter: 0.5 g proteose peptone, 0.5 g casamino acids, 0.5 g yeast extract, 0.5 g dextrose, 0.5 g soluble starch, 0.3 g dipotassium phosphate (K₂HPO₄), 0.05 g, magnesium sulfate (MgSO₄•7H₂O), 0.3 g sodium pyruvate and 8 g gelrite as a gelling agent] for 2 days at 28 °C in the dark. The bacteria were then collected and suspended in 20 ml sterile R2A broth (with the gelrite) supplemented with 25 % glycerol and kept on -80°C until use. Two weeks before field sowing, each strain was cultured on 25 R2G plates and regrown for 2 days at 28 °C in the dark. Cells were then harvested and suspended in 50 ml tap water supplemented with 2% Carboxy Methyl Cellulose (CMC) serving as a gluing agent. This suspension was mixed with bread wheat seeds (AGRIDERA Seeds & Agriculture Ltd - Yuval, Gedera and Omer varieties) in a ratio of 1 part of suspension (in gr) for every 20 parts of seeds (in gr). As a control, seeds were incubated with the water-CMC solution only. The mixture was shaken gently for 10 min and thereafter the seeds were separated and dried on a paper towel in a biological cabinet for few hours. The average number of CFUs on seeds was measured 3 days before sowing to ensure a titer of >10⁵ CFUs/ seed by vigorously mixing five coated seeds in PBS to release bacteria from coat, serially diluting the sample, plating the dilutions on R2G plates and enumerating CFUs 2 days after.

### Sowing and plant growth:

Coated wheat seeds (with bacteria and control) were sown using a manual seed planter. Untreated seeds were used as a second control that was not treated with neither bacteria, nor water-CMC solution. The planter box was cleaned between seed batches using 70 % ethanol and rinsing with tap water to eliminate ethanol traces. Each combination of seed with microbial strain coat, including the control, was sown in six or 8 replicated plots (*n*=6 or *n*=8) in a random blocks statistical design. Each plot was 8 m long and 1.4 m wide at a density of 220 seeds/ per m² (total of 2464 seeds per plot). Before the fields were sown, soil samples were taken for analysis of N.P.K (nitrogen, phosphorous, potassium) in the field. To adjust the fertilizer levels to common wheat growth practice, commercial fertilizer (Phosphorus - 10 ppm, Nitrogen 6-10 units, Gat fertilizers Ltd.) was added. The fields were sown in 3 locations with a range of yearly average rainfall between 200 to 450 mm. Moderate drought treatment was simulated in the location with an average rainfall of 200 to 300 mm per year, and high drought potential. In order to discover microbial strains that improve vegetative and reproductive pre-defined plant responses and plant traits in the field, plant responses and yield parameters were measured during the experiments.

Measured Responses in field wheat trait assay:
**1) Plant height [cm]** - Four randomly selected but representative plants from each plot were characterized for plant height. The height was measured from the base of the plant up to the canopy height of the highest tiller, every two weeks at three time points during the vegetative growth period.
**2) NDVI [float value]** - Each plot was characterized for NDVI (Normalized Difference Vegetation Index) using RapidSCAN CS-45 by Holland Scientific, every two weeks at 3 time points during the vegetative growth period. NDVI is calculated as the ratio between spectral bands (NIR-Red)/(NIR+Red), where red and NIR stand for the spectral reflectance measurements acquired in the red (visible) and near-infrared regions, respectively.
**3) NDRE [float value]** - Each plot was characterized for NDRE (Normalized Difference Red-Edge) using RapidSCAN CS-45 by Holland Scientific, every two weeks at 3 time points during the vegetative growth period. NDRE is calculated as the ratio between spectral bands (NIR-Red edge)/(NIR+Red edge), where NIR stand for the spectral reflectance measurements acquired in the near-infrared regions and red edge is the term used to describe the part of the spectrum centered around 715 nm.
**4) Plant height growth [cm/ day]** - Calculated as a slope of plant height and the time points taken during the vegetative growth period.
**5) SPAD [SPAD units]** - Chlorophyll content was determined using a SPAD 502 chlorophyll meter (Minolta) and measurement was performed at 3 time points during the growth period. SPAD meter readings were done on young fully developed leaf. Four measurements per leaf were taken per plot.
**6) Quantum yield [Fv/ Fm]** - Photosystem II efficiency was measured using the FluorPen-100 fluorometer (Photon System Instruments) at 3 time points during the growth period. Quantum yield readings were done on young fully developed leaf. Four measurements per leaf were taken per plot.
**7) Tillers per unit area [number\ m²]** - Tillers number in a defined area. Measured by counting the number of tillers in a represented area of a plot size of 0.5 m² at flowering.
**8) Tiller dry weight [gr]** - The weight of the tillers after 48 hours of drying at 70 °C, divided by the number of plants, per plot at flowering.
**9) Vegetative dry weight per unit area [gr\ m²]** - The weight after 48 hours drying at 70 °C, of the above-ground vegetative plant material without the spikes, per 0.5 m² per plot at flowering.
**10) Total dry matter per unit area [gr\ m²]** - The weight after 48 hours drying at 70 °C, of the above-ground vegetative plant material with the spikes per 0.5 m² per plot at flowering.
**11) Leaf temperature [°C]** - Leaf temperature was measured at vegetative stages using an IR thermometer 568 device (Fluke). Measurements were done on a fully developed leaf.
**12) Lower stem width [mm]** - Four plants from each plot were characterized for lower stem width. The stem was measured in the lower internode using diameter at flowering.
**13) Peduncle width (GF) [mm]** - Measurement of peduncle width (the internode below the spike) of plants at flowering.
**14) Spikes index** - Calculated as the ratio between the spikes DW and the total dry matter of the plant at flowering.
**15) Spike to tiller ratio** - The ratio between the number of spikes per unit area and the number of tillers per unit area at flowering.
**16) Harvest index** - The ratio between the grains yield per hectare and the total dry matter per hectare.
**17) Grains per spike** - Number of grains per fertile spikelet's in a plot.
**18) Spikes per unit area [number\ m²]** - Number of spikes per unit area (0.5 m²), per plot at heading.
**19) Fertile spikelets** - Number of fertile spikes per plant at harvest.
**20) 1,000 grains weight [gr]** - The ratio between the grains yield per plant, divided by the number of grains per plant, multiplied by 1,000.
**21) Spike dry weight [gr]** - The weight after 48 hours drying at 70 °C, of the spikes per unit area, divided by the number of plants per unit area at flowering.
**22) Spikes dry weight per unit area [gr/ m²] -** The weight after 48 hours of drying at 70 °C, of the spikes per unit area (0.5 m²) at flowering.
**23) Grains yield per hectare [kg\ hectare]** - The weight of the grains that were harvested using mechanical combine per plot area (11.2 m²), converted to hectare.

**Table 47**

| ***Microbial strains that improve responses indicative of the wheat trait "Early vigor and biomass establishment" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | | | |
|---|---|---|---|---|---|
| **VAR** | **Microbial strain number** | **Plant height** | | **Plant height growth** | |
| | | **% Improvement** | **p-value** | **% Improvement** | **p-value** |
| Gedera | EVO33394 | 8% * | 0.04 * | ND | ND |
| Yuval | EVO33399 | 7% * | 0.13 * | 5% * | 0.197 * |
| Yuval | EVO33398 | 8% * | 0.09 * | ND | ND |
| Yuval | EVO33441 | 10% * | 0.11 * | ND | ND |

**Table 48**

| ***Microbial strains that improve responses indicative of the wheat trait "Early vigor and biomass establishment" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | | | |
|---|---|---|---|---|---|
| **VAR** | **Microbial strain number** | **NDVI** | | **NDRE** | |
| | | **% Improvement** | **p-value** | **% Improvement** | **p-value** |
| Gedera | EVO33394 | 12% * | 0.12 * | 11% * | 0.12 * |
| Yuval | EVO33441 | ND | ND | 8% * | 0.14 * |
| Omer | EVO32839 | 7% * | 0.18 * | ND | ND |
| Gedera | EVO32845 | 9% * | 0.09 * | ND | ND |

**Table 49**

| ***Microbial strains that improve responses indicative of the wheat trait "Photosynthetic capacity" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | | | |
|---|---|---|---|---|---|
| **VAR** | **Microbial strain number** | **SPAD** | | **Quantum yield** | |
| | | **% Improvement** | **p-value** | **% Improvement** | **p-value** |
| Omer | EVO32839 | 3% * | 0.198 * | ND | ND |
| Omer | EVO33839 | 4% * | 0.17 * | ND | ND |
| Omer | EVO33394 | ND | ND | 4% * | 0.14 * |

**Table 50**

| ***Microbial strains that improve responses indicative of the wheat trait "Stem conductance" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk (Leaf temperature improvement is to the negative direction).*** | | | | | |
|---|---|---|---|---|---|
| **VAR** | **Microbial strain number** | **Peduncle width (GF)** | | **Lower stem width** | |
| | | **% Improvement** | **p-value** | **% Improvement** | **p-value** |
| Omer | EVO32839 | 16% * | 0.08 * | ND | ND |
| Gedera | EVO33403 | ND | ND | 11% * | 0.18 * |
| Gedera | EVO33433 | ND | ND | 20% * | 0.03 * |

**Table 51**

| ***Microbial strains that improve responses indicative of the wheat trait "Leaf transpiration rate"** in **the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk (Leaf temperature improvement is to the negative direction).*** | | | |
|---|---|---|---|
| **VAR** | **Microbial strain number** | **Leaf temperature** | |
| | | **% Improvement** | **p-value** |
| Gedera | EVO32845 | -5% * | 0.13 * |
| Omer | EVO33394 | -3% * | 0.12 * |
| Gedera | EVO33839 | -6% * | 0.07 * |

**Table 52**

| ***Microbial strains that improve responses indicative of the wheat trait "Maintain total biomass under stress" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | | | |
|---|---|---|---|---|---|
| **VAR** | **Microbial strain number** | **Tillers per unit area (F)** | | **Avr tiller DW (F)** | |
| | | **% Improvement** | **p-value** | **% Improvement** | **p-value** |
| Yuval | EVO32845 | 16% * | 0.17 * | 27% * | 0.001 * |
| Yuval | EVO33394 | 16% * | 0.17 * | ND | ND |
| Yuval | EVO33398 | 28% * | 0.02 * | ND | ND |
| Gedera | EVO33403 | 18% * | 0.11 * | ND | ND |
| Gedera | EVO33410 | 16% * | 0.16 * | ND | ND |
| Yuval | EVO33441 | 17% * | 0.18 * | 21% * | 0.02 * |
| Gedera | EVO33639 | 17% * | 0.13 * | ND | ND |
| Gedera | EVO33662 | 15% * | 0.17 * | ND | ND |
| Yuval | EVO33399 | ND | ND | 16% * | 0.05 * |
| Yuval | EVO33433 | ND | ND | 25% * | 0.003 * |

**Table 53**

| ***Microbial strains that improve responses indicative of the wheat traits "Maintain total biomass under stress" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | | | |
|---|---|---|---|---|---|
| **VAR** | **Microbial strain number** | **Vegetative DW per unit area (F)** | | **Total dry matter per unit area (F)** | |
| | | **% Improvement** | **p-value** | **% Improvement** | **p-value** |
| Yuval | EVO32845 | 47% * | 0.002 * | 41% * | 0.009 * |
| Yuval | EVO33394 | 26% * | 0.08 * | 27% * | 0.09 * |
| Yuval | EVO33398 | 37% * | 0.015 * | 40% * | 0.01 * |
| Gedera | EVO33410 | 18% * | 0.12 * | 17% * | 0.14 * |
| Yuval | EVO33410 | 20% * | 0.15 * | 21% * | 0.15 * |
| Yuval | EVO33433 | 41% * | 0.008 * | 38% * | 0.015 * |
| Yuval | EVO33441 | 41% * | 0.017 * | 42% * | 0.018 * |
| Gedera | EVO33639 | 15% * | 0.19 * | 16% * | 0.17 * |
| Yuval | EVO33639 | 23% * | 0.18 * | 26% * | 0.15 * |
| Gedera | EVO33662 | 15% * | 0.18 * | ND | ND |
| Omer | EVO32839 | 14% * | 0.03 * | ND | ND |
| Gedera | EVO32845 | 12% * | 0.18 * | ND | ND |
| Omer | EVO32845 | 9% * | 0.17 * | ND | ND |

**Table 54**

| ***Microbial strains that improve responses indicative of the wheat trait "Increased assimilate partitioning" in the W field trait and yield assay. In the list are microbial strains that passed the experiment successfully with a minimum of one response improved significantly (2-tails t-test, p-value < 0.2) compared to the non-inoculated control. Statistically significant improved responses are marked by an asterisk.*** | | | | | |
|---|---|---|---|---|---|
| **VAR** | **Microbial strain number** | **Spikes index (F)** | | **Harvest Index** | |
| | | **% Improvement** | **p-value** | **% Improvement** | **p-value** |
| Gedera | EVO33399 | 11% * | 0.05 * | ND | ND |
| Gedera | EVO33410 | ND | ND | 14% * | 0.14 * |

**Table 55**

| ***Microbial strains that improve responses indicative of the wheat trait "Increased assimilate partitioning" in the W field trait and yield assay. In the list are microbial strains that passed the experiment successfully with a minimum of one response improved significantly (2-tails t-test, p-value < 0.2) compared to the non-inoculated control. Statistically significant improved responses are marked by an asterisk.*** | | | |
|---|---|---|---|
| **VAR** | **Microbial strain number** | **Spike to tiller ratio (F)** | |
| | | **% Improvement** | **p-value** |
| Gedera | EVO32845 | 7% * | 0.18 * |
| Yuval | EVO33403 | 33% * | 0.16 * |
| Gedera | EVO33639 | 10% * | 0.06 * |

**Table 56**

| ***Microbial strains that improve responses indicative of the wheat trait "Increased kernel number per plant" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | | | | | |
|---|---|---|---|---|---|---|---|
| **VAR** | **Microbial strain number** | **Grains per spike** | | **Spikes per unit area** | | **Fertile spikelets** | |
| | | **% Improvement** | **p-value** | **% Improvement** | **p-value** | **% Improvement** | **p-value** |
| Gedera | EVO32845 | ND | ND | 20% * | 0.10 * | ND | ND |
| Yuval | EVO33398 | ND | ND | 58% * | 0.04 * | ND | ND |
| Yuval | EVO33441 | ND | ND | 47% * | 0.15 * | ND | ND |
| Gedera | EVO33639 | ND | ND | 28% * | 0.024 * | ND | ND |
| Yuval | EVO33639 | 18% * | 0.10 * | 45% * | 0.18 * | 7% * | 0.12 * |
| Omer | EVO32839 | ND | ND | 8% * | 0.14 * | ND | ND |
| Omer | EVO32845 | ND | ND | 10% * | 0.13 * | ND | ND |

**Table 57**

| ***Microbial strains that improve responses indicative of the wheat trait "Kernel volume and weight" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | |
|---|---|---|---|
| **VAR** | **Microbial strain number** | **1000 grain weight** | |
| | | **% Improvement** | **p-value** |
| Gedera | EVO33394 | 11% * | 0.06 * |

**Table 58**

| ***Microbial strains that improve responses indicative of the wheat traits "Increased yield" in the W field trait and yield assay. In the list are microbial strains that passed the experiment successfully with a minimum of one response improved significantly (2-tails t-test, p-value < 0.2) compared to the non-inoculated control on each variety (VAR). Statistically significant improved responses are marked by an asterisk.*** | | | | | | | |
|---|---|---|---|---|---|---|---|
| **VAR** | **Microbial strain number** | **Spike dry weight** | | **Spikes dry weight per unit area** | | **Grains yield per hectare** | |
| | | **% Improvement** | **p-value** | **% Improvement** | **p-value** | **% Improvement** | **p-value** |
| Yuval | EVO33410 | 16% * | 0.09 * | ND | ND | 5% * | 0.03 * |
| Yuval | EVO33639 | 16% * | 0.18 * | 46% * | 0.16 * | 2% * | 0.19 * |
| Omer | EVO32839 | 19% * | 0.04 * | ND | ND | ND | ND |
| Omer | EVO32845 | 17% * | 0.04 * | ND | ND | ND | ND |
| Omer | EVO33394 | 9% * | 0.19 * | ND | ND | ND | ND |
| Yuval | EVO33398 | ND | ND | 59% * | 0.03 * | ND | ND |
| Yuval | EVO33441 | ND | ND | 47% * | 0.13 * | ND | ND |
| Gedera | EVO33639 | ND | ND | 22% * | 0.11 * | ND | ND |
| Gedera | EVO32845 | ND | ND | ND | ND | 4% * | 0.11 * |
| Yuval | EVO33662 | ND | ND | ND | ND | 4% * | 0.04 * |
| Gedera | EVO32839 | ND | ND | ND | ND | 10% * | 0.08 * |
| Yuval | EVO32845 | ND | ND | ND | ND | 26% * | 0.06 * |
| Gedera | EVO33839 | ND | ND | ND | ND | 8% * | 0.13 * |

**Table 59**

| ***Allocation of wheat W responses to specific plant traits.*** | | |
|---|---|---|
| **#** | **Responses** | **Plant traits** |
| 1 | Plant height [cm] | Early vigor and biomass establishment, Maintain total biomass under stress |
| 2, 3 | NDVI, NDRE [float value] | Early vigor and biomass establishment, Photosynthetic capacity |
| 4 | Plant height growth [cm/ day] | Early vigor and biomass establishment, Maintain total biomass under stress |
| 5 | SPAD [SPAD units] | Photosynthetic capacity |
| 6 | Quantum yield [Fv/ Fm] | Photosynthetic capacity |
| 7 | Tillers per unit area [number/ m²] | Maintain total biomass under stress |
| 8 | Tiller dry weight [gr] | Maintain total biomass under stress |
| 9 | Vegetative dry weight per unit area [gr/ m²] | Maintain total biomass under stress |
| 10 | Total dry matter per unit area [gr/ m²] | Maintain total biomass under stress |
| 11 | Leaf temperature [°C] | Leaf transpiration rate |
| 12 | Lower stem width [mm] | Stem conductance |
| 13 | Peduncle width (GF) [mm] | Stem conductance |
| 14 | Spikes index [float value] | Increased assimilate partitioning |
| 15 | Spike to tiller ratio [float value] | Increased assimilate partitioning |
| 16 | Harvest Index [float value] | Increased assimilate partitioning |
| 17 | Grains per spike [number] | Increased kernel number per plant |
| 18 | Spikes per unit area [number\ m²] | Increased kernel number per plant |
| 19 | Fertile spikelets [number] | Increased kernel number per plant |
| 20 | 1000 grain weight [gr] | Kernel volume and weight |
| 21 | Spike dry weight [gr] | Increased yield |
| 22 | Spikes dry weight per unit area [gr/ m²] | Increased yield |
| 23 | Grains yield per hectare [Kg\hectare] | Increased yield |

### Discussion:

Listed in Tables 47-58 are 12 microbial strains that improve one or more of the above plant traits (Table 59) in the W Wheat Field Trait and Yield Assay. Table 59 describes the plant responses and traits improved by those microbial strains in this experiment and the allocation of plant responses to specific plant traits. Among these microbial strains, 9 improve the trait "Increased yield", resulting in an increase in the economic profit using these microbial strains. These results strongly suggest that microbial strains that have not yet been tested under field conditions are likely to improve plant production under field conditions. Consistent improvement of similar plant traits across experimental systems (M1, BD, M2 and F) by microbial strains is a strong indication for their ability to improve these traits and yield across location, years, seasons, crops and agriculture practices.

### EXAMPLE 12

### CLUSTERING OF MICROBIAL STRAINS USING STRAIN-SPECIFIC GENOMIC-MARKERS

### Experimental procedures

DNA fragments in a length ranging from 300 bp to 500 bp from genomes of the Microbial strains described in this invention, were screened against the NCBI nucleotide database using NCBI local alignment tool BLASTN (NCBI-blast-2.7.1+). Criteria for declaring a Microbial strain-specific marker are less than 90% coverage and less than 90% aliment identity. 5 Microbial strain-specific markers were selected for each Microbial strain described in this invention.

**Table 60**

| ***Markers summary*** | | | |
|---|---|---|---|
| **Marker SEQ ID NOs** | **Microbial strain number** | **Number of Microbial strain-specific markers** | **Sequences length, concordant with Marker SEQ ID NOs** |
| 37-41 | EVO11090 | 5 | 491;398;443;386;323 |
| 42-46 | EVO32828 | 5 | 483;474;472;472;488 |
| 47-51 | EVO32831 | 5 | 429;492;446;428;255 |
| 52-56 | EVO32834 | 5 | 497;476;489;499;423 |
| 57-61 | EVO32839 | 5 | 500;499;498;496;492 |
| 62-66 | EVO32844 | 5 | 493;497;478;491;490 |
| 67-71 | EVO32845 | 5 | 497;500;497;498;498 |
| 72-76 | EVO32868 | 5 | 492;489;451;448;345 |
| 77-81 | EVO33393 | 5 | 497;500;498;497;498 |
| 82-86 | EVO33394 | 5 | 494;497;497;499;496 |
| 87-91 | EVO33395 | 5 | 471;428;484;443;479 |
| 92-96 | EVO33398 | 5 | 491;468;446;471;441 |
| 97-101 | EVO33401 | 5 | 463;460;467;457;445 |
| 102-106 | EVO33402 | 5 | 493;499;492;500;494 |
| 107-111 | EVO33405 | 5 | 434;463;498;487;447 |
| 112-116 | EVO33407 | 5 | 497;497;497;499;494 |
| 117-121 | EVO33410 | 5 | 494;491;491;487;456 |
| 122-126 | EVO33415 | 5 | 489;494;494;490;497 |
| 127-131 | EVO33432 | 5 | 495;497;490;497;486 |
| 132-135 | EVO33441 | 4 | 304;233;308;265 |
| 136-140 | EVO33447 | 5 | 496;496;481;476;498 |
| 141-145 | EVO33657 | 5 | 494;499;495;498;500 |
| 146-150 | EVO33661 | 5 | 474;497;467;493;430 |
| 151-155 | EVO33746 | 5 | 469;443;379;343;269 |
| 156-160 | EVO33872 | 5 | 500;497;496;373;332 |

| **Marker SEQ ID NOs** | **Microbial strain number** | **Number of Microbial strain-specific markers** | **Sequences length, concordant with Marker SEQ ID NOs** |
|---|---|---|---|
| 161-165 | EVO33887 | 5 | 488;479;465;450;356 |
| 166-170 | EVO40185 | 5 | 454;446;479;364;441 |
| 171-175 | EVO40194 | 5 | 430;364;433;472;426 |

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

### SEQUENCE LISTING STATEMENT

The ASCII file, entitled 75298 Sequence Listing.txt, created on 22 November 2018, comprising 183 kilobytes, submitted concurrently with the filing of this application is incorporated herein by reference.

In addition, any priority document(s) of this application is/are hereby incorporated herein by reference in its/their entirety.

## Claims

1. A preparation comprising a microbial EVO40185 strain, deposited as Accession Number 42939 at NCIMB, or a functionally homologous strain which comprises a 16S rRNA that is at least 99.5 % identical to SEQ ID NO: 35, wherein said microbial strain or said functionally homologous strain improves an agricultural trait of a cultivated plant heterologous to said microbial strain or said functionally homologous strain as compared to a control plant not treated with said microbial strain or said functionally homologous strain, wherein said microbial strain or said functionally homologous strain is present in the preparation at a concentration which exceeds that found in nature.

2. A microbial culture comprising the preparation of claim 1.

3. The microbial culture of claim 2, being at least 99.1 % pure and/or comprising no more than 10 bacterial strains and/or being soilfree.

4. A composition comprising the preparation of claim 1 or the microbial culture of claim 2 or 3, and an agricultural carrier.

5. A method of improving an agricultural trait of a cultivated plant, the method comprising:
(a) contacting the plant or portion thereof with an effective amount of the preparation of any one of claims 1-3 or the composition-of-matter of claim 4; and
(b) growing the plant or portion thereof; and
(c) selecting for the agricultural trait.

6. The method of claim 5, wherein said contacting comprises contacting the plant's surrounding.

7. The method of any one of claims 5-6, wherein said contacting is selected from the group consisting of spraying, immersing, coating, encapsulating, and dusting.

8. The method of claim 5, wherein said contacting comprises coating.

9. The method of any one of claims 5-8, wherein said microbial strain is present at a concentration of at least 100 CFU or spores per plant or portion thereof after said contacting.

10. The method of any one of claims 5-9, wherein said portion comprises a seed.

11. A composition comprising the preparation of any one of claims 1-3, and a cultivated plant or portion thereof said cultivated plant or portion thereof being heterologous to the microbial strain.

12. A method of processing a cultivated plant or portion thereof to a processed product of interest, the method comprising:
(a) providing a composition comprising the preparation of any one of claims 1-3, and a cultivated plant or a portion thereof, said plant or portion thereof being heterologous to the microbial strain or culture; and
(b) subjecting said cultivated plant or portion thereof to a processing procedure so as to obtain the processed product.

13. A method for preparing an agricultural composition, said method comprising inoculating a microbial EVO40185 strain, deposited as Accession Number 42939 at NCIMB, or a functionally homologous strain which comprises a 16S rRNA that is at least 99.5 % identical to SEQ ID NO: 35, wherein said microbial strain or said functionally homologous strain improves an agricultural trait of a cultivated plant heterologous to said microbial strain or said functionally homologous strain as compared to a control plant not treated with said microbial strain or said functionally homologous strain,
into or onto a substratum and allowing said microbial strain or said functional homolog to grow at a temperature of 1-37°C until obtaining a number of cells or spores of at least 10²-10³ per milliliter or per gram.

14. The preparation, culture, composition or method of any one of claims 1-13, wherein said agricultural trait is total grain yield per plant, harvest index or total dry matter per plant.

15. The preparation, culture, composition or method of any one of claims 1-14, wherein said functional homologous strain comprises a genomic nucleic acid sequence which comprises sub-genomic sequences as set forth in SEQ ID NOs: 166-170.
